# EUROPEAN PATENT SPECIFICATION

(11) **EP 1 865 052 B1**
(45) Date of publication and mention of the grant of the patent: **01.02.2017**
(21) Application number: 07018601.0
(22) Date of filing: 05.11.2004
(51) Int. Cl.: C12N 1/20, C12N 1/14, C12P 21/02, C07K 5/06

(54) **Process for producing dipeptides**
Verfahren zur Produktion von Dipeptiden
Processus de production de dipeptides

(30) Priority: 05.11.2003 JP 2003375823; 25.06.2004 JP 2004189010
(43) Date of publication of application: 12.12.2007
(62) Divisional of application: 04026279.2
(73) Proprietor: Kyowa Hakko Bio Co., Ltd., Tokyo, 100-8185 (JP)
(72) Inventor: Hashimoto, Shin-ichi, Hofu-shi Yamaguchi 747-8522 (JP); Tabata, Kazuhiko, Machida-shi Tokyo 194-8533 (JP)
(74) Representative: Vossius & Partner Patentanwälte Rechtsanwälte mbB

(56) References cited:
- EP-A- 1 411 060
- EP-A- 1 411 062
- EP-A- 1 411 116
- EP-A- 1 433 791
- KOK J ET AL: "GENETIC MANIPULATION OF THE PEPTIDOLYTIC SYSTEM IN LACTIC ACID BACTERIA" INTERNATIONAL DAIRY JOURNAL, ELSEVIER APPLIED SCIENCE, BARKING,, GB, vol. 5, no. 8, 1995, pages 737-755, XP008009094 ISSN: 0958-6946
- NAKAMURA A ET AL: "USE OF A TRIPLE PROTEASE-DEFICIENT MUTANT OF BACILLUS-SUBTILIS AS A HOST FOR SECRETION OF A BACILLUS-SUBTILIS CELLULASE AND TEM BETA LACTAMASE", AGRICULTURAL AND BIOLOGICAL CHEMISTRY, vol. 55, no. 9, 1991, pages 2367-2374, ISSN: 0002-1369
- TRUMBLY R J ET AL: "ISOLATION AND CHARACTERIZATION OF AMINOPEPTIDASE MUTANTS OF SACCHAROMYCES CEREVISIAE", JOURNAL OF BACTERIOLOGY, AMERICAN SOCIETY FOR MICROBIOLOGY, WASHINGTON, DC; US, vol. 156, no. 1, 1 October 1983 (1983-10-01), pages 36-48, XP008042891, ISSN: 0021-9193
- WU X-C ET AL: "CLONING GENETIC ORGANIZATION AND CHARACTERIZATION OF A STRUCTURAL GENE ENCODING BACILLOPEPTIDASE F FROM BACILLUS-SUBTILIS", JOURNAL OF BIOLOGICAL CHEMISTRY, vol. 265, no. 12, 1990, pages 6845-6850, ISSN: 0021-9258
- MILLER C G ET AL: "PEPTIDASE DEFICIENT MUTANTS OF ESCHERICHIA-COLI", JOURNAL OF BACTERIOLOGY, AMERICAN SOCIETY FOR MICROBIOLOGY, US, vol. 135, no. 2, 1 August 1978 (1978-08-01), pages 603-611, XP009050077, ISSN: 0021-9193
- J. R. Klein ET AL: "Cloning, heterologous expression, and sequencing of a novel proline iminopeptidase gene, pepl. from Lactobacillus delbrueckii subsp. lactis DSM 7290", Microbiology, vol. 140, no. 5, 1 May 1994 (1994-05-01), pages 1133-1139, XP055150105, ISSN: 1350-0872, DOI: 10.1099/13500872-140-5-1133

## Description

### Background of the Invention

The present invention is defined by the claims and relates to a process for producing a dipeptide as charachterized in the appended claims.

Dipeptides are compounds that are important as foods, pharmaceuticals, cosmetics and the like.

Known methods for producing dipeptides include extraction from natural products, chemical synthesis and enzymatic methods. Extraction from natural products can be used only for producing limited kinds of dipeptides, and the productivity is low because the contents of desired dipeptides in natural products are low. In the synthesis of dipeptides by the chemical synthesis methods, operations such as introduction and removal of protective groups for functional groups are necessary, and racemates are also formed. The chemical synthesis methods are thus considered to be disadvantageous in respect of cost and efficiency. They are unfavorable also from the viewpoint of environmental hygiene because of the use of large amounts of organic solvents and the like.

As to the synthesis of dipeptides by the enzymatic methods, the following methods are known: a method utilizing reverse reaction of protease (J. Biol. Chem., 119, 707-720 (1937)); methods utilizing thermostable aminoacyl t-RNA synthetase (Japanese Published Unexamined Patent Application No. 146539/83, Japanese Published Unexamined Patent Application No. 209991/83, Japanese Published Unexamined Patent Application No. 209992/83 and Japanese Published Unexamined Patent Application No. 106298/84); methods utilizing non-ribosomal peptide synthetase (hereinafter referred to as NRPS) (Chem. Biol., 7, 373-384 (2000), FEBS Lett., 498, 42-45 (2001), U.S. Patent No. 5,795,738 and U.S. Patent No. 5,652,116); a method utilizing D-Ala-D-Ala ligase (Biochemistry, 35, 10464-10471 (1996)); methods utilizing bacilysin synthetase (J. Ind. Microbiol., 2, 201-208 (1987) and Enzyme. Microbial. Technol., 29, 400-406 (2001)); and a method utilizing L-amino-acid amide hydrolase or proline iminopeptidase (WO03/010307 pamphlet).

However, the method utilizing reverse reaction of protease requires introduction and removal of protective groups for functional groups of amino acids used as substrates, which causes difficulties in raising the efficiency of peptide-forming reaction and in preventing peptide-degrading reaction. The methods utilizing thermostable aminoacyl t-RNA synthetase have the defects that the expression of the enzyme and the prevention of reactions forming by-products are difficult.

On the other hand, the methods utilizing NRPS, D-Ala-D-Ala ligase and bacilysin synthetase do not have the problems described above and are capable of producing dipeptides having specific sequences. However, they are not efficient methods because they involve reactions requiring energy donors such as ATP.

With regard to the method utilizing L-amino-acid amide hydrolase or proline iminopeptidase, it is disclosed that dipeptides can be produced by using a culture of a microorganism producing the enzyme, microorganism cells isolated from the culture or a treated matter of the microorganism cells. However, the amounts of dipeptides produced by this method are not sufficient.

Living cells have metabolic systems in which unnecessary proteins are decomposed to constituent amino acids and the formed amino acids are used for synthesis of necessary proteins. As this function is essential for the survival and proliferation of cells, it is known that many kinds of proteases and peptidases exist in living organisms.

For example, it is known that in Escherichia coli, there exist at least seven kinds of dipeptidases as well as other proteases and peptidases capable of decomposing dipeptides (FEMS Microbial. Rev., 63, 265-276 (1989)). On the chromosomal DNAs of Corynebacterium glutamicum, Bacillus subtilis and Saccharomyces cerevisiae, there exist 25 or more, 14 or more, and 20 or more, respectively, genes identified as peptidase gene.

However, it is not known that introduction of peptidase deficiency into a microorganism producing a dipeptide increases the production of the dipeptide.

It is also known that living cells have plural peptide-incorporating systems. For example, Escherichia coli is reported to have three systems for incorporating dipeptides (Chem. Biol., 5, 489-504 (1998) and Microbiol., 145, 2891-2901 (1999)). Further, it has been confirmed from genomic DNA information that microorganisms belonging to the genera Escherichia, Bacillus, Corynebacterium and Saccharomyces all have three or more kinds of peptide-incorporating systems.

However, it is not known whether dipeptides synthesized in cells are discharged outside the cells or not. Still less is known that the production of a dipeptide by a dipeptide-producing microorganism is increased by introducing deficiency of a protein involved in the peptide incorporation into the microorganism.

An object of the present invention is to provide a process for producing a dipeptide using the microorganism.

### Summary of the Invention

The present invention relates to the following (1) to (16).
(1) A process for producing a dipeptide, which comprises:
   allowing an enzyme source and one or more kinds of amino acids to be present in an aqueous medium, said enzyme source being a culture of a microorganism in which the activities of three or more kinds of peptidases are reduced or lost as compared to the parent strain, said parent strain belonging to the genus Escherichia, Bacillus, Corynebacterium or Saccharomyces, wherein said microorganism has the ability to produce a dipeptide, and wherein said ability to produce a dipeptide is the ability to form a dipeptide by condensation and ligation of one or more kinds of amino acids; or a treated matter of the culture;
   allowing the dipeptide to form and accumulate in the aqueous medium; and
   recovering the dipeptide from the medium.
(2) The process according to the above (1), wherein the peptidase is a protein having the amino acid sequence shown in any of SEQ ID NOs: 1 to 4, or a protein having an amino acid sequence which has 80% or more homology to the amino acid sequence shown in any of SEQ ID NOs: 1 to 4 and having peptidase activity.
(3) The process according to the above (1) or (2), wherein the microorganism which has the ability to produce a dipeptide by condensation and ligation of one or more kinds of amino acids is a microorganism having the ability to produce a protein according to any of [1] to [4] below:
   [1] a protein having the amino acid sequence shown in any of SEQ ID NOs: 19 to 25 and 68;
   [2] a protein consisting of an amino acid sequence wherein one or more amino acid residues are deleted, substituted or added in the amino acid sequence shown in any of SEQ ID NOs: 19 to 25 and 68 and having the activity to synthesize a dipeptide;
   [3] a protein consisting of an amino acid sequence which has 65% or more homology to the amino acid sequence shown in any of SEQ ID NOs: 19 to 25 and 68 and having the activity to synthesize a dipeptide; and
   [4] a protein having an amino acid sequence which has 80% or more homology to the amino acid sequence shown in SEQ ID NO: 33 and having the activity to synthesize a dipeptide.
(4) The process according to the above (1) to (3), wherein the microorganism which has the ability to produce a dipeptide by condensation and ligation of one or more kinds of amino acids is a microorganism carrying DNA according to any of [1] to [4] below:
   [1] DNA encoding the protein according to any of [1] to [4] of the above (3);
   [2] DNA having the nucleotide sequence shown in any of SEQ ID NOs: 26 to 32, 64 and 65;
   [3] DNA which hybridizes with DNA having the nucleotide sequence shown in any of SEQ ID NOs: 26 to 32, 64 and 65 under stringent conditions and which encodes a protein having the activity to synthesize a dipeptide; and
   [4] DNA having a nucleotide sequence which has 80% or more homology to the nucleotide sequence shown in SEQ ID NO: 34 and encoding a protein having the activity to synthesize a dipeptide.
(5) The process according to the above (1) to (4), wherein the microorganism which has the ability to produce a dipeptide by condensation and ligation of one or more kinds of amino acids is a microorganism carrying a recombinant DNA in which the DNA according to any of [1] to [4] of the above (4) is ligated to a vector DNA.
(6) The process according to the above (1) to (5), wherein the dipeptide is a dipeptide represented by the following formula (I):

   R1 - R2 (I)

   (wherein R1 and R2, which may be the same or different, each represent an amino acid).
(7) A process for producing a dipeptide, which comprises:
   allowing an enzyme source, an L-amino acid ester and an L-amino acid to be present in an aqueous medium, said enzyme source being a culture of a microorganism in which the activities of three or more kinds of peptidases are reduced or lost as compared to the parent strain, said parent strain belonging to the genus Escherichia, Bacillus, Corynebacterium or Saccharomyces, wherein said microorganism has the ability to produce a dipeptide, and wherein said ability to produce a dipeptide is the ability to form a dipeptide from an L-amino acid ester and an L-amino acid; or a treated matter of the culture;
   allowing the dipeptide to form and accumulate in the aqueous medium; and
   recovering the dipeptide from the medium.
(8) The process according to the above (7), wherein the peptidase is a protein having the amino acid sequence shown in any of SEQ ID NOs: 1 to 4, or a protein having an amino acid sequence which has 80% or more homology to the amino acid sequence shown in any of SEQ ID NOs: 1 to 4 and having peptidase activity.
(9) The process according to the above (7) or (8), wherein the microorganism having the ability to form a dipeptide from an L-amino acid ester and an L-amino acid is a microorganism producing proline iminopeptidase.
(10) The process according to the above (7) to (9), wherein the microorganism having the ability to form a dipeptide from an L-amino acid ester and an L-amino acid is a microorganism carrying a recombinant DNA in which DNA encoding a protein having proline iminopeptidase activity is ligated to a vector DNA.
(11) A process for producing a dipeptide, which comprises:
   allowing an enzyme source, an L-amino acid amide and an L-amino acid to be present in an aqueous medium, said enzyme source being a culture of a microorganism in which the activities of three or more kinds of peptidases are reduced or lost as compared to the parent strain, said parent strain belonging to the genus Escherichia, Bacillus, Corynebacterium or Saccharomyces, wherein said microorganism has the ability to produce a dipeptide, and wherein said ability to produce a dipeptide is the ability to form a dipeptide from an L-amino acid amide and an L-amino acid; or a treated matter of the culture;
   allowing the dipeptide to form and accumulate in the aqueous medium; and
   recovering the dipeptide from the medium.
(12) The process according to the baove (11), wherein the peptidase is a protein having the amino acid sequence shown in any of SEQ ID NOs: 1 to 4, or a protein having an amino acid sequence which has 80% or more homology to the amino acid sequence shown in any of SEQ ID NOs: 1 to 4 and having peptidase activity.
(13) The process according to the above (11) or (12), wherein the microorganism having the ability to form a dipeptide from an L-amino acid amide and an L-amino acid is a microorganism producing a protein having L-amino acid amide hydrolase activity.
(14) The process according to the above (11) to (13), wherein the microorganism having the ability to form a dipeptide from an L-amino acid amide and an L-amino acid is a microorganism carrying a recombinant DNA in which DNA encoding a protein having L-amino acid amide hydrolase activity is ligated to a vector DNA.
(15) The process according to the above (1) to (14), wherein the treated matter of the culture is concentrated culture, dried culture, cells obtained by centrifuging the culture, or a product obtained by subjecting the cells to drying, freeze-drying, treatment with a surfactant, ultrasonication, mechanical friction, treatment with a solvent, enzymatic treatment or immobilization.
(16) The process according to the above (1) to (15), further comprising the formulation of the produced dipeptide into a food additive, a pharmaceutical composition or a cosmetic.

Also described herin is the following (1) to (21).
(1) A microorganism in which the activities of one or more kinds of peptidases and one or more kinds of proteins having peptide-transporting activity (hereinafter referred to also as peptide-transporting proteins) are reduced or lost and which has the ability to produce a dipeptide.
(2) A microorganism in which the activities of three or more kinds of peptidases are reduced or lost and which has the ability to produce a dipeptide.
(3) The microorganism according to the above (1) or (2), wherein the peptidase is a protein having the amino acid sequence shown in any of SEQ ID NOS: 1 to 4, or a protein having an amino acid sequence which has 80% or more homology to the amino acid sequence shown in any of SEQ ID NOS: 1 to 4 and having peptidase activity.
(4) The microorganism according to the above (1) or (3), wherein the peptide-transporting protein is a protein having the amino acid sequence shown in any of SEQ ID NOS: 5 to 9, or a protein having an amino acid sequence which has 80% or more homology to the amino acid sequence shown in any of SEQ ID NOS: 5 to 9 and having peptide-transporting activity.
(5) The microorganism according to any of the above (1) to (4), wherein the microorganism which has the ability to produce a dipeptide is a microorganism having the ability to produce a protein according to any of [1] to [4] below:
   [1] a protein having the amino acid sequence shown in any of SEQ ID NOS: 19 to 25 and 68;
   [2] a protein consisting of an amino acid sequence wherein one or more amino acid residues are deleted, substituted or added in the amino acid sequence shown in any of SEQ ID NOS: 19 to 25 and 68 and having the activity to synthesize a dipeptide;
   [3] a protein consisting of an amino acid sequence which has 65% or more homology to the amino acid sequence shown in any of SEQ ID NOS: 19 to 25 and 68 and having the activity to synthesize a dipeptide; and
   [4] a protein having an amino acid sequence which has 80% or more homology to the amino acid sequence shown in SEQ ID NO: 33 and having the activity to synthesize a dipeptide.
(6) The microorganism according to any of the above (1) to (4), wherein the microorganism which has the ability to produce a dipeptide is a microorganism carrying DNA according to any of [1] to [4] below:
   [1] DNA encoding the protein according to any of [1] to [4] of the above (5);
   [2] DNA having the nucleotide sequence shown in any of SEQ ID NOS: 26 to 32, 64 and 65;
   [3] DNA which hybridizes with DNA having the nucleotide sequence shown in any of SEQ ID NOS: 26 to 32, 64 and 65 under stringent conditions and which encodes a protein having the activity to synthesize a dipeptide; and
   [4] DNA having a nucleotide sequence which has 80% or more homology to the nucleotide sequence shown in SEQ ID NO: 34 and encoding a protein having the activity to synthesize a dipeptide.
(7) The microorganism according to any of the above (1) to (6), wherein the microorganism which has the ability to produce a dipeptide is a microorganism carrying a recombinant DNA in which the DNA according to any of [1] to [4] of the above (6) is ligated to a vector DNA.
(8) The microorganism according to any of the above (1) to (7), wherein the microorganism is a microorganism belonging to the genus Escherichia, Bacillus, Corynebacterium or Saccharomyces.
(9) The microorganism according to any of the above (1) to (4), wherein the microorganism which has the ability to produce a dipeptide is a microorganism having the ability to form a dipeptide from an L-amino acid ester and an L-amino acid.
(10) The microorganism according to the above (9), wherein the microorganism having the ability to form a dipeptide from an L-amino acid ester and an L-amino acid is a microorganism producing proline iminopeptidase.
(11) The microorganism according to the above (9) or (10), wherein the microorganism having the ability to form a dipeptide from an L-amino acid ester and an L-amino acid is a microorganism carrying a recombinant DNA in which DNA encoding a protein having proline iminopeptidase activity is ligated to a vector DNA.
(12) The microorganism according to any of the above (9) to (11), wherein the microorganism is a microorganism belonging to the genus Escherichia, Bacillus, Corynebacterium or Saccharomyces.
(13) The microorganism according to any of the above (1) to (4), wherein the microorganism is a microorganism having the ability to form a dipeptide from an L-amino acid amide and an L-amino acid.
(14) The microorganism according to the above (13), wherein the microorganism having the ability to form a dipeptide from an L-amino acid amide and an L-amino acid is a microorganism producing a protein having L-amino acid amide hydrolase activity.
(15) The microorganism according to the above (13), wherein the microorganism having the ability to form a dipeptide from an L-amino acid amide and an L-amino acid is a microorganism carrying a recombinant DNA in which DNA encoding a protein having L-amino acid amide hydrolase activity is ligated to a vector DNA.
(16) The microorganism according to any of the above (13) to (15), wherein the microorganism is a microorganism belonging to the genus Escherichia, Bacillus, Corynebacterium or Saccharomyces.
(17) A process for producing a dipeptide, which comprises:
   allowing an enzyme source and one or more kinds of amino acids to be present in an aqueous medium, said enzyme source being a culture of the microorganism according to any of the above (1) to (8) or a treated matter of the culture;
   allowing the dipeptide to form and accumulate in the aqueous medium; and
   recovering the dipeptide from the medium.
(18) The process according to the above (17), wherein the dipeptide is a dipeptide represented by the following formula (I):

   R¹ - R² (I)

   (wherein R¹ and R², which may be the same or different, each represent an amino acid).
(19) A process for producing a dipeptide, which comprises:
   allowing an enzyme source, an L-amino acid ester and an L-amino acid to be present in an aqueous medium, said enzyme source being a culture of the microorganism according to any of the above (9) to (12) or a treated matter of the culture;
   allowing the dipeptide to form and accumulate in the aqueous medium; and
   recovering the dipeptide from the medium.
(20) A process for producing a dipeptide, which comprises:
   allowing an enzyme source, an L-amino acid amide and an L-amino acid to be present in an aqueous medium, said enzyme source being a culture of the microorganism according to any of the above (13) to (16) or a treated matter of the culture;
   allowing the dipeptide to form and accumulate in the aqueous medium; and
   recovering the dipeptide from the medium.
(21) The process according to any of the above (17) to (20), wherein the treated matter of the culture is concentrated culture, dried culture, cells obtained by centrifuging the culture, or a product obtained by subjecting the cells to drying, freeze-drying, treatment with a surfactant, ultrasonication, mechanical friction, treatment with a solvent, enzymatic treatment or immobilization.

In accordance with the present invention, there is provided a process for producing a dipeptide as charachterized in the appended claims.

### Brief Description of the Drawings

Fig. 1 shows the steps for constructing His-tagged ywfE gene expression vector pQE60ywfE.
Fig. 2 shows the steps for constructing ywfE gene expression-enhanced vector pPE56.

### Explanation of Symbols

PT5: T5 promoter
Ptrp: Tryptophan promoter

### Detailed Description

The present invention is described in detail below.

### 1. Microorganisms

The microorganisms described herein are microorganisms in which the activities of one or more kinds of peptidases and one or more kinds of proteins having peptide-transporting activity (hereinafter referred to as peptide-transporting proteins) are reduced or lost and which have the ability to produce a dipeptide, or microorganisms in which the activities of three or more kinds of peptidases are reduced or lost and which have the ability to produce a dipeptide.

The microorganisms in which the activities of one or more kinds of peptidases and one or more kinds of peptide-transporting proteins are reduced or lost include microorganisms in which the activities of one or more arbitrary kinds of peptidases and one or more arbitrary kinds of peptide-transporting proteins are reduced or lost provided that the microorganisms can normally grow, specifically, microorganisms in which the activities of preferably one to nine kinds, more preferably one to seven kinds, further preferably one to four kinds of peptidases and preferably one to five kinds, more preferably one to three kinds, further preferably one or two kinds, particularly preferably one kind of peptide-transporting protein are reduced or lost.

Examples of such microorganisms are microorganisms in which the activities of one or more kinds of peptidases and one or more kinds of peptide-transporting proteins are reduced or lost because the nucleotide sequences of one or more kinds of genes encoding peptidases (hereinafter referred to as peptidase genes) and one or more kinds of genes encoding peptide-transporting proteins (hereinafter referred to as peptide-transporting protein genes) among the peptidase genes and peptide-transporting protein genes existing on the genomic DNA of the microorganisms are entirely or partially deleted or said nucleotide sequences contain nucleotide substitutions or additions.

The expression "the activity of peptidase is reduced" means that the peptide-degrading activity is reduced compared with peptidase having none of the above deletions, substitutions and additions of nucleotides.

The peptide-degrading activity of a microorganism can be measured by allowing a peptide as a substrate and microorganism cells to be present in an aqueous medium, thereby performing peptide-degrading reaction, and then determining the amount of the remaining peptide by a known method, e.g., HPLC analysis.

The above peptidases may be any proteins having peptide-degrading activity. Preferred are proteins having high dipeptide-hydrolyzing activity. More preferred are dipeptidases.

Examples of peptidases include: those existing in Escherichia coli such as PepA having the amino acid sequence shown in SEQ ID NO: 1, PepB having the amino acid sequence shown in SEQ ID NO: 2, PepD having the amino acid sequence shown in SEQ ID NO: 3, PepN having the amino acid sequence shown in SEQ ID NO: 4, PepP [GenBank accession No. (hereinafter abbreviated as Genbank) AAC75946], PepQ (GenBank AAC76850), PepE (GenBank AAC76991), PepT (GenBank AAC74211), Dcp (GenBank AAC74611) and IadA (GenBank AAC77284); those existing in Bacillus subtilis such as AmpS (GenBank AF012285), PepT (GenBank X99339), YbaC (GenBank Z99104), YcdD (GenBank Z99105), YjbG (GenBank Z99110), YkvY (GenBank Z99111), YqjE (GenBank Z99116) and YwaD (GenBank Z99123); those existing in Corynebacterium glutamicum such as proteins having the amino acid sequences represented by BAB97732, BAB97858, BAB98080, BAB98880, BAB98892, BAB99013, BAB99598 and BAB99819 (registration Nos. of DNA Data Bank of Japan); and those existing in Saccharomyces cerevisiae such as OCT1 (GenBank NC_001143), SPC2 (GenBank NC_003143), SPY2 [Saccharomyces genome database (http://www.yeastgenome.org/) accession No. L0002875] and YIM1 (GenBank NC_001145). Examples of dipeptidases include PepA, PepB, PepD and PepN having the amino acid sequences shown in SEQ ID NOS: 1 to 4, PepQ, PepE and IadA. Proteins having amino acid sequences which have 80% or more, preferably 90% or more, more preferably 95% or more homology to the amino acid sequence shown in any of SEQ ID NOS: 1 to 4 and having peptidase activity are also included in the proteins having high dipeptide-degrading activity.

The homology among amino acid sequences and nucleotide sequences can be determined by using algorithm BLAST by Karlin and Altschul [Proc. Natl. Acad. Sci. USA, 90, 5873 (1993)] and FASTA [Methods Enzymol., 183, 63 (1990)]. On the basis of the algorithm BLAST, programs such as BLASTN and BLASTX have been developed [J. Mol. Biol., 215, 403 (1990)]. When a nucleotide sequence is analyzed by BLASTN on the basis of BLAST, the parameters, for instance, are as follows: score=100 and wordlength=12. When an amino acid sequence is analyzed by BLASTX on the basis of BLAST, the parameters, for instance, are as follows: score=50 and wordlength=3. When BLAST and Gapped BLAST programs are used, default parameters of each program are used. The specific techniques for these analyses are known (http://www.ncbi.nlm.nih.gov.).

The expression "the activity of a peptide-transporting protein is reduced" means that the peptide-transporting activity is reduced compared with a peptide-transporting protein having none of the above deletions, substitutions and additions of nucleotides.

The peptide-transporting activity of a microorganism can be measured by allowing a peptide as a substrate and microorganism cells to be present in an aqueous medium, thereby performing peptide-transporting reaction, and then determining the amount of the remaining peptide by a known method, e.g., HPLC analysis:

The above peptide-transporting proteins may be any proteins involved in peptide transporation of microorganisms, for example, proteins encoded by genes forming an operon on chromosomal DNA which form a complex on cell membrane to express dipeptide-transporting activity and those which have peptide-transporting activity as individual proteins. Preferred are proteins having high peptide-transporting activity.

Examples of the peptide-transporting proteins include: those existing in Escherichia coli such as DppA having the amino acid sequence shown in SEQ ID NO: 5, DppB having the amino acid sequence shown in SEQ ID NO: 6, DppC having the amino acid sequence shown in SEQ ID NO: 7, DppD having the amino acid sequence shown in SEQ ID NO: 8, DppF having the amino acid sequence shown in SEQ ID NO: 9, OppA (GenBank AAC76569), OppB (GenBank AAC76568), OppC (GenBank AAC76567), OppD (GenBank AAC76566), OppF (GenBank AAC76565), YddO (GenBank AAC74556), YddP (GenBank AAC74557), YddQ (GenBank AAC74558), YddR (GenBank AAC74559), YddS (GenBank AAC74560), YbiK (GenBank AAC73915), MppA (GenBank AAC74411), SapA (GenBank AAC74376), SapB (GenBank AAC74375), SapC (GenBank AAC74374), SapD (GenBank AAC74373) and SapF (GenBank AAC74372); those existing in Bacillus subtilis such as DppA (GenBank CAA40002), DppB (GenBank CAA40003), DppC (GenBank CAA40004), DppD (GenBank CAA40005), DppE (GenBank CAA40006), OppA (GenBank CAA39787), OppB (GenBank CAA39788), OppC (GenBank CAA39789), OppD (GenBank CAA39790), OppF (GenBank CAA39791), AppA (GenBank CAA62358), AppB (GenBank CAA62359), AppC (GenBank CAA62360), AppD (GenBank CAA62356), AppF (GenBank CAA62357), YclF (GenBank CAB12175) and YkfD (GenBank CAB13157); those existing in Corynebacterium glutamicum such as proteins having the amino acid sequences represented by BAB99048, BAB99383, BAB99384, BAB99385, BAB99713, BAB99714, BAB99715, BAB99830, BAB99831 and BAB99832 (registration Nos. of DNA Data Bank of Japan); and those existing in Saccharomyces cerevisiae such as OPT1 (GenBank NP_012323), OPT2 (GenBank NP_015520) and PTR2 (GenBank CAA82172). Examples of the proteins having high peptide-transporting activity include DppA, DppB, DppC, DppD and DppF having the amino acid sequences shown in SEQ ID NOS: 5 to 9, and proteins having amino acid sequences which have 80% or more, preferably 90% or more, more preferably 95% or more homology to the amino acid sequence shown in any of SEQ ID NOS: 5 to 9.

The homology among amino acid sequences can be determined by using programs such as BLAST and FASTA described above.

The microorganisms in which the activities of three or more kinds of peptidases are reduced or lost include microorganisms in which the activities of three or more arbitrary kinds of peptidases are reduced or lost provided that the microorganisms can normally grow, specifically, microorganisms in which the activities of preferably three to nine kinds, more preferably three to six kinds, further preferably three or four kinds of peptidases are reduced or lost.

Examples of peptidases include the above-described peptidases and dipeptidases existing in Escherichia coli, Bacillus subtilis, Corynebacterium glutamicum and Saccharomyces cerevisiae. Proteins consisting of amino acid sequences which have 80% or more, preferably 90% or more, more preferably 95% or more homology to the amino acid sequence shown in any of SEQ ID NOS: 1 to 4 and having peptidase activity are also included in the proteins having high dipeptide-degrading activity.

The homology among amino acid sequences can be determined by using programs such as BLAST and FASTA described above.

There is not any specific restriction as to the microorganism having the ability to produce a dipeptide so long as it has the ability to produce a dipeptide. Suitable microorganisms include microorganisms producing proteins having the activity to synthesize a dipeptide by condensation and ligation of one or more kinds of amino acids, microorganisms producing proteins having the activity to synthesize a dipeptide from an L-amino acid ester and an L-amino acid, and microorganisms producing proteins having the activity to synthesize a dipeptide from an L-amino acid amide and an L-amino acid.

The microorganisms producing proteins having the activity to synthesize a dipeptide by condensation and ligation of one or more kinds of amino acids include microorganisms producing a protein selected from the group consisting of NRPS, D-Ala-D-Ala ligase and bacilysin synthetase.

Examples of the microorganisms producing NRPS include procaryotes such as microorganisms of the genus Bacillus, eucaryotes such as microorganisms of the genus Penicillium, microorganisms producing BacA, BacB and BacC (GenBank AF007865), microorganisms producing TycA, TycB and TycC (GenBank AF004835), microorganisms producing PcbAB (GenBank M57425), and microorganisms producing a protein having an amino acid sequence which has 80% or more, preferably 90% or more, more preferably 95% or more homology to the amino acid sequence of any protein selected from BacA, BacB, BacC, TycA, TycB, TycC and PcbAB and having NRPS activity.

Examples of the microorganisms producing D-Ala-D-Ala ligase include procaryotes forming peptidoglycans, microorganisms producing DdlA (GenBank accession No. M58467), microorganisms producing DdlB (GenBank accession No. AE000118), microorganisms producing DdlC (GenBank accession No. D88151), and microorganisms producing a protein consisting of an amino acid sequence wherein one or more amino acid residues are deleted, substituted or added in the amino acid sequence of any protein selected from DdlA, DdlB and DdlC and having D-Ala-D-Ala ligase activity.

The homology among amino acid sequences can be determined by using programs such as BLAST and FASTA described above.

Examples of the microorganisms producing bacilysin synthetase include microorganisms belonging to the genus Bacillus, preferably, Bacillus subtilis, Bacillus amyloliquefaciens, Bacillus coagulans, Bacillus licheniformis, Bacillus megaterium and Bacillus pumilus, and microorganisms producing a protein selected from the following [1] to [4]:
[1] a protein having the amino acid sequence shown in any of SEQ ID NOS: 19 to 25 and 68;
[2] a protein consisting of an amino acid sequence wherein one or more amino acid residues are deleted, substituted or added in the amino acid sequence shown in any of SEQ ID NOS: 19 to 25 and 68 and having the activity to synthesize a dipeptide;
[3] a protein consisting of an amino acid sequence which has 65% or more homology to the amino acid sequence shown in any of SEQ ID NOS: 19 to 25 and 68 and having the activity to synthesize a dipeptide; and
[4] a protein having an amino acid sequence which has 80% or more homology to the amino acid sequence shown in SEQ ID NO: 33 and having the activity to synthesize a dipeptide.

The microorganisms producing proteins having the activity to synthesize a dipeptide from an L-amino acid ester and an L-amino acid include microorganisms producing proline iminopeptidase, specifically, those belonging to the genera Bacillus, Corynebacterium and Pseudomonas. Examples of such microorganisms are Bacillus subtilis ATCC 6633, Bacillus coagulans EK01 [J. Bacteriol., 174, 7919 (1992)], Corynebacterium glutamicum ATCC 13286, Pseudomonas putida AJ-2402 (FERM BP-8101), Pseudomonas putida ATCC 12633 and Pseudomonas putida AJ-2048 (FERM BP-8123) (microorganisms described in WO03/010307). The microorganisms producing proline iminopeptidase also include Arthrobacter nicotianae [FEMS Microbiol. Lett., 78, 191 (1999)], Escherichia coli (Japanese Published Unexamined Patent Application No. 113887/90), Flavobacterium meningosepticum [Arch. Biochem. Biophys., 336, 35 (1996)], Hafnia alvei [J. Biochem., 119, 468 (1996)], Lactobacillus delbrueckii [Microbiology, 140, 527 (1994)], Bacillus coagulans [J. Bacteriol., 174, 7919 (1994)], Aeromonas sobria [J. Biochem., 116, 818 (1994)], Xanthomonas campestris (Japanese Published Unexamined Patent Application No. 121860/97), Neisseria gonorrhoeae [Mol. Microbiol., 9, 1203 (1993)], Propionibacterium freudenreichii [Appl. Environ. Microbiol., 64, 4736 (1998)], Serratia marcescens [J. Biochem., 122, 601 (1997)], Corynebacterium variabilis [J. Appl. Microbiol., 90, 449 (2001)], Thermoplasma acidophilum [FEBS Lett., 398, 101 (1996)] and Pseudomonas aeruginosa [Nature, 406, 959 (2000)].

Further, the microorganisms producing proline iminopeptidase include microorganisms having the ability to produce a protein selected from the following [1] to [3]:
[1] proline iminopeptidase described in any of WO03/010307; FEMS Microbiol. Lett., 78, 191 (1999); Japanese Published Unexamined Patent Application No. 113887/90; Arch. Biochem. Biophys., 336, 35 (1996); J. Biochem., 119, 468 (1996); Microbiology, 140, 527 (1994); J. Bacteriol., 174, 7919 (1994); J. Biochem., 116, 818 (1994); Japanese Published Unexamined Patent Application No. 121860/97; Mol. Microbiol., 9, 1203 (1993); Appl. Environ. Microbiol., 64, 4736 (1998); J. Biochem., 122, 601 (1997); FEBS Lett., 398, 101 (1996); and Nature, 406, 959 (2000);
[2] a protein consisting of an amino acid sequence wherein one or more amino acid residues are deleted, substituted or added in the amino acid sequence of any proline iminopeptidase of the above [1] and having proline iminopeptidase activity; and
[3] a protein consisting of an amino acid sequence which has 80% or more homology to the amino acid sequence of any proline iminopeptidase of the above [1] and having proline iminopeptidase activity.

The microorganisms producing proteins having the activity to synthesize a dipeptide from an L-amino acid amide and an L-amino acid include microorganisms producing L-amino acid amide hydrolase, specifically, those belonging to the genera Bacillus, Corynebacterium, Erwinia, Rhodococcus, Chryseobacterium, Micrococcus, Pseudomonas, Cryptococcus, Trichosporon, Rhodosporidium, Sporobolomyces, Tremella, Torulaspora, Sterigmatomyces and Rhodotolura. Preferred are microorganisms belonging to the genera Bacillus, Corynebacterium and Pseudomonas. More preferred examples are Bacillus megaterium AJ3284 (FERM BP-8090), Corynebacterium glutamicum ATCC 13286, Micrococcus luteus ATCC 9341 and Pseudomonas saccharophila ATCC 15946 (microorganisms described in WO03/010187).

The microorganisms producing proteins having L-amino acid amide hydrolase activity include microorganisms having the ability to produce a protein of the following [1] or [2]:
[1] a protein consisting of an amino acid sequence wherein one or more amino acid residues are deleted, substituted or added in the amino acid sequence of L-amino acid amide hydrolase described in WO03/010187 and having L-amino acid amide hydrolase activity;
[2] a protein consisting of an amino acid sequence which has 80% or more homology to the amino acid sequence of L-amino acid amide hydrolase described in WO03/010187 and having L-amino acid amide hydrolase activity.

The above protein consisting of an amino acid sequence wherein one or more amino acid residues are deleted, substituted or added and having the activity to synthesize a dipeptide can be obtained, for example, by introducing a site-directed mutation into DNA encoding a protein selected from a protein consisting of the amino acid sequence shown in any of SEQ ID NOS: 19 to 25 and 68, a protein having proline iminopeptidase activity and a protein having L-amino acid amide hydrolase activity by site-directed mutagenesis described in Molecular Cloning, A Laboratory Manual, Second Edition, Cold Spring Harbor Laboratory Press (1989) (hereinafter referred to as Molecular Cloning, Second Edition); Current Protocols in Molecular Biology, John Wiley & Sons (1987-1997) (hereinafter referred to as Current Protocols in Molecular Biology); Nucleic Acids Research, 10, 6487 (1982); Proc. Natl. Acad. Sci. USA, 79, 6409 (1982); Gene, 34, 315 (1985); Nucleic Acids Research, 13, 4431 (1985); Proc. Natl. Acad. Sci. USA, 82, 488 (1985), etc.

The number of amino acid residues which are deleted, substituted or added is not specifically limited, but is within the range where deletion, substitution or addition is possible by known methods such as the above site-directed mutagenesis. The suitable number is 1 to dozens, preferably 1 to 20, more preferably 1 to 10, further preferably 1 to 5.

The expression "one or more amino acid residues are deleted, substituted or added in any of the amino acid sequences shown in SEQ ID NOS: 19 to 25 and 68 and the amino acid sequences of a protein having proline iminopeptidase activity and a protein having L-amino acid amide hydrolase activity" means that the amino acid sequence may contain deletion, substitution or addition of a single or plural amino acid residues at an arbitrary position therein.

Deletion, substitution and addition may be simultaneously contained in one sequence, and amino acids to be substituted or added may be either natural or not. Examples of the natural amino acids are L-alanine, L-asparagine, L-aspartic acid, L-arginine, L-glutamine, L-glutamic acid, glycine, L-histidine, L-isoleucine, L-leucine, L-lysine, L-methionine, L-phenylalanine, L-proline, L-serine, L-threonine, L-tryptophan, L-tyrosine, L-valine and L-cysteine.

The following are examples of the amino acids capable of mutual substitution. The amino acids in the same group can be mutually substituted.
- Group A:: leucine, isoleucine, norleucine, valine, norvaline, alanine, 2-aminobutanoic acid, methionine, O-methylserine, t-butylglycine, t-butylalanine, cyclohexylalanine
- Group B:: aspartic acid, glutamic acid, isoaspartic acid, isoglutamic acid, 2-aminoadipic acid, 2-aminosuberic acid
- Group C:: asparagine, glutamine
- Group D:: lysine, arginine, ornithine, 2,4-diaminobutanoic acid, 2,3-diaminopropionic acid
- Group E:: proline, 3-hydroxyproline, 4-hydroxyproline
- Group F:: serine, threonine, homoserine
- Group G:: phenylalanine, tyrosine

There is not any specific restriction as to the position where the above deletion, substitution or addition of one or more amino acid residues is introduced, so long as a protein having an amino acid sequence carrying the introduced mutation has the dipeptide-synthesizing activity. Suitable examples include amino acid residues which are not conserved in one or more amino acid sequences among the amino acid sequences shown in SEQ ID NOS: 19 to 25 and 68 when these sequences are compared.

The above proteins consisting of an amino acid sequence wherein one or more amino acid residues are deleted, substituted or added and having the activity to synthesize a dipeptide include a protein consisting of an amino acid sequence which has usually 65% or more, preferably 80% or more, more preferably 90% or more, particularly preferably 95% or more homology to the amino acid sequence shown in any of SEQ ID NOS: 19 to 25 and 68, and a protein consisting of an amino acid sequence which has usually 80% or more, preferably 90% or more, more preferably 95% or more homology to the amino acid sequence of proline iminopeptidase or L-amino acid amide hydrolase.

The homology among amino acid sequences and nucleotide sequences can be determined by using programs such as BLAST and FASTA described above.

The amino acid sequence shown in SEQ ID NO: 33 is a region conserved among the proteins having the amino acid sequences shown in SEQ ID NOS: 19 to 25 and is also a region corresponding to the consensus sequence of proteins having Ala-Ala ligase activity derived from various microorganisms.

Microorganisms producing a protein having an amino acid sequence which has 80% or more, preferably 90% or more, further preferably 95% or more homology to the amino acid sequence shown in SEQ ID NO: 33 and having the activity to synthesize a dipeptide are also included in the dipeptide-producing microorganisms.

In order that the protein having an amino acid sequence which has 80% or more, preferably 90% or more, further preferably 95% or more homology to the amino acid sequence shown in SEQ ID NO: 33 may have the activity to synthesize a dipeptide, it is desirable that the homology of its amino acid sequence to the amino acid sequence shown in any of SEQ ID NOS: 19 to 25 is at least 80% or more, usually 90% or more, and particularly 95% or more.

The homology among amino acid sequences can be determined by using programs such as BLAST and FASTA described above.

The microorganisms described herein also include microorganisms carrying a recombinant DNA obtained by ligating, to a vector DNA, DNA encoding a protein having the activity to synthesize a dipeptide by condensation and ligation of one or more kinds of amino acids, DNA encoding a protein having the activity to synthesize a dipeptide from an L-amino acid ester and an L-amino acid, or DNA encoding a protein having the activity to synthesize a dipeptide from an L-amino acid amide and an L-amino acid.

Examples of the microorganisms include those belonging to the genera Escherichia, Bacillus, Corynebacterium and Saccharomyces.

The DNAs encoding a protein having the activity to synthesize a dipeptide by condensation and ligation of one or more kinds of amino acids include DNAs encoding NRPS, D-Ala-D-Ala ligase or bacilysin synthetase.

Examples of the DNAs encoding NRPS include DNAs encoding a protein selected from the group consisting of BacA, BacB, BacC, TycA, TycB, TycC and PcbAB.

Examples of the DNAs encoding D-Ala-D-Ala ligase include DNAs encoding a protein selected from the group consisting of DdlA, DdlB and DdlC.

Examples of the DNAs encoding bacilysin synthetase include DNAs encoding proteins of the following [1] to [4]:
[1] a protein having the amino acid sequence shown in any of SEQ ID NOS: 19 to 25 and 68;
[2] a protein consisting of an amino acid sequence wherein one or more amino acid residues are deleted, substituted or added in the amino acid sequence shown in any of SEQ ID NOS: 19 to 25 and 68 and having the activity to synthesize a dipeptide;
[3] a protein consisting of an amino acid sequence which has 65% or more homology to the amino acid sequence shown in any of SEQ ID NOS: 19 to 25 and 68 and having the activity to synthesize a dipeptide; and
[4] a protein having an amino acid sequence which has 80% or more homology to the amino acid sequence shown in SEQ ID NO: 33 and having the activity to synthesize a dipeptide;
and DNAs of the following [5] to [7]:
[5] DNA having the nucleotide sequence shown in any of SEQ ID NOS: 26 to 32, 64 and 65;
[6] DNA which hybridizes with DNA having the nucleotide sequence shown in any of SEQ ID NOS: 26 to 32, 64 and 65 under stringent conditions and which encodes a protein having the activity to synthesize a dipeptide; and
[7] DNA having a nucleotide sequence which has 80% or more homology to the nucleotide sequence shown in SEQ ID NO: 34 and encoding a protein having the activity to synthesize a dipeptide.

Examples of the DNAs encoding a protein having the activity to synthesize a dipeptide from an L-amino acid ester and an L-amino acid include DNAs encoding proteins of the following [1] to [3]:
[1] proline iminopeptidase described in any of WO03/010307; FEMS Microbiol. Lett., 78, 191 (1999); Japanese Published Unexamined Patent Application No. 113887/90; Arch. Biochem. Biophys., 336, 35 (1996); J. Biochem., 119, 468 (1996); Microbiology, 140, 527 (1994); J. Bacteriol., 174, 7919 (1994); J. Biochem., 116, 818 (1994); Japanese Published Unexamined Patent Application No. 121860/97; Mol. Microbiol., 9, 1203 (1993); Appl. Environ. Microbiol., 64, 4736 (1998); J. Biochem., 122, 601 (1997); FEBS Lett., 398, 101 (1996); and Nature, 406, 959 (2000);
[2] a protein consisting of an amino acid sequence wherein one or more amino acid residues are deleted, substituted or added in the amino acid sequence of any proline iminopeptidase of the above [1] and having proline iminopeptidase activity; and
[3] a protein consisting of an amino acid sequence which has 80% or more homology to the amino acid sequence of any proline iminopeptidase of the above [1] and having proline iminopeptidase activity;
and DNAs of the following [4] and [5]:
[4] DNA encoding proline iminopeptidase and having the nucleotide sequence described in any of WO03/010307; FEMS Microbiol. Lett., 78, 191 (1999); Japanese Published Unexamined Patent Application No. 113887/90; Arch. Biochem. Biophys., 336, 35 (1996); J. Biochem., 119, 468 (1996); Microbiology, 140, 527 (1994); J. Bacteriol., 174, 7919 (1994); J. Biochem., 116, 818 (1994); Japanese Published Unexamined Patent Application No. 121860/97; Mol. Microbiol., 9, 1203 (1993); Appl. Environ. Microbiol., 64, 4736 (1998); J. Biochem., 122, 601 (1997); FEBS Lett., 398, 101 (1996); and Nature, 406, 959 (2000); and
[5] DNA which hybridizes with any DNA encoding proline iminopeptidase of the above [4] under stringent conditions and which encodes a protein having proline iminopeptidase activity.

Examples of the DNAs encoding a protein having the activity to synthesize a dipeptide from an L-amino acid amide and an L-amino acid include DNAs encoding proteins of the following [1] and [2]:
[1] a protein consisting of an amino acid sequence wherein one or more amino acid residues are deleted, substituted or added in the amino acid sequence of L-amino acid amide hydrolase described in WO03/010187 and having L-amino acid amide hydrolase activity; and
[2] a protein consisting of an amino acid sequence which has 80% or more homology to the amino acid sequence of L-amino acid amide hydrolase described in WO03/010187 and having L-amino acid amide hydrolase activity;
and DNAs of the following [3] and [4]:
[3] DNA having the nucleotide sequence described in WO03/010187 and encoding L-amino acid amide hydrolase; and
[4] DNA which hybridizes with DNA consisting of the nucleotide sequence described in WO03/010187 and encoding L-amino acid amide hydrolase under stringent conditions and which encodes a protein having L-amino acid amide hydrolase activity.

The above DNA capable of hybridization under stringent conditions refers to DNA which is obtained by colony hybridization, plaque hybridization, Southern blot hybridization, or the like using a part or the whole of any of the above DNAs as a probe. A specific example of such DNA is DNA which can be identified by performing hybridization at 65°C in the presence of 0.7 to 1.0 mol/l, preferably 0.9 mol/l sodium chloride using a filter with colony- or plaque-derived DNA immobilized thereon, and then washing the filter at 65°C with a 0.1 to 2-fold conc., preferably 0.1-fold conc. SSC solution (1-fold conc. SSC solution: 150 mmol/l sodium chloride and 15 mmol/l sodium citrate). Hybridization can be carried out according to the methods described in Molecular Cloning, Second Edition; Current Protocols in Molecular Biology; DNA Cloning 1: Core Techniques, A Practical Approach, Second Edition, Oxford University (1995), etc. Specifically, the hybridizable DNA includes DNA having at least 80% or more homology, preferably 90% or more homology, more preferably 95% or more homology to the nucleotide sequence of any of the above DNAs as calculated by use of a program such as BLAST or FASTA described above based on the above parameters.

It is possible to confirm that the DNA hybridizing with the above DNA under stringent conditions is DNA encoding a protein having the activity to synthesize a dipeptide in the following manner. That is, a recombinant DNA expressing the DNA is prepared and the recombinant DNA is introduced into a host cell to obtain a microorganism to be used as an enzyme source. Then, 1) the enzyme source and one or more kinds of amino acids are allowed to be present in an aqueous medium, followed by HPLC analysis or the like to know whether a dipeptide is formed and accumulated in the aqueous medium, 2) the enzyme source, an L-amino acid ester and an L-amino acid are allowed to be present in an aqueous medium, followed by HPLC analysis or the like to know whether a dipeptide is formed and accumulated in the aqueous medium, or 3) the enzyme source, an L-amino acid amide and an L-amino acid are allowed to be present in an aqueous medium, followed by HPLC analysis or the like to know whether a dipeptide is formed and accumulated in the aqueous medium.

The homology among nucleotide sequences can be determined by using programs such as BLAST and FASTA described above.

### 2. Methods for Preparing the Microorganisms

The microorganisms decribed herein can be obtained by any of the following methods: 1) methods of imparting the dipeptide-producing ability to microorganisms in which the functions of one or more kinds of peptidases and one or more kinds of proteins having peptide-transporting activity are reduced or lost, or microorganisms in which the functions of three or more kinds of peptidases are reduced or lost; and 2) methods of reducing or causing loss of the functions of a) one or more kinds of peptidases and one or more kinds of peptide-transporting proteins or b) three or more kinds of peptidases of microorganisms having the ability to produce a dipeptide.

### (1) Methods of imparting the dipeptide-producing ability to microorganisms in which the functions of one or more kinds of peptidases and one or more kinds of peptide-transporting proteins are reduced or lost, or microorganisms in which the functions of three or more kinds of peptidases are reduced or lost

### (i) Preparation of microorganisms in which the activities of peptidases and peptide-transporting proteins are reduced or lost

The microorganisms in which the activities of peptidases and peptide-transporting proteins are reduced or lost may be obtained by any method capable of preparing such microorganisms. For example, they can be obtained by introducing a deletion, substitution or addition of a nucleotide into peptidase genes and genes encoding peptide-transporting proteins on chromosomal DNAs of microorganisms as described below.

The methods for introducing a deletion, substitution or addition of a nucleotide into a gene on the chromosomal DNA of a microorganism include methods utilizing homologous recombination. An example of the methods utilizing general homologous recombination is a method using a plasmid for homologous recombination prepared by ligating a mutant gene having an introduced nucleotide deletion, substitution or addition with a plasmid DNA incapable of autonomous replication in a host cell into which the nucleotide deletion or the like is to be introduced and carrying a drug resistance gene.

The plasmid for homologous recombination is introduced into a host cell by an ordinary method, followed by selection of a transformant in which the plasmid for homologous recombination has been integrated into the chromosomal DNA by homologous recombination using the drug resistance as a marker. The obtained transformant is cultured using a medium which does not contain the drug for several hours to one day, and then speread on an agar medium containing the drug and on an agar medium without the drug. By selecting a strain which does not grow on the former medium but can grow on the latter medium, the strain in which second homologous recombination occurred on the chromosomal DNA can be obtained. Introduction of a nucleotide deletion, substitution or addition into a desired gene on the chromosomal DNA can be confirmed by determining the nucleotide sequence of a region of the chromosomal DNA containing the gene into which the deletion or the like has been introduced.

By use of the above method, a nucleotide deletion, substitution or addition can be introduced into desired genes on chromosomal DNAs of microorganisms such as those belonging to the genera Escherichia, Bacillus, Corynebacterium and Saccharomyces.

Further, a nucleotide deletion, substitution or addition can be efficiently introduced into plural genes by utilizing homologous recombination according to a method using a linear DNA.

Specifically, a linear DNA containing a gene into which a nucleotide deletion, substitution or addition is to be introduced is incorporated into a cell to cause homologous recombination between chromosomal DNA and the introduced linear DNA. This method is applicable to any microorganisms capable of efficiently incorporating a linear DNA. Preferred microorganisms are those belonging to the genera Escherichia and Bacillus. Escherichia coli is more preferred, and Escherichia coli expressing a group of recombinant proteins derived from *λ* phage (Red recombination system) is further preferred.

An example of Escherichia coli expressing *λ* Red recombination system is Escherichia coli JM101 carrying pKD46, which is a plasmid DNA comprising a *λ* Red recombination system gene (available from Escherichia coli Genetic Stock Center, Yale University, U.S.A.)

Examples of the DNAs useful for homologous recombination are as follows:
(a) linear DNA in which DNAs present on the outside of a region of chromosomal DNA to be subjected to introduction of a nucleotide deletion, substitution or addition or DNAs having homology to the DNAs are present at both termini of a drug resistance gene;
(b) linear DNA in which DNAs present on the outside of a region of chromosomal DNA to be subjected to introduction of a nucleotide deletion, substitution or addition or DNAs having homology to the DNAs are directly ligated with each other;
(c) linear DNA in which DNAs present on the outside of a region of chromosomal DNA to be subjected to introduction of a nucleotide deletion, substitution or addition or DNAs having homology to the DNAs are present at both termini of a drug resistance gene and a gene that can be used for negative selection; and
(d) linear DNA of the above (a) in which a nucleotide sequence recognized by yeast-derived Flp recombinase [Proc. Natl. Acad. Sci. USA., 82, 5875 (1985)] is additionally present between the drug resistance gene and DNAs present on the outside of a region of chromosomal DNA or DNAs having homology to the DNAs.

As the drug resistance gene, any drug resistance genes that impart resistance to a drug to which the host microorganism shows sensitivity can be used. When Escherichia coli is used as the host microorganism, examples of the drug resistance genes are kanamycin resistance gene, chloramphenicol resistance gene, gentamicin resistance gene, spectinomycin resistance gene, tetracycline resistance gene and ampicillin resistance gene.

The "gene that can be used for negative selection" refers to a gene that is fatal to a host microorganism under certain culture conditions when the gene is expressed in the host microorganism. Examples of the genes are sacB gene derived from a microorganism belonging to the genus Bacillus [Appl. Environ. Microbiol., 59, 1361-1366 (1993)] and rpsL gene derived from a microorganism belonging to the genus Escherichia [Genomics, 72, 99-104 (2001)].

The DNAs present on the outside of a region of chromosomal DNA to be subjected to introduction of a substitution or deletion or DNAs having homology to the DNAs in the above linear DNAs are located in the same direction as that on the chromosomal DNA, and their length is preferably about 10 bp to 100 bp, more preferably about 20 bp to 50 bp, and further preferably about 30 bp to 40 bp.

The nucleotide sequence recognized by yeast-derived Flp recombinase is not specifically limited so long as it is a nucleotide sequence recognized by the said protein and catalyzing homologous recombination. Preferred examples are DNA having the nucleotide sequence shown in SEQ ID NO: 39, and DNA having a nucleotide sequence wherein one to several nucleotides are deleted, substituted or added in the said DNA and having a nucleotide sequence recognized by yeast-derived Flp recombinase and catalyzing homologous recombination.

The "DNA having homology" refers to DNA having such a degree of identity that allows occurrence of homologous recombination between the subject region of chromosomal DNA and the above linear DNA, specifically, DNA having 80% or more homology, preferably 90% or more homology, more preferably 95% or more homology, further preferably 100% homology.

The homology among nucleotide sequences can be determined by using programs such as BLAST and FASTA described above.

The above linear DNA fragments can be prepared by PCR. The desired linear DNA can also be obtained by constructing DNA containing the above linear DNA on plasmid and then carrying out treatment with restriction enzymes.

Examples of the methods for introducing a nucleotide deletion, substitution or addition into the chromosomal DNA of a microorganism include the following Methods 1 to 4.

### Method 1:

A method which comprises introducing the linear DNA of the above (a) or (d) into a host microorganism and selecting a transformant carrying the linear DNA inserted on its chromosomal DNA by homologous recombination using the drug resistance as a marker.

### Method 2:

A method which comprises introducing the DNA, in which DNAs present on the outside of a region of chromosomal DNA to be subjected to introduction of a nucleotide deletion, substitution or addition or DNAs having homology to the DNAs are directly ligated with each other, into the transformant obtained according to the above Method 1 and eliminating the drug resistance gene inserted on its chromosomal DNA according to the method to substitute or delete a region of the chromosomal DNA of the microorganism.

### Method 3:

A method which comprises:
[1] introducing the linear DNA of the above (c) into a host microorganism and selecting a transformant carrying the linear DNA inserted on its chromosomal DNA by homologous recombination using the drug resistance as a marker;
[2] synthesizing DNA by ligating DNAs having homology to the DNAs present on the outside of a region of chromosomal DNA to be subjected to introduction of a substitution or deletion in the same direction as that on the chromosomal DNA, and introducing the synthesized DNA into the transformant obtained in the above [1]; and
[3] culturing the transformant subjected to the operation of the above [2] under conditions such that the gene that can be used for negative selection is expressed, and selecting a strain capable of growing by the culturing as a strain in which the drug resistance gene and the gene that can be used for negative selection are eliminated from the chromosomal DNA.

### Method 4:

A method which comprises:
[1] introducing the linear DNA of the above (d) into a host microorganism and selecting a transformant carrying the linear DNA inserted on its chromosomal DNA by homologous recombination using the drug resistance as a marker; and
[2] introducing a Flp recombinase gene expression plasmid into the transformant obtained in the above [1], and after expression of the gene, obtaining a strain sensitive to the drug used in the above [1].

In the above methods, introduction of the linear DNA into a host microorganism can be carried out by any of the methods for introducing DNA into the microorganism, for example, the method using calcium ion [Proc. Natl. Acad. Sci. USA, 69, 2110 (1972)], the protoplast method (Japanese Published Unexamined Patent Application No. 248394/88) and electroporation [Nucleic Acids Res., 16, 6127 (1988)].

By using a DNA in which an arbitrary gene to be inserted to chromosomal DNA is incorporated in the center part of the DNA used in Method 2 or Method 3 [2], it is possible to eliminate the drug resistance gene and at the same time to insert an arbitrary gene to the chromosomal DNA.

The above Methods 2 to 4 are methods that leave no foreign genes such as a drug resistance gene and a gene usable for negative selection on the chromosomal DNA of the transformant to be finally obtained. Therefore, it is possible to readily produce a microorganism having nucleotide deletions, substitutions or additions in two or more different regions of the chromosomal DNA by repeating the operations of Method 2, Method 3 [1] to [3], and Method 4 [1] and [2] using the same drug resistance gene and the same gene usable for negative selection.

### (ii) Methods of imparting the dipeptide-producing ability

The methods of imparting the dipeptide-producing ability to the microorganisms used in the above (i) include the following methods.

### (a) Preparation of DNA encoding a protein having the activity to synthesize a dipeptide

The above DNA encoding a protein having the activity to synthesize a dipeptide can be obtained by the methods described below utiliting nucleotide sequence information on the DNA.

For example, the DNA encoding bacilysin synthetase can be obtained by Southern hybridization of a chromosomal DNA library from a microorganism belonging to the genus Bacillus using a probe designed based on the nucleotide sequence shown in any of SEQ ID NOS: 26 to 32, 64 and 65, or by PCR [PCR Protocols, Academic Press (1990)] using primer DNAs designed based on the nucleotide sequence shown in any of SEQ ID NOS: 26 to 32, 64 and 65 and, as a template, the chromosomal DNA of a microorganism belonging to the genus Bacillus.

The DNA encoding a protein having the activity to synthesize a dipeptide can also be obtained by conducting a search through various gene sequence databases for a sequence having 65% or more homology, preferably 80% or more homology, more preferably 90% or more homology, further preferably 95% or more homology to the nucleotide sequence of DNA encoding the amino acid sequence shown in any of SEQ ID NOS: 19 to 25, 33 and 68, and obtaining the desired DNA, based on the nucleotide sequence obtained by the search, from a chromosomal DNA or cDNA library of an organism having the nucleotide sequence according to the above-described method.

The obtained DNA, as such or after cleavage with appropriate restriction enzymes, is inserted into a vector by a conventional method to obtain a recombinant DNA. A plasmid DNA is extracted from a transformant obtained by introducing the recombinant DNA into Escherichia coli. Then, the nucleotide sequence of the DNA can be determined by a conventional sequencing method such as the dideoxy method [Proc. Natl. Acad. Sci., USA, 74, 5463 (1977)] or by using a nucleotide sequencer such as 373A DNA Sequencer (Perkin-Elmer Corp.).

In cases where the obtained DNA is found to be a partial DNA by the analysis of nucleotide sequence, the full length DNA can be obtained by Southern hybridization of a chromosomal DNA library using the partial DNA as a probe.

It is also possible to prepare the desired DNA by chemical synthesis using a DNA synthesizer (e.g., Model 8905, PerSeptive Biosystems) based on the determined nucleotide sequence of the DNA.

Examples of the DNAs that can be obtained by the above-described method are DNAs having the nucleotide sequences shown in SEQ ID NOS: 26 to 32, 64 and 65.

Examples of the vectors for inserting the DNA include pBluescriptII KS(+) (Stratagene), pDIRECT [Nucleic Acids Res., 18, 6069 (1990)], pCR-Script Amp SK(+) (Stratagene), pT7 Blue (Novagen, Inc.), pCR II (Invitrogen Corp.) and pCR-TRAP (Genhunter Corp.).

Examples of Escherichia coli include Escherichia coli XL1-Blue, Escherichia coli XL2-Blue, Escherichia coli DH1, Escherichia coli MC1000, Escherichia coli KY3276, Escherichia coli W1485, Escherichia coli JM101, Escherichia coli JM109, Escherichia coli HB101, Escherichia coli No. 49, Escherichia coli W3110, Escherichia coli NY49, Escherichia coli MP347, Escherichia coli NM522 and Escherichia coli ME8415.

Introduction of the recombinant DNA can be carried out by any of the methods for introducing DNA into the above host cells, for example, the method using calcium ion [Proc. Natl. Acad. Sci. USA, 69, 2110 (1972)], the protoplast method (Japanese Published Unexamined Patent Application No. 248394/88) and electroporation [Nucleic Acids Res., 16, 6127 (1988)].

An example of the microorganism carrying the DNA encoding a protein having the dipeptide-synthesizing activity obtained by the above method is Escherichia coli NM522/pPE43, which is a microorganism carrying a recombinant DNA comprising DNA having the sequence shown in SEQ ID NO: 19 described below.

### (b) Production of a protein having the dipeptide-synthesizing activity

The protein having the dipeptide-synthesizing activity can be produced by expressing the DNA obtained by the methods described in the above (a) in host cells using the methods described in Molecular Cloning, Second Edition, Current Protocols in Molecular Biology, etc., for example, in the following manner.

On the basis of the DNA obtained by the methods described in the above (a), a DNA fragment of an appropriate length comprising a region encoding the protein having the dipeptide synthesizing-activity is prepared according to need. The productivity of the protein can be enhanced by replacing a nucleotide in the nucleotide sequence of the region encoding the protein so as to make a codon most suitable for the expression in a host cell.

The DNA fragment is inserted downstream of a promoter in an appropriate expression vector to prepare a recombinant DNA.

A transformant producing the protein having the dipeptide synthesizing-activity can be obtained by introducing the recombinant DNA into a host cell suited for the expression vector.

As the host cell, any microorganisms such as bacterial cells and yeast cells that are capable of expressing the desired gene can be used.

The expression vectors that can be employed are those capable of autonomous replication or integration into the chromosome in the above host cells and comprising a promoter at a position appropriate for the transcription of the DNA encoding the protein having the dipeptide synthesizing-activity.

When a procaryote such as a bacterium is used as the host cell, it is preferred that the recombinant DNA comprising the DNA encoding a protein having the dipeptide-synthesizing activity is a recombinant DNA which is capable of autonomous replication in the procaryote and which comprises a promoter, a ribosome binding sequence, the DNA encoding a protein having the dipeptide-synthesizing activity, and a transcription termination sequence. The recombinant DNA may further comprise a gene regulating the promoter.

Examples of suitable expression vectors are pBTrp2, pBTac1 and pBTac2 (products of Boehringer Mannheim GmbH), pHelix1 (Roche Diagnostics Corp.), pKK233-2 (Amersham Pharmacia Biotech), pSE280 (Invitrogen Corp.), pGEMEX-1 (Promega Corp.), pQE-8 (Qiagen, Inc.), pET-3 (Novagen, Inc.), pKYP10 (Japanese Published Unexamined Patent Application No. 110600/83), pKYP200 [Agric. Biol. Chem., 48, 669 (1984)], pLSA1 [Agric. Biol. Chem., 53, 277 (1989)], pGEL1 [Proc. Natl. Acad. Sci. USA, 82, 4306 (1985)], pBluescript II SK(+), pBluescript II KS(-) (Stratagene), pTrS30 [prepared from Escherichia coli JM109/pTrS30 (FERM BP-5407)], pTrS32 [prepared from Escherichia coli JM109/pTrS32 (FERM BP-5408)], pPAC31 (WO98/12343), pUC19 [Gene, 33, 103 (1985)], pSTV28 (Takara Shuzo Co., Ltd.), pUC118 (Takara Shuzo Co., Ltd.) and pPA1 (Japanese Published Unexamined Patent Application No. 233798/88).

As the promoter, any promoters capable of functioning in host cells such as Escherichia coli can be used. For example, promoters derived from Escherichia coli or phage, such as trp promoter (Pₜᵣₚ), lac promoter (P_{lac}), P_{L} promoter, P_{R} promoter and P_{SE} promoter, SPO1 promoter, SPO2 promoter and penP promoter can be used. Artificially designed and modified promoters such as a promoter in which two Pₜᵣₚs are combined in tandem, tac promoter, lacT7 promoter and letI promoter, etc. can also be used.

Also useful are promoters such as xylA promoter for the expression in bacteria belonging to the genus Bacillus [Appl. Microbiol. Biotechnol., 35, 594-599 (1991)] and P54-6 promoter for the expression in bacteria belonging to the genus Corynebacterium [Appl. Microbiol. Biotechnol., 53, 674-679 (2000)].

It is preferred to use a plasmid in which the distance between the Shine-Dalgarno sequence (ribosome binding sequence) and the initiation codon is adjusted to an appropriate length (e.g., 6 to 18 nucleotides).

In the recombinant DNA wherein the DNA encoding the protein having the dipeptide synthesizing-activity is ligated to an expression vector, the transcription termination sequence is not essential, but it is preferred to place the transcription termination sequence immediately downstream of the structural gene.

An example of such recombinant DNA is pPE43 described below.

Examples of procaryotes suitable for use as host cells include microorganisms belonging to the genera Escherichia, Bacillus and Corynebacterium. Specific examples are Escherichia coli XL1-Blue, Escherichia coli XL2-Blue, Escherichia coli DH1, Escherichia coli DH5*α*, Escherichia coli MC1000, Escherichia coli KY3276, Escherichia coli W1485, Escherichia coli JM101, Escherichia coli JM109, Escherichia coli HB101, Escherichia coli No. 49, Escherichia coli W3110, Escherichia coli NY49, Escherichia coli MP347, Escherichia coli NM522, Bacillus subtilis ATCC 33712, Bacillus megaterium, Bacillus sp. FERM BP-6030, Bacillus amyloliquefaciens, Bacillus coagulans, Bacillus licheniformis, Bacillus pumilus, Corynebacterium glutamicum ATCC 13032 and Corynebacterium glutamicum ATCC 14297.

Introduction of the recombinant DNA can be carried out by any of the methods for introducing DNA into the above host cells, for example, the method using calcium ion [Proc. Natl. Acad. Sci. USA, 69, 2110 (1972)], the protoplast method (Japanese Published Unexamined Patent Application No. 248394/88) and electroporation [Nucleic Acids Res., 16, 6127 (1988)].

When a strain belonging to the genus Saccharomyces is used as the host cell, YEp13 (ATCC 37115), YEp24 (ATCC 37051), YCp50 (ATCC 37419), pHS19, pHS15, etc. can be used as the expression vector.

As the promoter, any promoters capable of functioning in strains belonging to the genus Saccharomyces can be used. Suitable promoters include PHO5 promoter, PGK promoter, GAP promoter, ADH promoter, gal 1 promoter, gal 10 promoter, heat shock polypeptide promoter, MFα1 promoter and CUP 1 promoter.

Examples of suitable host cells are strains belonging to the genus Saccharomyces, specifically, Saccharomyces cerevisiae.

Introduction of the recombinant DNA can be carried out by any of the methods for introducing DNA into yeast, for example, electroporation [Methods Enzymol., 194, 182 (1990)], the spheroplast method [Proc. Natl. Acad. Sci. USA, 81, 4889 (1984)] and the lithium acetate method [J. Bacteriol., 153, 163 (1983)].

### (2) Methods of reducing or causing loss of the functions of a) one or more kinds of peptidases and one or more kinds of peptide-transporting proteins or b) three or more kinds of peptidases of microorganisms having the ability to produce a dipeptide

Microorganisms having the ability to produce a dipeptide can be prepared by carrying out the methods described in the above (1) (ii) using arbitrary microorganisms as host cells. By carrying out the methods described in the above (1) (i) using the microorganisms thus prepared, microorganisms in which the functions of one or more kinds of peptidases and one or more kinds of peptide-transporting proteins or the functions of three or more kinds of peptidases are reduced or lost and which have the ability to produce a dipeptide can be prepared.

The microorganisms described herein can also be obtained by carrying out the methods described in the above (1) (i) using microorganisms inherently having the ability to produce a dipeptide.

Examples of the above microorganisms include those belonging to the genera Escherichia, Bacillus, Corynebacterium and Saccharomyces. Preferred are Escherichia coli, Bacillus subtilis, Corynebacterium glutamicum and Saccharomyces cerevisiae.

### 3. Process for Producing a Dipeptide

The production processes described herein include:
(i) a process for producing a dipeptide, which comprises:
   allowing an enzyme source and one or more kinds of amino acids to be present in an aqueous medium, said enzyme source being a culture of the microorganism described herein or a treated matter of the culture; allowing the dipeptide to form and accumulate in the aqueous medium;
   and recovering the dipeptide from the medium;
(ii) a process for producing a dipeptide, which comprises:
   allowing an enzyme source, an L-amino acid ester and an L-amino acid to be present in an aqueous medium, said enzyme source being a culture of the microorganism described herein or a treated matter of the culture; allowing the dipeptide to form and accumulate in the aqueous medium;
   and recovering the dipeptide from the medium; and
(iii) a process for producing a dipeptide, which comprises: allowing an enzyme source, an L-amino acid amide and an L-amino acid to be present in an aqueous medium, said enzyme source being a culture of the microorganism described herein or a treated matter of the culture; allowing the dipeptide to form and accumulate in the aqueous medium; and recovering the dipeptide from the medium.

One or more kinds, preferably one or two kinds of amino acids used as substrates in the above production process (i) are amino acids, preferably amino acid selected from the group consisting of L-amino acids, glycine (Gly) and *β*-alanine (*β*-Ala), and can be used in any combination. Examples of L-amino acids are L-alanine (L-Ala), L-glutamine (L-Gln), L-glutamic acid (L-Glu), L-valine (L-Val), L-leucine (L-Leu), L-isoleucine (L-Ile), L-proline (L-Pro), L-phenylalanine (L-Phe), L-tryptophan (L-Trp), L-methionine (L-Met), L-serine (L-Ser), L-threonine (L-Thr), L-cysteine (L-Cys), L-asparagine (L-Asn), L-tyrosine (L-Tyr), L-lysine (L-Lys), L-arginine (L-Arg), L-histidine (L-His), L-aspartic acid (L-Asp), L-α-aminobutyric acid (L-*α*-AB), L-azaserine, L-theanine, L-4-hydroxyproline (L-4-HYP), L-3-hydroxyproline (L-3-HYP), L-ornithine (L-Orn), L-citrulline (L-Cit) and L-6-diazo-5-oxo-norleucine.

The amino acids which are more preferably used in the above process (i) include the following: a combination of one kind of amino acid selected from the group consisting of L-Ala, Gly, L-Met, L-Ser, L-Thr and *β*-alma, and one kind of amino acid selected from the group consisting of L-Ala, L-Gln, L-Glu, Gly, L-Val, L-Leu, L-Ile, L-Pro, L-Phe, L-Trp, L-Met, L-Ser, L-Thr, L-Cys, L-Asn, L-Tyr, L-Lys, L-Arg, L-His, L-Asp, L-α-AB, *β*-Ala, L-azaserine, L-theanine, L-4-HYP, L-3-HYP, L-Orn, L-Cit and L-6-diazo-5-oxo-norleucine; a combination of L-Gln and L-Phe; and a combination of L-*α*-AB and L-Gln, L-Arg or L-*α* -AB. Further preferred amino acids are: a combination of L-Ala and one kind of amino acid selected from the group consisting of L-Ala, L-Gln, Gly, L-Val, L-Leu, L-Ile, L-Phe, L-Trp, L-Met, L-Ser, L-Thr, L-Cys, L-Asn, L-Tyr, L-Lys, L-Arg, L-His, L-*α*-AB, L-azaserine, L-Cit and L-theanine; a combination of Gly and one kind of amino acid selected from the group consisting of L-Gln, Gly, L-Phe, L-Trp, L-Met, L-Ser, L-Thr, L-Cys, L-Tyr, L-Lys, L-Arg, L-*α*-AB and L-Cit; a combination of L-Met and one kind of amino acid selected from the group consisting of L-Phe, L-Met, L-Ser, L-Thr, L-Cys, L-Tyr, L-Lys and L-His; a combination of L-Ser and one kind of amino acid selected from the group consisting of L-Gln, L-Phe, L-Ser, L-Thr, L-Tyr, L-His and L-*α*-AB; a combination of L-Thr and one kind of amino acid selected from the group consisting of L-Gln, L-Phe, L-Leu, L-Thr and L-*α*-AB; a combination of L-Gln and L-Phe; a combination of *β*-all and one kind of amino acid selected from the group consisting of L-Phe, L-Met, L-His and L-Cit; and a combination of L-*α*-AB and L-Gln, L-Arg or L-*α*-AB.

In the above production process (i), the L-amino acid used as a substrate is added to the aqueous medium at the start or in the course of reaction to give a concentration of 0.1 to 500 g/l, preferably 0.2 to 200 g/l.

The dipeptides produced by the above process (i) include the dipeptides represented by the following formula (I):

R¹ - R² (I)

(wherein R¹ and R², which may be the same or different, each represent an amino acid). Preferred dipeptides are those represented by the above formula (I) wherein R¹ and R², which may be the same or different, each represent an amino acid selected from the group consisting of L-Ala, L-Gln, L-Glu, Gly, L-Val, L-Leu, L-Ile, L-Pro, L-Phe, L-Trp, L-Met, L-Ser, L-Thr, L-Cys, L-Asn, L-Tyr, L-Lys, L-Arg, L-His, L-Asp, L-*α*-AB, *β*-Ala, L-azaserine, L-theanine, L-4-HYP, L-3-HYP, L-Orn and L-6-diazo-5-oxo-norleucine. More preferred are dipeptides wherein R¹ is L-Ala, Gly, L-Met, L-Ser, L-Thr or *β*-Ala, and R² is L-Gln, L-Glu, Gly, L-Val, L-Leu, L-Ile, L-Pro, L-Phe, L-Trp, L-Met, L-Ser, L-Thr, L-Cys, L-Asn, L-Tyr, L-Lys, L-Arg, L-His, L-Asp, L-*α*-AB, *β-*Ala, L-azaserine_{;} L-theanine, L-4-HYP, L-3-HYP, L-Orn or L-6-diazo-5-oxo-norleucine. Further preferred dipeptides are: dipeptides wherein R¹ is L-Ala and R² is L-Gln, Gly, L-Val, L-Leu, L-Ile, L-Phe, L-Trp, L-Met, L-Ser, L-Thr, L-Cys, L-Asn, L-Tyr, L-Lyls, L-Arg, L-His, L-α-AB, L-azaserine or L-theanine; dipeptides wherein R¹ is Gly and R² is L-Gln, Gly, L-Trp, L-Met, L-Ser, L-Thr, L-Cys, L-Tyr, L-Lys, L-Arg or L-*α*-AB; dipeptides wherein R¹ is L-Met and R² is L-Phe, L-Met, L-Cys, L-Tyr, L-Lys or L-His; dipeptides wherein R¹ is L-Ser and R² is L-Gln, Gly, L-Phe, L-Met, L-Ser, L-Thr, L-Tyr, L-His or L-*α*-AB; dipeptides wherein R¹ is L-Thr and R² is L-Gln, L-Gly, L-Phe, L-Met, L-Ser, L-Thr or L-*α*-AB; dipeptides wherein R¹ is L-Gln and R² is L-Phe or L-*α*-AB; a dipeptide wherein R¹ is L-Phe and R² is L-Gln; a dipeptide wherein R¹ is L-Trp and R² is Gly; dipeptides wherein R¹ is L-Cys and R² is L-Ala, L-Gln, Gly or L-Met; dipeptides wherein R¹ is L-Lys and R² is L-Ala, Gly or L-Met; a dipeptide wherein R¹ is L-Arg and R² is L-*α*-AB; a dipeptide wherein R¹ is L-His and R² is L-Met; and dipeptides wherein R¹ is L-*α*-AB and R² is L-Ala, L-Gln, Gly, L-Ser, L-Thr, L-Arg or L-*α*-AB.

Further, in the above process, compounds which can be metabolized by the microorganism described herein to produce ATP, for example, sugars such as glucose, alcohols such as ethanol, and organic acids such as acetic acid may be added, as ATP source, to the aqueous medium according to need.

The L-amino acid ester and L-amino acid used as substrates in the above production process (ii) may be any of L-amino acid esters and L-amino acids that can be used as substrates by the microorganism described herein to form a dipeptide, and they can be used in any combination. Preferably, the L-amino acid ester is selected from the group consisting of L-alanine ester, glycine ester, L-valine ester, L-isoleucine ester, L-methionine ester, L-phenylalanine ester, L-serine ester, L-threonine ester, L-glutamine ester, L-tyrosine ester, L-arginine ester, L-aspartic acid-*α*-ester, L-aspartic acid-*β*-ester, L-leucine ester, L-asparagine ester, L-lysine ester, L-aspartic acid-*α*,*β*-dimethyl ester and L-glutamine-*γ*-ester, and the L-amino acid is selected from the group consisting of L-Gln, L-Asn, Gly, L-Ala, L-Leu, L-Met, L-Pro, L-Phe, L-Trp, L-Ser, L-Thr, L-Tyr, L-Lys, L-Arg, L-His and L-Glu.

In the above process (ii), the L-amino acid ester and L-amino acid used as substrates are added to the aqueous medium at the start or in the course of reaction to give a concentration of 0.1 to 500 g/l, preferably 0.2 to 200 g/l.

The L-amino acid amide and L-amino acid used as substrates in the above production process (iii) may be any of L-amino acid amides and L-amino acids that can be used as substrates by the microorganism described herein to form a dipeptide, and they can be used in any combination. Preferably, the L-amino acid amide is selected from the group consisting of L-alanine amide, glycine amide and L-aspartic acid amide, and the L-amino acid is selected from the group consisting of L-Gln, L-Asn, Gly, L-Ala, L-Val, L-Leu, L-Ile, L-Met, L-Pro, L-Phe, L-Trp, L-Ser, L-Thr, L-Tyr, L-Lys, L-Arg, L-His and L-Glu.

In the above process (iii), the L-amino acid amide and L-amino acid used as substrates are added to the aqueous medium at the start or in the course of reaction to give a concentration of 0.1 to 500 g/l, preferably 0.2 to 200 g/l.

The aqueous medium used in the production processes described herein may comprise any components and may have any composition so far as the dipeptide-forming reaction is not inhibited. Suitable aqueous media include water and buffers such as phosphate buffer, carbonate buffer, acetate buffer, borate buffer, citrate buffer and Tris buffer. The aqueous medium may comprise alcohols such as methanol and ethanol, esters such as ethyl acetate, ketones such as acetone, and amides such as acetamide.

The dipeptide-forming reaction is carried out in the aqueous medium at pH 5 to 11, preferably pH 6 to 10, at 20 to 60°C, preferably 25 to 45°C, for 2 to 150 hours, preferably 6 to 120 hours.

If necessary, a surfactant or an organic solvent may further be added to the aqueous medium.

Any surfactant that promotes the formation of a dipeptide can be used. Suitable surfactants include nonionic surfactants such as polyoxyethylene octadecylamine (e.g., Nymeen S-215, NOF Corporation), cationic surfactants such as cetyltrimethylammonium bromide and alkyldimethylbenzylammonium chloride (e.g., Cation F2-40E, NOF Corporation), anionic surfactants such as lauroyl sarcosinate, and tertiary amines such as alkyldimethylamine (e.g., Tertiary Amine FB, NOF Corporation), which may be used alone or in combination. The surfactant is usually used at a concentration of 0.1 to 50 g/l. As the organic solvent, xylene, toluene, aliphatic alcohols, acetone, ethyl acetate, etc. may be used usually at a concentration of 0.1 to 50 ml/l.

The treated matters of the culture include concentrated culture, dried culture, cells obtained by centrifuging the culture, and products obtained by treating the cells by various means such as drying, freeze-drying, treatment with a surfactant, ultrasonication, mechanical friction, treatment with a solvent, enzymatic treatment and immobilization. The treated matters of the culture described herein also include crude extracts of protein obtained by removing insoluble matters and the like from the treated matters obtained by treating the above cells by means such as treatment with a surfactant, ultrasonication, mechanical friction, treatment with a solvent and enzymatic treatment.

When the culture or a treated matter of the culture is used as the enzyme source, the amount of the enzyme source to be added varies according to its specific activity, etc., but is, for example, 5 to 1000 mg, preferably 10 to 400 mg per mg of amino acid, L-amino acid ester or L-amino acid amide used as a substrate.

Recovery of the dipeptide formed and accumulated in the aqueous medium can be carried out by ordinary methods using active carbon, ion-exchange resins, etc. or by means such as extraction with an organic solvent, crystallization, thin layer chromatography and high performance liquid chromatography.

Further, the above production processes (ii) and (iii) can be carried out according to the descriptions in WO03/010189 or WO03/010187.

Experimental examples of the present invention are shown below.

### Experimental Example 1

### Construction of a Plasmid Expressing ywfE Gene Derived from Bacillus subtilis

A ywfE gene fragment of Bacillus subtilis was obtained in the following manner.

By using a DNA synthesizer (Model 8905, PerSeptive Biosystems, Inc.), DNAs having the nucleotide sequences shown in SEQ ID NOS: 35 and 36 (hereinafter referred to as primer A and primer B, respectively) were synthesized. Primer A has a nucleotide sequence containing a region wherein the initiation codon of ywfE gene (atg) is substituted by the NcoI recognition sequence (ccatgg). Primer B has a nucleotide sequence containing a region wherein the termination codon of ywfE gene is substituted by the BamHI recognition sequence (ggatcc).

PCR was carried out using the chromosomal DNA of Bacillus subtilis as a template and the above primer A and primer B as a set of primers. That is, PCR was carried out by 30 cycles, one cycle consisting of reaction at 94°C for one minute, reaction at 55°C for 2 minutes and reaction at 72°C for 3 minutes, using 40 *µ*l of a reaction mixture comprising 0.1 *µ*g of the chromosomal DNA, 0.5 *µ* mol/l each of the primers, 2.5 units of Pfu DNA polymerase, 4 *µ*l of buffer for Pfu DNA polymerase (10 x) and 200 *µ* mol/l each of dNTPs.

One-tenth of the resulting reaction mixture was subjected to agarose gel electrophoresis to confirm that a ca. 1.4 kb fragment corresponding to the ywfE gene fragment was amplified. Then, the remaining reaction mixture was mixed with an equal amount of phenol/chloroform saturated with TE. The resulting mixture was centrifuged, and the obtained upper layer was mixed with a two-fold volume of cold ethanol and allowed to stand at -80°C for 30 minutes. The resulting solution was centrifuged, and the obtained DNA precipitate was dissolved in 20 *µ*l of TE.

The thus obtained solution (5 *µ*l) was subjected to reaction to cleave the amplified DNA with restriction enzymes NcoI and BamHI. DNA fragments were separated by agarose gel electrophoresis, and a 1.4 kb DNA fragment containing ywfE gene was recovered using GENECLEAN II Kit (Bio 101).

C-Terminal His-tagged recombinant expression vector pQE60 (Qiagen, Inc.) (0.2 *µ*g) was cleaved with restriction enzymes NcoI and BamHI. DNA fragments were separated by agarose gel electrophoresis, and a 3.4 kb DNA fragment was recovered in the same manner as above.

The 1.4 kb DNA fragment containing ywfE gene and the 3.4 kb DNA fragment obtained above were subjected to ligation reaction using a ligation kit (Takara Shuzo Co., Ltd.) at 16°C for 16 hours.

Escherichia coli NM522 (Stratagene) was transformed using the ligation reaction mixture according to the method using calcium ion [Proc. Natl. Acad. Sci. USA, 69, 2110 (1972)], spread on LB agar medium containing 50 *µ* g/ml ampicillin, and cultured overnight at 30°C.

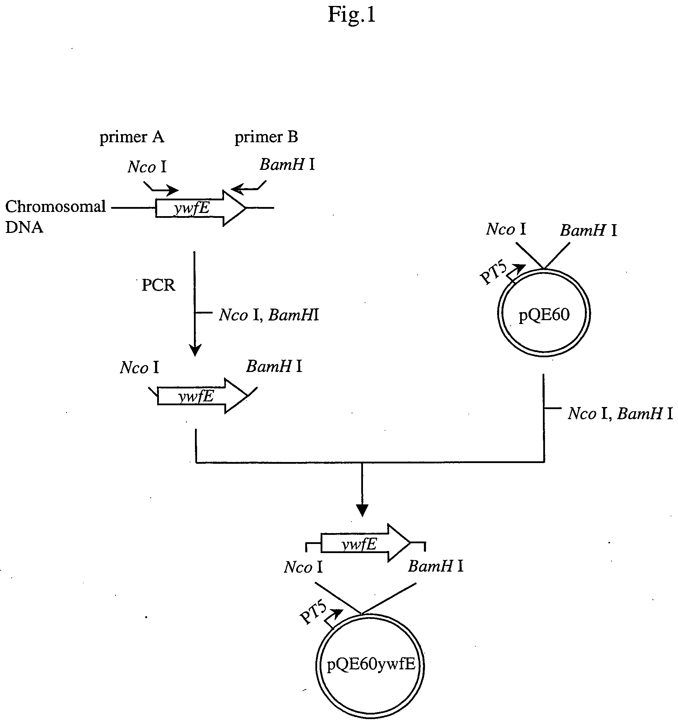

A plasmid was extracted from a colony of the transformant that grew on the medium according to a known method, whereby pQE60ywfE, which is a C-terminal His-tagged ywfE gene expression vector, was obtained. The structure of the vector was confirmed by digestion with restriction enzymes (Fig. 1).

### Experimental Example 2

### Acquisition of a ywfE Gene Product

Escherichia coli NM522/pQE60ywfE carrying pQE60ywfE was inoculated into 8 ml of LB medium containing 50 *µ*g/ml ampicillin in a test tube, and cultured at 28°C for 17 hours. The resulting culture was inoculated into 50 ml of LB medium containing 50 *µ*g/ml ampicillin in a 250-ml Erlenmeyer flask, and cultured at 30°C for 3 hours. Then, isopropyl-*β*-D-thiogalactopyranoside (IPTG) was added to give a final concentration of 1 mmol/l, followed by further culturing at 30°C for 4 hours. The resulting culture was centrifuged to obtain wet cells, and a His-tagged recombinant enzyme was purified from the wet cells using HisTrap (His-tagged protein purification kit, Amersham Pharmacia Biotech) according to the instructions attached thereto.

### Experimental Example 3

### Production of Dipeptides Using the His-Tagged Recombinant Enzyme (1)

(i) A reaction mixture (0.1 ml) comprising 0.04 mg of the purified His-tagged recombinant enzyme obtained in Experimental Example 2, 100 mmol/l Tris-HCl (pH 8.0), 60 mmol/l magnesium chloride, 60 mmol/l ATP, 30 mmol/l L-Ala and 30 mmol/l L-Gln was prepared, and reaction was carried out at 37°C for 16 hours.
   After the completion of reaction, the reaction product was derivatized by the dinitrophenol method and then analyzed by HPLC. The HPLC analysis was carried out using, as a separation column, Lichrosorb-RP-18 column (Kanto Kagaku) and, as an eluting solution, 1% (v/v) phosphoric acid and 25% (v/v) acetonitrile at a flow rate of 0.7 ml/min. As a result, it was confirmed that 3.7 g/l L-Ala-L-Gln and 0.3 g/l L-alanyl-L-alanine (L-Ala-L-Ala) were formed and accumulated in the reaction mixture.
(ii) Reactions were carried out under the same conditions as in the above (i) using reaction mixtures having the same composition as that of the reaction mixture of the above (i) except that 0.01 mg of the enzyme was used and L-Phe, L-Met, L-Leu and L-Val, respectively, were used in place of L-Gln.
   After the completion of reactions, the reaction products were analyzed in the same manner as in the above (i), whereby it was confirmed that the following dipeptides were formed and accumulated in the respective reaction mixtures: 7.0 g/l L-alanyl-L-phenylalanine (L-Ala-L-Phe) alone; 7.0 g/l L-alanyl-L-methionine (L-Ala-L-Met) and 0.03 g/l L-Ala-L-Ala; 5.0 g/l L-alanyl-L-leucine (L-Ala-L-Leu) and 0.2 g/l L-Ala-L-Ala; and 1.6 g/l L-alanyl-L-valine (L-Ala-L-Val) and 0.3 g/l L-Ala-L-Ala.
(iii) Reactions were carried out under the same conditions as in the above (i) using reaction mixtures having the same composition as that of the reaction mixture of the above (i) except that 0.01 mg of the enzyme was used, Gly was used in place of L-Ala, and L-Phe and L-Met, respectively, were used in place of L-Gln.

After the completion of reactions, the reaction products were analyzed in the same manner as in the above (i), whereby it was confirmed that 5.2 g/l glycyl-L-phenylalanine (Gly-L-Phe) and 1.1 g/l glycyl-L-methionine (Gly-L-Met) were formed and accumulated in the respective reaction mixtures.

When ATP was excluded from the compositions of the above reaction mixtures, no dipeptide was formed.

The above results revealed that the ywfE gene product has the activity to produce, in the presence of ATP, the following dipeptides: L-Ala-L-Gln plus L-Ala-L-Ala, L-Ala-L-Phe, L-Ala-L-Met plus L-Ala-L-Ala, L-Ala-L-Leu plus L-Ala-L-Ala, or L-Ala-L-Val plus L-Ala-L-Ala from L-Ala plus L-Gln, L-Phe, L-Met, L-Leu or L-Val; and Gly-L-Phe or Gly-L-Met from Gly plus L-Phe or L-Met.

### Experimental Example 4

### Production of Dipeptides Using the His-Tagged Recombinant Enzyme (2)

A reaction mixture (0.1 ml) comprising 0.04 mg of the purified His-tagged recombinant enzyme obtained in Experimental Example 2, 100 mmol/l Tris-HCl (pH 8.0), 60 mmol/l magnesium chloride and 60 mmol/l ATP was prepared. To this mixture were respectively added combinations of various L-amino acids, Gly and *β*-Ala selected from the amino acids shown in the first row of Table 1 and in the leftmost column of Table 1 to give a concentration of 30 mmol/l each, and the resulting mixtures were subjected to reaction at 37°C for 16 hours. After the completion of reactions, the reaction products were analyzed by HPLC, whereby it was confirmed that the dipeptides shown in Table 1 were formed.

**Table 1-1**

| | Ala | Gln | Glu | Gly | Val | Leu | Ile | Pro |
|---|---|---|---|---|---|---|---|---|
| Ala | AlaAla | AlaGln | AlaAla | AlaGly | AlaVal | AlaLeu | AlaIle | AlaAla |
| | | AlaAla | | AlaAla | AlaAla | AlaAla | AlaAla | |
| Gln | | × | × | GlyGln | × | × | × | × |
| | | | | GlyGly | | | | |
| Glu | | | | GlyGly | | | | |
| Gly | | | | GlyGly | | | | GlyGly |
| Val | | | | | | | | |
| Leu | | | | | | | | |
| Ile | | | | | | | | |
| Pro | | | | | | | | |
| Phe | | | | | | | | |
| Trp | | | | | | | | |
| Met | | | | | | | | |
| Ser | | | | | | | | |
| Thr | | | | | | | | |
| Cys | | | | | | | | |
| Asn | | | | | | | | |
| Tyr | | | | | | | | |
| Lys | | | | | | | | |
| Arg | | | | | | | | |
| His | | | | | | | | |
| Asp | | | | | | | | |
| *α* AB | | | | | | | | |
| *β*-Ala | | | | | | | | |
| Cit | | | | | | | | |

**Table 1-2**

| | Phe | Trp | Met | Ser | Thr | Cys | Asn | Tyr |
|---|---|---|---|---|---|---|---|---|
| Ala | AlaPhe | AlaTrp | AlaMet | AlaSer | AlaThr | AlaAla | AlaAsn | AlaTyr |
| | AlaAla | AlaAla | | AlaAla | AlaAla | ○ | AlaAla | AlaAla |
| Gln | ○ | × | MetMet | SerGln | ThrGln | ○ | × | × |
| | | | | SerSer | ThrThr | | | |
| Glu | | | | | | | | |
| Gly | GlyPhe | GlyGly | GlyMet | GlySer | GlyThr | GlyGly | GlyGly | GlyTyr |
| | | ○ | GlyGly | GlyGly | GlyGly | ○ | | GlyGly |
| | | | | SerGly | ThrGly | | | |
| | | | | SerSer | ThrThr | | | |
| Val | | | × | | | | | |
| Leu | | | MetMet | | ThrLeu | | | |
| Ile | | | MetMet | | | | | |
| Pro | | | MetMet | SerSer | ThrThr | | | |
| Phe | | | MetPhe | SerPhe | ThrPhe | | | |
| | | | MetMet | | ThrThr | | | |
| Trp | | | | | | | | |
| Met | | | MetMet | SerMet | ThrMet | MetMet | | MetTyr |
| | | | | | ThrThr | ○ | | MetMet |
| Ser | | | | SerSer | SerThr | | | SerTyr |
| | | | | | SerSer | | | SerSer |
| | | | | | ThrSer | | | |
| | | | | | ThrThr | | | |
| Thr | | | | | ThrThr | | | |
| Cys | | | | | | | | |
| Asn | | | | | | | | |
| Tyr | | | | | | | | |
| Lys | | | | | | | | |
| Arg | | | | | | | | |
| His | | | | | | | | |
| Asp | | | | | | | | |
| *α*-AB | | | | | | | | |
| *β*-Ala | | | | | | | | |
| Cit | | | | | | | | |

**Table 1-3**

| | Lys | Arg | His | Asp | *α*-AB | *β*-Ala | Cit | Azaserine | Theanine |
|---|---|---|---|---|---|---|---|---|---|
| Ala | AlaAla | AlaArg | AlaHis | AlaAla | AlaAla | | AlaAla | AlaAla | AlaAla |
| | ○ | AlaAla | AlaAla | | ○ | | ○ | ○ | ○ |
| Gln | × | × | × | × | ○ | | | | |
| Glu | | | | | | | | | |
| Gly | GlyGly | GlyArg | GlyGly | GlyGly | GlyGly | | ○ | | |
| | ○ | GlyGly | | | ○ | | | | |
| Val | | | | | | | | | |
| Leu | | | | | | | | | |
| Ile | | | | | | | | | |
| Pro | | | | | | | | | |
| Phe | | | | × | | ○ | | | |
| Trp | | | | | | | | | |
| Met | MetMet | | MetMet | | | ○ | | | |
| | ○ | | ○ | | | | | | |
| Ser | | | SerHis | | SerSer | | | | |
| | | | | | ○ | | | | |
| Thr | | | | | ThrThr | | | | |
| | | | | | ○ | | | | |
| Cys | | | | | | | | | |
| Asn | | | | | | | | | |
| Tyr | | | | | | | | | |
| Lys | | | | | | | | | |
| Arg | | | | | ○ | | | | |
| His | | | | | | *β*-AlaHis | | | |
| Asp | | | | | | | | | |
| *α*-AB | | | | | ○ | | | | |
| *β*-Ala | | | | | | | | | |
| | Cit | | | | | ○ | | | |

The dipeptides formed by the reaction using, as substrates, two (or one) kinds of L-amino acids, Gly and *β*-Ala shown in the first row and the leftmost column of Table 1 are shown in the respective cells of the table. In the table, ○ means that a dipeptide was formed though its sequence was unidentified; × means that formation of a dipeptide was not confirmed; and a blank means that reaction was not carried out.

### Experimental Example 5

### Production of a Dipeptide Using the Strain Expressing the His-Tagged Recombinant Enzyme

Escherichia coli NM522/pQE60ywfE obtained in Experimental Example 1 was inoculated into 8 ml of LB medium containing 50 *µ*g/ml ampicillin in a test tube, and cultured at 28°C for 17 hours. The resulting culture was inoculated into 50 ml of LB medium containing 50 *µ*g/ml ampicillin in a 250-ml Erlenmeyer flask, and cultured at 30°C for 3 hours. Then, IPTG was added to give a final concentration of 1 mmol/l, followed by further culturing at 30°C for 4 hours. The resulting culture was centrifuged to obtain wet cells.

A reaction mixture (20 ml, pH 7.2) comprising 200 g/l wet cells, 50 g/l glucose, 5 g/l phytic acid (diluted to neutrality with 33% conc. sodium hydroxide solution), 15 g/l potassium dihydrogenphosphate, 5 g/l magnesium sulfate heptahydrate, 4 g/l Nymeen S-215, 10 ml/l xylene, 200 mmol/l L-Ala and 200 mmol/l L-Gln was put in a 50-ml beaker, and reaction was carried out at 32°C at 900 rpm for 2 hours. During the reaction, the pH of the reaction mixture was maintained at 7.2 by using 2 mol/l potassium hydroxide.

The reaction product was analyzed by the same method as in Experimental Example 3, whereby it was confirmed that 25 mg/l L-Ala-L-Gln was accumulated.

### Experimental Example 6

### Cloning of Genes Corresponding to the ywfE Gene from Various Microorganisms of the Genus Bacillus and Analysis Thereof

On the basis of the nucleotide sequence shown in SEQ ID NO: 26, genes corresponding to the ywfE gene which exist in Bacillus subtilis ATCC 15245, ATCC 6633, IAM 1213, IAM 1107, IAM 1214, ATCC 9466, IAM 1033 and ATCC 21555, Bacillus amyloliquefaciens IFO 3022 and Bacillus pumilus NRRL B-12025 were obtained in the following manner.

That is, Bacillus subtilis ATCC 15245, ATCC 6633, IAM 1213, IAM 1107, IAM 1214, ATCC 9466, IAM 1033 and ATCC 21555, Bacillus amyloliquefaciens IFO 3022 and Bacillus pumilus NRRL B-12025 were respectively inoculated into LB medium and subjected to static culture overnight at 30°C. After the culturing, the chromosomal DNAs of the respective microorganisms were isolated and purified according to the method using saturated phenol described in Current Protocols in Molecular Biology.

By using a DNA synthesizer (Model 8905, PerSeptive Biosystems, Inc.), DNAs having the nucleotide sequences shown in SEQ ID NOS: 37 and 38 (hereinafter referred to as primer C and primer D, respectively) were synthesized. Primer C has a sequence containing a region upstream of the initiation codon of ywfE gene of the chromosomal DNA of Bacillus subtilis 168, and primer D has a sequence complementary to a sequence containing a region downstream of the termination codon of ywfE gene.

PCR was carried out using each of the chromosomal DNAs of Bacillus subtilis ATCC 15245, ATCC 6633, IAM 1213, IAM 1107, IAM 1214, ATCC 9466, IAM 1033 and ATCC 21555 and Bacillus amyloliquefaciens IFO 3022 as a template and the above primer C and primer D as a set of primers. That is, PCR was carried out by 30 cycles, one cycle consisting of reaction at 94°C for one minute, reaction at 55°C for 2 minutes and reaction at 72°C for 3 minutes, using 40 *µ*l of a reaction mixture comprising 0.1 *µ*g of the chromosomal DNA, 0.5 *µ*mol/l each of the primers, 2.5 units of Pfu DNA polymerase, 4 *µ*l of buffer for Pfu DNA polymerase (10 x) and 200 *µ*mol/ each of dNTPs.

One-tenth of each of the resulting reaction mixtures was subjected to agarose gel electrophoresis to confirm that a ca. 1.4 kb fragment corresponding to the ywfE gene fragment was amplified. Then, the remaining reaction mixture was mixed with an equal amount of phenol/chloroform saturated with TE. The resulting solution was centrifuged, and the obtained upper layer was mixed with a two-fold volume of cold ethanol and allowed to stand at -80°C for 30 minutes. The resulting solution was centrifuged, and the obtained DNA precipitate was dissolved in 20 *µ*l of TE.

Each of the thus obtained 1.4 kb DNA fragments derived from the chromosomal DNAs of the respective strains and pCR-blunt (Invitrogen Corp.) were subjected to ligation reaction using a ligation kit at 16°C for 16 hours.

Escherichia coli NM522 was transformed using each ligation reaction mixture according to the method using calcium ion, spread on LB agar medium containing 50 *µ*g/ml ampicillin, and cultured overnight at 30°C.

A plasmid was extracted from a colony of each transformant that grew on the medium according to a known method and the structure of each plasmid was analyzed using restriction enzymes. As a result, it was confirmed that the following plasmids containing a gene corresponding to the ywfE gene were obtained: pYWFE1 (derived from ATCC 15245, DNA having the nucleotide sequence shown in SEQ ID NO: 65), pYWFE2 (derived from ATCC 6633, DNA having the nucleotide sequence shown in SEQ ID NO: 27), pYWFE3 (derived from IAM 1213, DNA having the nucleotide sequence shown in SEQ ID NO: 28), pYWFE4 (derived from IAM 1107, DNA having the nucleotide sequence shown in SEQ ID NO: 29), pYWFE5 (derived from IAM 1214, DNA having the nucleotide sequence shown in SEQ ID NO: 30), pYWFE6 (derived from ATCC 9466, DNA having the nucleotide sequence shown in SEQ ID NO: 26), pYWFE7 (derived from IAM 1033, DNA having the nucleotide sequence shown in SEQ ID NO: 65), pYWFE8 (derived from ATCC 21555, DNA having the nucleotide sequence shown in SEQ ID NO: 31) and pYWFE9 (derived from IFO 3022, DNA having the nucleotide sequence shown in SEQ ID NO: 32).

On the other hand, a gene corresponding to ywfE gene derived from Bacillus pumilus NRRL B-12025 (DNA having the nucleotide sequence shown in SEQ ID NO: 64) was obtained in the following manner.

PCR was carried out using the chromosomal DNA of the NRRL B-12025 strain prepared above as a template and DNAs respectively consisting of the nucleotide sequences shown in SEQ ID NOS: 66 and 67 as a set of primers. That is, PCR was carried out by 30 cycles, one cycle consisting of reaction at 98°C for 5 seconds, reaction at 55°C for 30 seconds and reaction at 72°C for one minute, using 50 *µ*l of a reaction mixture comprising 0.1 *µ*g of the chromosomal DNA, 0.5 *µ*mol/l each of the primers, 2.5 units of Z-taq polymerase (Takara Bio Inc.), 5 *µ*l of buffer for Z-taq polymerase (10 x) (Takara Bio Inc.) and 200 *µ*mol/l each of dNTPs.

One-tenth of the resulting reaction mixture was subjected to agarose gel electrophoresis to confirm that a ca. 0.8 kb fragment was amplified. Then, the remaining reaction mixture was mixed with an equal amount of phenol/chloroform saturated with TE. The resulting mixture was centrifuged, and the obtained upper layer was mixed with a two-fold volume of cold ethanol and allowed to stand at -80°C for 30 minutes. The resulting solution was centrifuged, and the obtained DNA precipitate was dissolved in 20 *µ*l of TE.

The thus obtained 0.8 kb fragment derived from the chromosomal DNA and pGEM T-easy (Promega Corp.) were subjected to ligation reaction using a ligation kit at 16°C for 16 hours.

Escherichia coli DH5α was transformed using the reaction mixture according to the method using calcium ion, spread on LB agar medium containing 50 *µ*g/ml ampicillin, and cultured overnight at 30°C.

A plasmid was extracted from the transformant obtained above and the nucleotide sequence of the ca. 0.8 kb DNA insert was determined, whereby a sequence from nucleotides 358 to 1160 in the nucleotide sequence shown in SEQ ID NO: 64 was confirmed.

The above plasmid was cleaved with EcoRI and then subjected to agarose gel electrophoresis to separate a DNA fragment. The DNA fragment was purified using GENECLEAN II Kit, and about 0.5 *µ*g of the purified DNA fragment was DIG-labeled using DIG-High Prime DNA Labeling & Detection Starter Kit I (Roche Diagnostics Corp.) according to the instructions attached thereto.

Southern analysis of the chromosomal DNA of the NRRL B-12025 strain was carried out using the DIG-labeled DNA obtained above.

The chromosomal DNA of the NRRL B-12025 strain was completely digested with BamHI, EcoRI, HindIII, KpnI, PstI, SacI, SalI and SphI, respectively, and subjected to agarose gel electrophoresis to separate DNA fragments, followed by transfer to nylon membrane plus charge (Roche Diagnostics Corp.) according to an ordinary method.

After the DNA fragments were fixed on the nylon membrane by UV irradiation, Southern hybridization was carried out using the above probe DNA and the nylon membrane.

The hybridization was carried out by contacting the nylon membrane with the probe DNA at 65°C for 16 hours, washing the nylon membrane twice with a solution consisting of 0.1% SDS and 2 x SSC at room temperature for 5 minutes, and further washing the membrane twice with a solution consisting of 0.1% SDS and 0.5 x SSC at 65°C for 15 minutes. The other operations and conditions and detection of the hybridized DNA were carried out according to the instructions attached to the above-mentioned DIG-High Prime DNA Labeling & Detection Starter Kit I.

As a result, color development was observed at around 3.5 kbp of the fragments completely digested with HindIII and PstI.

Subsequently, the chromosomal DNA of the NRRL B-12025 strain was completely digested with HindIII and PstI, respectively, and subjected to agarose gel electrophoresis to separate DNA fragments. From the respective restriction enzyme-digested DNAs, 3-4 kbp fragments were purified using GENECLEAN II Kit, followed by autocyclization using a ligation kit.

On the basis of the nucleotide sequence of the 0.8 kb DNA fragment determined above, the nucleotide sequences shown in SEQ ID NOS: 71 and 72 were designed and synthesized, and they were used in PCR using the cyclized DNA obtained above as a template. PCR was carried out by 30 cycles, one cycle consisting of reaction at 98°C for 5 seconds, reaction at 55°C for 30 seconds and reaction at 72°C for 3 minutes and 30 seconds, using 50 *µ*l of a reaction mixture comprising 10 ng of the cyclized DNA, 0.5 *µ*mol/l each of the primers, 2.5 units of pyrobest polymerase (Takara Bio Inc.), 5 *µ*l of buffer for pyrobest polymerase (10 x) (Takara Bio Inc.) and 200 *µ*mol/l each of dNTPs.

One-tenth of the resulting reaction mixture was subjected to agarose gel electrophoresis to confirm that a ca. 3.0 kb fragment was amplified. Then, the remaining reaction mixture was mixed with an equal amount of phenol/chloroform saturated with TE. The resulting mixture was centrifuged, and the obtained upper layer was mixed with a two-fold volume of cold ethanol and allowed to stand at -80°C for 30 minutes. The resulting solution was centrifuged, and the obtained DNA precipitate was dissolved in 20 *µ*l of TE.

The thus obtained DNA fragment and Zero Blunt PCR Cloning Kit (Invitrogen Corp.) were subjected to ligation reaction using a ligation kit.

Escherichia coli NM522 was transformed using the reaction mixture according to the method using calcium ion, spread on LB agar medium containing 50 *µ*g/ml ampicillin, and cultured overnight at 30°C.

A plasmid was extracted from a colony of the transformant that grew on the medium according to a known method and the structure of the plasmid was analyzed using restriction enzymes. As a result, it was confirmed that plasmid pYWFE10 (derived from NRRL B-12025, DNA having the nucleotide sequence shown in SEQ ID NO: 64) containing a gene corresponding to the ywfE gene was obtained.

The nucleotide sequences of the genes corresponding to the ywfE gene which are respectively contained in the plasmids pYWFE1 to pYWFE10 obtained above were determined using 373A DNA Sequencer.

The amino acid sequences of the proteins encoded by the genes respectively contained in pYWFE1, pYWFE6 and pYWFE7 were identical with the amino acid sequence of the protein encoded by the ywfE gene, whereas those of the proteins encoded by the genes respectively contained in pYWFE2, pYWFE3, pYWFE4, pYWFE5, pYWFE8, pYWFE9 and pYWFE10 were different from the amino acid sequence of the protein encoded by the ywfE gene.

The amino acid sequences of the proteins encoded by the genes corresponding to the ywfE gene which are contained in pYWFE2, pYWFE3, pYWFE4, pYWFE5, pYWFE8, pYWFE9 and pYWFE10, and pYWFE1 and pYWFE7 are shown in SEQ ID NOS: 20 to 25, 68 and 19, respectively, and the nucleotide sequences of these genes are shown in SEQ ID NOS: 27 to 32, 65 and 26, respectively.

### Experimental Example 7

### Purification of C-Terminal His-Tagged Recombinant Dipeptide Synthetase

PCR was carried out using each of the chromosomal DNAs of Bacillus subtilis ATCC 15245, ATCC 6633, IAM 1213, IAM 1107, IAM 1214, ATCC 9466, IAM 1033 and ATCC 21555 and Bacillus amyloliquefaciens IFO 3022 as a template and primer A and primer B described in Experimental Example 1 as a set of primers. That is, PCR was carried out by 30 cycles, one cycle consisting of reaction at 94°C for one minute, reaction at 55°C for 2 minutes and reaction at 72°C for 3 minutes, using 40 *µ*l of a reaction mixture comprising 0.1 *µ*g of the chromosomal DNA, 0.5 *µ*mol/l each of the primers, 2.5 units of Pfu DNA polymerase, 4 *µ*l of buffer for Pfu DNA polymerase (10 x) and 200 *µ*mol/l each of dNTPs.

When the chromosomal DNA of Bacillus pumilus NRRL B-12025 was used as a template, PCR was carried out using DNAs respectively having the nucleotide sequences shown in SEQ ID NOS: 69 and 70 as a set of primers under the same conditions as above.

One-tenth of each of the resulting reaction mixtures was subjected to agarose gel electrophoresis to confirm that a ca. 1.4 kb DNA fragment corresponding to the ywfE fragment was amplified. Then, the remaining reaction mixture was mixed with an equal amount of phenol/chloroform saturated with TE. The resulting mixture was centrifuged, and the obtained upper layer was mixed with a two-fold volume of cold ethanol and allowed to stand at -80°C for 30 minutes. The resulting solution was centrifuged, and the obtained DNA precipitate was dissolved in 20 *µ*l of TE.

Each of the thus obtained solutions (5 *µ*l) was subjected to reaction to cleave the amplified DNA with restriction enzymes NcoI and BamHI. DNA fragments were separated by agarose gel electrophoresis, and a 1.4 kb DNA fragment containing a gene corresponding to the ywfE gene was recovered using GENECLEAN II Kit.

Subsequently, 0.2 *µ*g of the C-terminal His-tagged recombinant expression vector pQE60 was cleaved with restriction enzymes NcoI and BamHI. DNA fragments were separated by agarose gel electrophoresis, and a 3.4 kb DNA fragment was recovered in the same manner as above.

Each of the 1.4 kb DNA fragments containing a gene corresponding to the ywfE gene of Bacillus subtilis 168 and the 3.4 kb DNA fragment obtained above were subjected to ligation reaction using a ligation kit at 16°C for 16 hours. Escherichia coli NM522 was transformed using each ligation reaction mixture according to the method using calcium ion, spread on LB agar medium containing 50 *µ*g/ml ampicillin, and cultured overnight at 30°C.

A plasmid was extracted from a colony of each transformant that grew on the medium according to a known method and the structure of each plasmid was analyzed using restriction enzymes. As a result, it was confirmed that the following C-terminal His-tagged gene expression vectors were obtained: pQE60ywfE1 (a vector containing the gene derived from ATCC 15245), pQE60ywfE2 (a vector containing the gene derived from ATCC 6633), pQE60ywfE3 (a vector containing the gene derived from IAM 1213), pQE60ywfE4 (a vector containing the gene derived from IAM 1107), pQE60ywfE5 (a vector containing the gene derived from IAM 1214), pQE60ywfE6 (a vector containing the gene derived from ATCC 9466), pQE60ywfE7 (a vector containing the gene derived from IAM 1033), pQE60ywfE8 (a vector containing the gene derived from ATCC 21555), pQE60ywfE9 (a vector containing the gene derived from IFO 3022) and pQE60ywfE10 (a vector containing the gene derived from NRRL B-12025).

Escherichia coli NM522/pQE60ywfE1 to NM522/pQE60ywfE10 strains obtained above were respectively inoculated into 8 ml of LB medium containing 50 *µ*g/ml ampicillin in a test tube, and cultured at 28°C for 17 hours. Each of the resulting cultures was inoculated into 50 ml of LB medium containing 50 *µ*g/ml ampicillin in a 250-ml Erlenmeyer flask, and cultured at 30°C for 3 hours. Then, IPTG was added to give a final concentration of 1 mmol/l, followed by further culturing at 30°C for 4 hours. The resulting culture was centrifuged to obtain wet cells, and His-tagged recombinant enzymes were purified from the respective wet cells using HisTrap according to the instructions attached thereto.

### Experimental Example 8

### Production of Dipeptides Using Purified Enzymes

Reaction mixtures (0.1 ml each) comprising 0.04 mg of the respective recombinant enzymes obtained in Experimental Example 7, 100 mmol/l Tris-HCl (pH 8.0), 60 mmol/l magnesium chloride, 60 mmol/l ATP, 30 mmol/l L-Ala and 30 mmol/l L-Gln were prepared, and reactions were carried out at 37°C for 16 hours.

After the completion of reactions, the reaction mixtures were analyzed by the method described in Experimental Example 3, whereby it was confirmed that 3.0 to 3.5 g/l L-Ala-L-Gln and 0.25 to 0.3 g/l L-Ala-L-Ala were formed and accumulated.

When ATP was excluded from the compositions of the above reaction mixtures, L-Ala-L-Gln or L-Ala-L-Ala was not formed at all.

The above results revealed that all of the products of the genes obtained in Experimental Example 7 have the activity to produce L-Ala-L-Gln and L-Ala-L-Ala from L-Ala and L-Gln in the presence of ATP.

### Experimental Example 9

### Construction of Escherichia coli for Enhanced Expression of the ywfE Gene

By using a DNA synthesizer (Model 8905, PerSeptive Biosystems, Inc.), DNAs having the sequences shown in SEQ ID NOS: 60 to 63 (hereinafter referred to as primer E, primer F, primer G and primer H, respectively) were synthesized. The sequence of SEQ ID NO: 60 is a sequence wherein a sequence containing the XhoI recognition sequence is added to the 5' end of a region containing the Shine-Dalgarno sequence (ribosome binding sequence) of ywfE gene on the plasmid pQE60ywfE. The sequence of SEQ ID NO: 61 is a sequence wherein a sequence containing the BamHI recognition sequence is added to the 5' end of a sequence complementary to a sequence containing the termination codon of ywfE gene. The sequence of SEQ ID NO: 62 is a sequence wherein a sequence containing the EcoRI recognition sequence is added to the 5' end of the sequence of trp promoter region of expression vector pTrS30 containing trp promoter. The sequence of SEQ ID NO: 63 is a sequence wherein a sequence containing the XhoI recognition sequence is added to the 5' end of a sequence complementary to the sequence of trp promoter region of expression vector pTrS30 containing trp promoter.

A ywfE gene fragment and a trp promoter region fragment were amplified by PCR using the above primers E and F and primers G and H as a set of primers, respectively, and the plasmid pQE60ywfE as a template. PCR was carried out by 30 cycles, one cycle consisting of reaction at 94°C for one minute, reaction at 55°C for 2 minutes and reaction at 72°C for 3 minutes, using 40 *µ*l of a reaction mixture comprising 10 ng of pQE60ywfE, 0.5 *µ*mol/l each of the primers, 2.5 units of Pfu DNA polymerase, 4 *µ*l of buffer for Pfu DNA polymerase (10 x) and 200 *µ*mol/l each of dNTPs.

One-tenth of each of the resulting reaction mixtures was subjected to agarose gel electrophoresis to confirm that a ca. 1.4 kb fragment corresponding to the ywfE gene fragment and a ca. 0.3 kb fragment corresponding to the trp promoter region fragment were respectively amplified in the PCR using primer E and primer F and the PCR using primer G and primer H. Then, the remaining reaction mixture was mixed with an equal amount of phenol/chloroform saturated with TE. The resulting solution was centrifuged, and the obtained upper layer was mixed with a two-fold volume of cold ethanol and allowed to stand at -80°C for 30 minutes. The resulting solution was centrifuged, and the obtained DNA was dissolved in 20 *µ*l of TE.

The thus obtained DNA solutions (5 *µ*l each) were respectively subjected to reaction to cleave the DNA amplified using primer E and primer F with restriction enzymes XhoI and BamHI and to reaction to cleave the DNA amplified using primer G and primer H with restriction enzymes EcoRI and XhoI. DNA fragments were separated by agarose gel electrophoresis, and a 1.4 kb fragment containing ywfE gene and a 0.3 kb fragment containing trp promoter region were respectively recovered using GENECLEAN II Kit.

pTrs30 (a trp promoter-containing expression vector, 0.2 *µ*g) was cleaved with restriction enzymes EcoRI and BamHI. DNA fragments were separated by agarose gel electrophoresis and a 4.5 kb DNA fragment was recovered in the same manner as above.

The 1.4 kb fragment containing the ywfE gene, the 0.3 kb fragment containing trp promoter region and the 4.5 kb DNA fragment obtained above were subjected to ligation reaction using a ligation kit at 16°C for 16 hours.

Escherichia coli NM522 was transformed using the reaction mixture according to the method using calcium ion, spread on LB agar medium containing 50 *µ*g/ml ampicillin, and cultured overnight at 30°C.

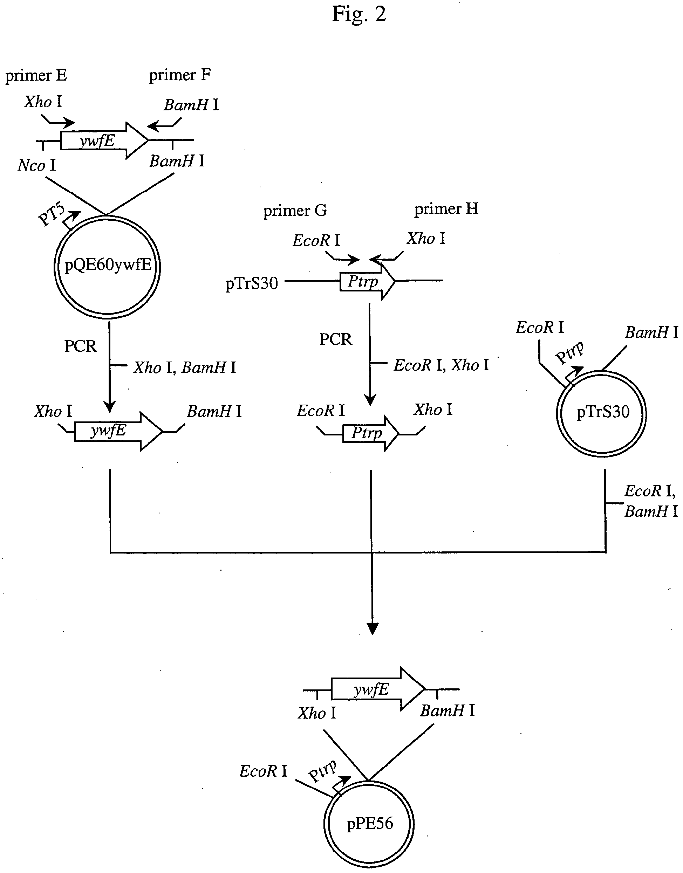

A plasmid was extracted from a colony of the transformant that grew on the medium according to a known method, whereby expression vector pPE56 containing the ywfE gene in a downstream position of the trp promoter was obtained. The structure of the vector was confirmed by digestion with restriction enzymes (Fig. 2).

Certain embodiments of the present invention are illustrated in the following examples. These examples are not to be construed as limiting the scope of the invention.

### Example 1

### Preparation of Strains Having pepD gene, pepN gene, pepB gene, pepA gene and dpp Operon Deletions

Strains in which specific genes on Escherichia coli chromosomal DNA are deleted were prepared according to the method utilizing the homologous recombination system of lambda phage [Proc. Natl. Acad. Sci. USA, 97, 6641-6645 (2000)].

Plasmids pKD46, pKD3 and pCP20 used below were prepared by extraction from Escherichia coli strains carrying them which were obtained from Escherichia coli Genetic Stock Center, Yale University, U.S.A.

### (1) Cloning of DNA Fragments for Gene Deletion

For the purpose of deleting the following genes existing on the chromosomal DNA of Escherichia coli K12, DNAs having nucleotide sequences homologous to 36-bp nucleotide sequences that lie upstream and downstream of the respective genes to be deleted on the chromosomal DNA of Escherichia coli K12 and the nucleotide sequence shown in SEQ ID NO: 39 which is recognized by yeast-derived Flp recombinase were synthesized using a DNA synthesizer (Model 8905, PerSeptive Biosystems, Inc.). The genes to be deleted are pepD gene having the nucleotide sequence shown in SEQ ID NO: 10, pepN gene having the nucleotide sequence shown in SEQ ID NO: 11, pepB gene having the nucleotide sequence shown in SEQ ID NO: 12, pepA gene having the nucleotide sequence shown in SEQ ID NO: 13, dppA gene having the nucleotide sequence shown in SEQ ID NO: 14, dppB gene having the nucleotide sequence shown in SEQ ID NO: 15, dppC gene having the nucleotide sequence shown in SEQ ID NO: 16, dppD gene having the nucleotide sequence shown in SEQ ID NO: 17 and dppF gene having the nucleotide sequence shown in SEQ ID NO: 18. In the case of dppA gene, dppB gene, dppC gene, dppD gene and dppF gene, which form an operon, DNAs having nucleotide sequences homologous to the nucleotide sequences that lie upstream and downstream of the operon were synthesized.

That is, DNAs consisting of the following nucleotide sequences were synthesized as respective sets of primers for amplification of DNA fragments for gene deletion: SEQ ID NOS: 40 and 41 for pepD gene deletion; SEQ ID NOS: 42 and 43 for pepN gene deletion; SEQ ID NOS: 44 and 45 for pepA gene deletion; SEQ ID NOS: 46 and 47 for pepB gene deletion; and SEQ ID NOS: 48 and 49 for dpp operon deletion.

Subsequently, PCR was carried out using each set of the above synthetic DNAs as a set of primers and pKD3 DNA as a template. That is, PCR was carried out by 30 cycles, one cycle consisting of reaction at 94°C for one minute, reaction at 55°C for 2 minutes and reaction at 72°C for 3 minutes, using 40 *µ*l of a reaction mixture comprising 10 ng of the plasmid DNA, 0.5 *µ*mol/l each of the primers, 2.5 units of Pfu DNA polymerase (Stratagene), 4 *µ*l of buffer for Pfu DNA polymerase (10 x) (Stratagene) and 200 *µ*mol/l each of deoxyNTPs (dATP, dGTP, dCTP and dTTP).

One-tenth of each of the resulting reaction mixtures was subjected to agarose gel electrophoresis to confirm that the desired fragment was amplified. Then, the remaining reaction mixture was mixed with an equal amount of phenol/chloroform (1 vol/1 vol) saturated with TE [10 mmol/l Tris-HCl (pH 8.0), 1 mmol/l EDTA].

The resulting mixture was centrifuged, and the obtained upper layer was mixed with a two-fold volume of cold ethanol and allowed to stand at -80°C for 30 minutes, followed by centrifugation. By this procedure, chloramphenicol resistance gene-containing DNA fragments for deletion of pepD gene, pepN gene, pepB gene, pepA gene and dpp operon were obtained.

### (2) Preparation of Escherichia coli JM101 Having pepD Gene Deletion

Escherichia coli JM101 was transformed with pKD46, spread on LB agar medium containing 100 mg/l ampicillin, and cultured at 30°C to select a transformant.

The plasmid pKD46 carries inserted *λ* Red recombinase gene and is designed so that the expression of the gene is induced by L-arabinose. Accordingly, when the Escherichia coli grown in the presence of L-arabinose is transformed using a linear DNA, homologous recombination occurs with high frequency. Further, as pKD46 has a thermosensitive replication origin, curing of the plasmid can be readily caused by culturing the strain at 42°C.

The chloramphenicol resistance gene-containing DNA fragment for pepD gene deletion obtained above was introduced into Escherichia coli JM101/pKD46 obtained by culturing in the presence of 10 mmol/l L-arabinose and 50 *µ*g/ml ampicillin by electroporation. The resulting cells were spread on LB agar medium (10 g/l Bacto-tryptone, 5 g/l Bacto-yeast extract, 5 g/l sodium chloride and 15 g/l agar) containing 25 mg/l chloramphenicol and cultured at 30°C to select a transformant in which the chloramphenicol resistance gene-containing DNA fragment for pepD gene deletion was integrated into the chromosomal DNA of Escherichia coli JM101 by homologous recombination.

The selected chloramphenicol-resistant strain was inoculated onto LB agar medium containing 25 mg/l chloramphenicol and cultured at 42°C for 14 hours, followed by single colony isolation. Replicas of the obtained colonies were made on LB agar medium containing 25 mg/l chloramphenicol and LB agar medium containing 100 mg/l ampicillin, followed by culturing at 37°C. By selecting a colony showing chloramphenicol resistance and ampicillin sensitivity, a pKD46-cured strain was obtained.

The pKD46-cured strain thus obtained was transformed using pCP20, followed by selection on LB agar medium containing 100 mg/l ampicillin to obtain a pKD46-cured strain carrying pCP20.

The plasmid pCP20 carries inserted yeast-derived Flp recombinase gene and is designed so that the expression of the gene is induced at a temperature of 42°C.

The chloramphenicol resistance gene-containing DNA fragments for deletion of pepD gene, pepN gene, pepB gene, pepA gene and dpp operon prepared above contain nucleotide sequences recognized by Flp recombinase at both termini of the chloramphenicol resistance gene. Therefore, the resistance gene can be readily deleted by homologous recombination catalyzed by Flp recombinase.

Further, as pCP20 has a thermosensitive replication origin, expression of Flp recombinase and curing of pCP20 can be simultaneously induced by culturing the pCP20-carrying strain at 42°C.

The pCP20-carrying pKD46-cured strain obtained above was inoculated onto drug-free LB agar medium and cultured at 42°C for 14 hours, followed by single colony isolation. Replicas of the obtained colonies were made on drug-free LB agar medium, LB agar medium containing 25 mg/l chloramphenicol and LB agar medium containing 100 mg/l ampicillin, followed by culturing at 30°C. Then, colonies showing chloramphenicol sensitivity and ampicillin sensitivity were selected.

Chromosomal DNAs were prepared from the respective strains selected above according to an ordinary method [Seibutsukogaku Jikkensho (Experiments in Biotechnology), edited by The Society for Biotechnology, Japan, p. 97-98, Baifukan (1992)]. PCR was carried out using, as a set of primers, DNAs having the nucleotide sequences shown in SEQ ID NOS: 50 and 51 which were designed based on an inner nucleotide sequence of the pepD gene to be deleted, and using each of the chromosomal DNAs as a template. That is, PCR was carried out by 30 cycles, one cycle consisting of reaction at 94°C for one minute, reaction at 55°C for 2 minutes and reaction at 72°C for 3 minutes, using 40 *µ*l of a reaction mixture comprising 0.1 *µ*g of the chromosomal DNA, 0.5 *µ*mol/l each of the primers, 2.5 units of Pfu DNA polymerase, 4 *µ*l of buffer for Pfu DNA polymerase (10 x) and 200 *µ*mol/l each of deoxyNTPs.

A strain with which no amplified DNA fragment was detected in the above PCR was identified as a strain having pepD gene deletion and was designated as Escherichia coli JPD1.

### (3) Preparation of a Strain in Which the pepD Gene and pepN Gene on the Chromosomal DNA of Escherichia coli JM101 are Deleted

Escherichia coli JPD1 obtained in the above (2) was transformed with pKD46, spread on LB agar medium containing 100 mg/l ampicillin, and cultured at 30°C to select a transformant. The chloramphenicol resistance gene-containing DNA fragment for pepN gene deletion was introduced into the obtained transformant (Escherichia coli JPD1/pKD46) by electroporation to obtain a transformant in which the chloramphenicol resistance gene-containing DNA fragment for pepN gene deletion was integrated into the chromosomal DNA of Escherichia coli JPD1/pKD46 by homologous recombination.

Subsequently, the same procedure as in the above (2) was carried out to obtain a strain in which the chloramphenicol resistance gene was deleted from the chromosomal DNA, which was designated as Escherichia coli JPDN2.

### (4) Preparation of Strains in Which pepN Gene, pepA Gene, pepB Gene or dpp Operon on the Chromosomal DNA of Escherichia coli JM101 is Deleted and Strains Having Multiple Gene Deletion

The strains having pepN gene, pepA gene, pepB gene or dpp operon deletion were prepared according to the same procedure as in the above (2) using the respective chloramphenicol resistance gene-containing DNA fragments for gene or operon deletion prepared in the above (1).

Acquisition of the strains having gene deletions by the above method was confirmed by carrying out PCR in the same manner as in the above (2) using, as sets of primers, DNAs having the nucleotide sequences shown in SEQ ID NOS: 52 to 59 which were designed and syntheseized based on inner nucleotide sequences of the respective genes to be deleted. That is, DNAs having the following nucleotide sequences were used as respective sets of primers for the confirmation of gene deletion: SEQ ID NOS: 52 and 53 for pepN gene deletion; SEQ ID NOS: 54 and 55 for pepA gene deletion; SEQ ID NOS: 56 and 57 for pepB gene deletion; and SEQ ID NOS: 58 and 59 for dpp operon deletion.

The thus obtained dpp operon-deleted strain, pepN gene-deleted strain, pepA gene-deleted strain and pepB gene-deleted strain were designated as Escherichia coli JDPP1, Escherichia coli JPN1, Escherichia coli JPA1 and Escherichia coli JPB7, respectively.

Further, strains having multiple gene deletions, i.e., deletions of two or more genes or operon selected from the group consisting of pepD gene, pepN gene, pepA gene, pepB gene and dpp operon were prepared according to the method of the above (3). Acquisition of the strains having multiple gene deletions was confirmed by PCR similar to that in the above (2). The thus obtained double gene-deleted strain having pepD gene and dpp operon deletions was designated as Escherichia coli JPDP49, triple gene-deleted strain having pepB gene, pepD gene and pepN gene deletions as Escherichia coli JPDNB43, triple gene-deleted strain having pepD gene, pepN gene and dpp operon deletions as Escherichia coli JPNDDP36, quadruple gene-deleted strain having pepA gene, pepD gene, pepN gene and dpp operon deletions as Escherichia coli JPNDAP5, and quadruple gene-deleted strain having pepB gene, pepD gene, pepN gene and dpp operon deletions as Escherichia coli JPNDBP7. The genes deleted in the gene-deleted strains are shown in Table 2.

**Table 2**

| Strain | Deleted gene |
|---|---|
| JM101 | none |
| JDPP1 | dpp operon |
| JPN1 | pepN |
| JPA1 | pepA |
| JPB7 | pepB |
| JPD1 | pepD |
| JPDN2 | pepD, pepN |
| JPNDB43 | pepB, pepD, pepN |
| JPDP49 | pepD, dpp operon |
| JPNDDP36 | pepD, pepN, dpp operon |
| JPNDAP5 | pepA, pepD, pepN, dpp operon |
| JPNDBP7 | pepB, pepD, pepN, dpp operon |

### Example 2

### Evaluation of Productivity of L-Alanyl-L-glutamine (hereinafter referred to as AlaGln) and L-Alanyl-L-alanine (hereinafter referred to as AlaAla) by Escherichia coli Strains in Which Peptidase and Peptide-transporting Activities are Lost

The strains having deletions of genes encoding various peptidase genes and an operon encoding peptide-transporting protein which were obtained in Example 1 were transformed using the plasmid pPE56 constructed in Experimental Example 8 to obtain ampicillin-resistant transformants.

Each of the obtained transformants was inoculated into 8 ml of LB medium containing 50 *µ*g/ml ampicillin in a test tube and cultured at 28°C for 17 hours. The resulting culture was inoculated into 8 ml of a production medium [16 g/l dipotassium hydrogenphosphate, 14 g/l potassium dihydrogenphosphate, 5 g/l ammonium sulfate, 1 g/l citric acid (anhydrous), 0.5 g/l Casamino acid (Difco), 1 g/l L-Pro, 2.5 g/l L-Ala, 2.5 g/l L-Gln, 10 g/l glucose, 10 mg/l vitamin B₁, 25 mg/l magnesium sulfate heptahydrate and 50 mg/l ferrous sulfate heptahydrate; pH adjusted to 7.2 with 10 mol/l sodium hydroxide solution; L-Gln was added after sterilization by filtration of a 10-fold conc. solution; glucose, vitamin B₁, magnesium sulfate heptahydrate and ferrous sulfate heptahydrate were added after separate autoclaving] containing 100 *µ*g/ml ampicillin in a test tube in an amount of 1% and cultured at 30°C for 24 hours. The resulting culture was centrifuged to obtain a culture supernatant.

The product in the culture supernatant was derivatized by the F-moc method and then analyzed by HPLC. The HPLC analysis was carried out using ODS-HG5 (Nomura Kagaku Co., Ltd.) as a separation column and solution A (6 ml/l acetic acid and 20% (v/v) acetonitrile, pH adjusted to 4.8 with triethylamine) and solution B (6 ml/l acetic acid and 70% (v/v) acetonitrile, pH adjusted to 4.8 with triethylamine) as eluting solutions. The ratio of solution A to solution B was 8:2 during the first 5 minutes of elution and thereafter changed with a linear gradient so that the ratio became 1:1 at 20 minutes after the start of elution. The results of analysis are shown in Table 3.

**Table 3**

| Strain | Deleted gene | AlaGln (g/l) | AlaAla (g/l) |
|---|---|---|---|
| JM101 | none | 0 | 0 |
| JDPP1 | dpp operon | 0.02 | 0.01 |
| JPN1 | pepN | 0.01 | 0.01 |
| JPA1 | pepA | 0.01 | 0.01 |
| JPB7 | pepB | 0.01 | 0.01 |
| JPD1 | pepD | 0.01 | 0.01 |
| JPDN2 | pepD, pepN | 0.02 | 0.03 |
| JPNDB43 | pepB, pepD, pepN | 0.05 | 0.12 |
| JPDP49 | pepD, dpp operon | 0.11 | 0.08 |
| JPNDDP36 | pepD, pepN, dpp operon | 0.16 | 0.21 |
| JPNDAP5 | pepA, pepD, pepN, dpp operon | 0.28 | 0.26 |
| JPNDBP7 | pepB, pepD, pepN, dpp operon | 0.43 | 0.22 |

As can be seen from Table 3, small amounts of dipeptides were formed and accumulated by use of the microorganisms having deletions of two or less kinds of peptidase genes or one kind of peptide-transporting protein gene, whereas the amounts of dipeptides formed and accumulated were greatly increased by use of the microorganisms having deletions of one or more kinds of peptidase genes and one kind of peptide-transporting protein gene or microorganisms having deletions of three or more kinds of peptidase genes.

### Example 3

### Evaluation of Productivity of L-Alanyl-L-valine (hereinafter referred to as AlaVal) by Escherichia coli Strains in Which Peptidase and Peptide-transporting Protein Activities are Lost

Similarly to Example 2, the Escherichia coli strains having deletions of various peptidase genes and an operon encoding a peptide-transporting protein were transformed using pPE56. Each of the obtained transformants was inoculated into 8 ml of LB medium containing 50 *µ*g/ml ampicillin in a test tube and cultured at 28°C for 17 hours. The resulting culture was inoculated into 8 ml of a production medium [16 g/l dipotassium hydrogenphosphate, 14 g/l potassium dihydrogenphosphate, 5 g/l ammonium sulfate, 1 g/l citric acid (anhydrous), 0.5 g/l Casamino acid (Difco), 1 g/l L-Pro, 2.5 g/l L-Ala, 2.5 g/l L-Val, 10 g/l glucose, 10 mg/l vitamin B₁, 25 mg/l magnesium sulfate heptahydrate and 50 mg/l ferrous sulfate heptahydrate; pH adjusted to 7.2 with 10 mol/l sodium hydroxide solution; glucose, vitamin B₁, magnesium sulfate heptahydrate and ferrous sulfate heptahydrate were added after separate autoclaving] containing 100 *µ*g/ml ampicillin in a test tube in an amount of 1% and cultured at 30°C for 24 hours. The resulting culture was centrifuged to obtain a culture supernatant.

The product in the culture supernatant was analyzed by the method described in Example 2. The results are shown in Table 4.

**Table 4**

| Strain | Deleted gene | AlaVal (g/l) |
|---|---|---|
| JM101 | none | 0 |
| JDPP1 | dpp operon | 0 |
| JPN1 | pepN | 0 |
| JPA1 | pepA | 0 |
| JPB7 | pepB | 0 |
| JPD1 | pepD | 0 |
| JPDN2 | pepD, pepN | 0 |
| JPNDB43 | pepB, pepD, pepN | 0.04 |
| JPDP49 | pepD, dpp operon | 0.11 |
| JPNDDP36 | pepD, pepN, dpp operon | 0.22 |
| JPNDBP7 | pepB, pepD, pepN, dpp operon | 0.20 |

As can be seen from Table 4, the dipeptide was not produced by use of the microorganisms having deletions of two or less kinds of peptidase genes or one kind of peptide-transporting protein gene, whereas the dipeptide was produced by use of the microorganisms having deletions of three or more kinds of peptidase genes or microorganisms having deletions of one or more kinds of peptidase genes and one kind of peptide-transporting protein gene.

### Example 4

### Evaluation of Productivity of Glycyl-L-glutamine (hereinafter referred to as GlyGln) by Escherichia coli Strains in Which Peptidase and Peptide-transporting Protein Activities are Lost

Similarly to Example 2, the strains having deletions of various peptidase genes and an operon encoding a peptide-transporting protein were transformed using pPE56. Each of the obtained transformants was inoculated into 8 ml of LB medium containing 50 *µ*g/ml ampicillin in a test tube and cultured at 28°C for 17 hours.

The resulting culture was inoculated into 8 ml of a production medium [16 g/l dipotassium hydrogenphosphate, 14 g/l potassium dihydrogenphosphate, 5 g/l ammonium sulfate, 1 g/l citric acid (anhydrous), 0.5 g/l Casamino acid (Difco), 1 g/l L-Pro, 2.5 g/l Gly, 2.5 g/l L-Gln, 10 g/l glucose, 10 mg/l vitamin B₁, 25 mg/l magnesium sulfate heptahydrate and 50 mg/l ferrous sulfate heptahydrate; pH adjusted to 7.2 with 10 mol/l sodium hydroxide solution; L-Gln was added after sterilization by filtration of a 10-fold conc. solution; glucose, vitamin B₁, magnesium sulfate heptahydrate and ferrous sulfate heptahydrate were added after separate autoclaving] containing 100 *µ*g/ml ampicillin in a test tube in an amount of 1% and cultured at 30°C for 24 hours. The resulting culture was centrifuged to obtain a culture supernatant.

The product in the culture supernatant was analyzed by the method described in Example 2. The results are shown in Table 5.

**Table 5**

| Strain | Deleted gene | GlyGln (g/l) |
|---|---|---|
| JM101 | none | 0 |
| JDPP1 | dpp operon | 0 |
| JPDN2 | pepD, pepN | 0 |
| JPNDB43 | pepB, pepD, pepN | 0.01 |
| JPNDDP36 | pepD, pepN, dpp operon | 0.02 |
| JPNDBP7 | pepB, pepD, pepN, dpp operon | 0.03 |

As can be seen from Table 5, the dipeptide was not produced by use of the microorganisms having deletions of two or less kinds of peptidase genes or one kind of peptide-transporting protein gene, whereas the dipeptide was produced by use of the microorganisms having deletions of three or more kinds of peptidase genes or microorganisms having deletions of two or more kinds of peptidase genes and one kind of peptide-transporting protein gene.

### SEQUENCE LISTING FREE TEXT

SEQ ID NO: 35 - Description of Artificial Sequence: Synthetic DNA
SEQ ID NO: 36 - Description of Artificial Sequence: Synthetic DNA
SEQ ID NO: 37 - Description of Artificial Sequence: Synthetic DNA
SEQ ID NO: 38 - Description of Artificial Sequence: Synthetic DNA
SEQ ID NO: 39 - Description of Artificial Sequence: Synthetic DNA
SEQ ID NO: 40 - Description of Artificial Sequence: Synthetic DNA
SEQ ID NO: 41 - Description of Artificial Sequence: Synthetic DNA
SEQ ID NO: 42 - Description of Artificial Sequence: Synthetic DNA
SEQ ID NO: 43 - Description of Artificial Sequence: Synthetic DNA
SEQ ID NO: 44 - Description of Artificial Sequence: Synthetic DNA
SEQ ID NO: 45 - Description of Artificial Sequence: Synthetic DNA
SEQ ID NO: 46 - Description of Artificial Sequence: Synthetic DNA
SEQ ID NO: 47 - Description of Artificial Sequence: Synthetic DNA
SEQ ID NO: 48 - Description of Artificial Sequence: Synthetic DNA
SEQ ID NO: 49 - Description of Artificial Sequence: Synthetic DNA
SEQ ID NO: 50 - Description of Artificial Sequence: Synthetic DNA
SEQ ID NO: 51 - Description of Artificial Sequence: Synthetic DNA
SEQ ID NO: 52 - Description of Artificial Sequence: Synthetic DNA
SEQ ID NO: 53 - Description of Artificial Sequence: Synthetic DNA
SEQ ID NO: 54 - Description of Artificial Sequence: Synthetic DNA
SEQ ID NO: 55 - Description of Artificial Sequence: Synthetic DNA
SEQ ID NO: 56 - Description of Artificial Sequence: Synthetic DNA
SEQ ID NO: 57 - Description of Artificial Sequence: Synthetic DNA
SEQ ID NO: 58 - Description of Artificial Sequence: Synthetic DNA
SEQ ID NO: 59 - Description of Artificial Sequence: Synthetic DNA
SEQ ID NO: 60 - Description of Artificial Sequence: Synthetic DNA
SEQ ID NO: 61 - Description of Artificial Sequence: Synthetic DNA
SEQ ID NO: 62 - Description of Artificial Sequence: Synthetic DNA
SEQ ID NO: 63 - Description of Artificial Sequence: Synthetic DNA
SEQ ID NO: 66 - Description of Artificial Sequence: Synthetic DNA
SEQ ID NO: 67 - Description of Artificial Sequence: Synthetic DNA
SEQ ID NO: 69 - Description of Artificial Sequence: Synthetic DNA
SEQ ID NO: 70 - Description of Artificial Sequence: Synthetic DNA
SEQ ID NO: 71 - Description of Artificial Sequence: Synthetic DNA
SEQ ID NO: 72 - Description of Artificial Sequence: Synthetic DNA

## Claims

1. A process for producing a dipeptide, which comprises:
allowing an enzyme source and one or more kinds of amino acids to be present in an aqueous medium,
said enzyme source being a culture of a microorganism in which the activities of three or more kinds of peptidases are reduced or lost as compared to the parent strain, said parent strain belonging to the genus Escherichia, Bacillus, Corynebacterium or Saccharomyces, wherein said microorganism has the ability to produce a dipeptide, and wherein said ability to produce a dipeptide is the ability to form a dipeptide by condensation and ligation of one or more kinds of amino acids; or a treated matter of the culture;
allowing the dipeptide to form and accumulate in the aqueous medium; and
recovering the dipeptide from the medium.

2. The process according to claim 1, wherein the peptidase is a protein having the amino acid sequence shown in any of SEQ ID NOs: 1 to 4, or a protein having an amino acid sequence which has 80% or more homology to the amino acid sequence shown in any of SEQ ID NOs: 1 to 4 and having peptidase activity.

3. The process according to claim 1 or 2, wherein the microorganism which has the ability to produce a dipeptide by condensation and ligation of one or more kinds of amino acids is a microorganism having the ability to produce a protein according to any of [1] to [4] below:
[1] a protein having the amino acid sequence shown in any of SEQ ID NOs: 19 to 25 and 68;
[2] a protein consisting of an amino acid sequence wherein one or more amino acid residues are deleted, substituted or added in the amino acid sequence shown in any of SEQ ID NOs: 19 to 25 and 68 and having the activity to synthesize a dipeptide;
[3] a protein consisting of an amino acid sequence which has 65% or more homology to the amino acid sequence shown in any of SEQ ID NOs: 19 to 25 and 68 and having the activity to synthesize a dipeptide; and
[4] a protein having an amino acid sequence which has 80% or more homology to the amino acid sequence shown in SEQ ID NO: 33 and having the activity to synthesize a dipeptide.

4. The process according to any one of claims 1 to 3, wherein the microorganism which has the ability to produce a dipeptide by condensation and ligation of one or more kinds of amino acids is a microorganism carrying DNA according to any of [1] to [4] below:
[1] DNA encoding the protein according to any of [1] to [4] of Claim 3;
[2] DNA having the nucleotide sequence shown in any of SEQ ID NOs: 26 to 32, 64 and 65;
[3] DNA which hybridizes with DNA having the nucleotide sequence shown in any of SEQ ID NOs: 26 to 32, 64 and 65 under stringent conditions and which encodes a protein having the activity to synthesize a dipeptide; and
[4] DNA having a nucleotide sequence which has 80% or more homology to the nucleotide sequence shown in SEQ ID NO: 34 and encoding a protein having the activity to synthesize a dipeptide.

5. The process according to any one of claims 1 to 4, wherein the microorganism which has the ability to produce a dipeptide by condensation and ligation of one or more kinds of amino acids is a microorganism carrying a recombinant DNA in which the DNA according to any of [1] to [4] of Claim 4 is ligated to a vector DNA.

6. The process according to any one of claims 1 to 5, wherein the dipeptide is a dipeptide represented by the following formula (I):
R1 - R2 (I)
(wherein R1 and R2, which may be the same or different, each represent an amino acid).

7. A process for producing a dipeptide, which comprises:
allowing an enzyme source, an L-amino acid ester and an L-amino acid to be present in an aqueous medium,
said enzyme source being a culture of a microorganism in which the activities of three or more kinds of peptidases are reduced or lost as compared to the parent strain, said parent strain belonging to the genus Escherichia, Bacillus, Corynebacterium or Saccharomyces, wherein said microorganism has the ability to produce a dipeptide, and wherein said ability to produce a dipeptide is the ability to form a dipeptide from an L-amino acid ester and an L-amino acid; or a treated matter of the culture;
allowing the dipeptide to form and accumulate in the aqueous medium; and
recovering the dipeptide from the medium.

8. The process according to claim 7, wherein the peptidase is a protein having the amino acid sequence shown in any of SEQ ID NOs: 1 to 4, or a protein having an amino acid sequence which has 80% or more homology to the amino acid sequence shown in any of SEQ ID NOs: 1 to 4 and having peptidase activity.

9. The process according to claim 7 or 8, wherein the microorganism having the ability to form a dipeptide from an L-amino acid ester and an L-amino acid is a microorganism producing proline iminopeptidase.

10. The process according to any one of claims 7 to 9, wherein the microorganism having the ability to form a dipeptide from an L-amino acid ester and an L-amino acid is a microorganism carrying a recombinant DNA in which DNA encoding a protein having proline iminopeptidase activity is ligated to a vector DNA.

11. A process for producing a dipeptide, which comprises:
allowing an enzyme source, an L-amino acid amide and an L-amino acid to be present in an aqueous medium,
said enzyme source being a culture of a microorganism in which the activities of three or more kinds of peptidases are reduced or lost as compared to the parent strain, said parent strain belonging to the genus Escherichia, Bacillus, Corynebacterium or Saccharomyces, wherein said microorganism has the ability to produce a dipeptide, and wherein said ability to produce a dipeptide is the ability to form a dipeptide from an L-amino acid amide and an L-amino acid; or a treated matter of the culture;
allowing the dipeptide to form and accumulate in the aqueous medium; and
recovering the dipeptide from the medium.

12. The process according to claim 11, wherein the peptidase is a protein having the amino acid sequence shown in any of SEQ ID NOs: 1 to 4, or a protein having an amino acid sequence which has 80% or more homology to the amino acid sequence shown in any of SEQ ID NOs: 1 to 4 and having peptidase activity.

13. The process according to claim 11 or 12, wherein the microorganism having the ability to form a dipeptide from an L-amino acid amide and an L-amino acid is a microorganism producing a protein having L-amino acid amide hydrolase activity.

14. The process according to any one of claims 11 to 13, wherein the microorganism having the ability to form a dipeptide from an L-amino acid amide and an L-amino acid is a microorganism carrying a recombinant DNA in which DNA encoding a protein having L-amino acid amide hydrolase activity is ligated to a vector DNA.

15. The process according to any one of claims 1 to 14, wherein the treated matter of the culture is concentrated culture, dried culture, cells obtained by centrifuging the culture, or a product obtained by subjecting the cells to drying, freeze-drying, treatment with a surfactant, ultrasonication, mechanical friction, treatment with a solvent, enzymatic treatment or immobilization.

16. The process according to any one of claims 1 to 15, further comprising the formulation of the produced dipeptide into a food additive, a pharmaceutical composition or a cosmetic.

## Patentansprüche

1. Verfahren zur Herstellung eines Dipeptides, das umfasst:
Ermöglichen, dass eine Enzymquelle und eine oder mehrere Arten von Aminosäuren in einem wässrigen Medium vorhanden sind,
wobei die Enzymquelle eine Mikroorganismenkultur ist, in der die Aktivitäten von drei oder mehr Arten von Peptidasen im Vergleich zum parentalen Stamm reduziert oder verloren gegangen sind, wobei der parentale Stamm zur Gattung *Escherichia, Bacillus, Corynebacterium* oder *Saccharomyces* gehört, wobei der Mikroorganismus ein Dipeptid herstellen kann, und wobei die Fähigkeit zur Herstellung eines Dipeptides die Fähigkeit ist, ein Dipeptid mittels Kondensation und Ligation einer oder mehrerer Arten von Aminosäuren zu bilden; oder ein behandeltes Material der Kultur ist;
Ermöglichen, dass sich das Dipeptid bildet und in dem wässrigen Medium ansammelt; und
Gewinnen des Dipeptides aus dem Medium.

2. Verfahren nach Anspruch 1, wobei die Peptidase ein Protein ist, das die Aminosäuresequenz wie in einer der SEQ ID Nummern: 1 bis 4 gezeigt hat, oder ein Protein ist, das eine Aminosäuresequenz hat, die 80% oder eine höhere Homologie zu der Aminosäuresequenz hat, die in einer der SEQ ID Nummern: 1 bis 4 gezeigt ist, und Peptidase-Aktivität hat.

3. Verfahren nach Anspruch 1 oder 2, wobei der Mikroorganismus, der ein Dipeptid mittels Kondensation und Ligation einer oder mehrerer Arten von Aminosäuren herstellen kann, ein Mikroorganismus ist, der ein Protein gemäß einem der unten aufgeführten Punkte [1] bis [4] herstellen kann:
[1] ein Protein, das die Aminosäuresequenz wie in einer der SEQ ID Nummern: 19 bis 25 und 68 gezeigt hat;
[2] ein Protein bestehend aus einer Aminosäuresequenz in der ein oder mehrere Aminosäurereste in der Aminosäuresequenz, die in den SEQ ID Nummern: 19 bis 25 und 68 gezeigt ist deletiert, substituiert oder hinzugefügt ist/sind, und das eine Dipeptidsynthese-Aktivität hat;
[3] ein Protein bestehend aus einer Aminosäuresequenz, die 65% oder eine höhere Homologie zu der Aminosäuresequenz hat, die in einer der SEQ ID Nummern: 19 bis 25 und 68 gezeigt ist, und das eine Dipeptidsynthese-Aktivität hat; und
[4] ein Protein, das eine Aminosäuresequenz hat, die 80% oder eine höhere Homologie zu der Aminosäuresequenz hat, die in SEQ ID Nr: 33 gezeigt ist, und das eine Dipeptidsynthese-Aktivität hat.

4. Verfahren nach einem der Ansprüche 1 bis 3, wobei der Mikroorganismus, der ein Dipeptid mittels Kondensation und Ligation einer oder mehrerer Arten von Aminosäuren herstellen kann, ein Mikroorganismus ist, der eine DNA gemäß einem der unten aufgeführten Punkte [1] bis [4] enthält:
[1] DNA, die ein Protein gemäß einem der Punkte [1] bis [4] aus Anspruch 3 kodiert;
[2] DNA, die die Nukleotidsequenz wie in einer der SEQ ID Nummern: 26 bis 32, 64 und 65 gezeigt hat;
[3] DNA, die unter stringenten Bedingungen mit DNA hybridisiert, die die Nukleotidsequenz wie in einer der SEQ ID Nummern: 26 bis 32, 64 und 65 gezeigt hat, und die ein Protein kodiert, das eine Dipeptidsynthese-Aktivität hat; und
[4] DNA, die eine Nukleotidsequenz hat, die 80% oder eine höhere Homologie zu der in SEQ ID Nr: 34 gezeigten Nukleotidsequenz hat, und die ein Protein kodiert, das eine Dipeptidsynthese-Aktivität hat.

5. Verfahren nach einem der Ansprüche 1 bis 4, wobei der Mikroorganismus, der ein Dipeptid mittels Kondensation und Ligation einer oder mehrerer Aminosäuren herstellen kann, ein Mikroorganismus ist, der eine rekombinante DNA enthält, in der die DNA nach einem der Punkte [1] bis [4] des Anspruchs 4 an eine Vektor-DNA ligiert ist.

6. Verfahren nach einem der Ansprüche 1 bis 5, wobei das Dipeptid ein durch die folgende Formel (I) repräsentiertes Dipeptid ist:
R1 - R2 (I)
(wobei R1 und R2, die gleich oder unterschiedlich sein können, jeweils eine Aminosäure darstellen).

7. Verfahren zur Herstellung eines Dipeptides, das umfasst:
Ermöglichen, dass eine Enzymquelle, ein L-Aminosäureester und eine L-Aminosäure in einem wässrigen Medium vorhanden sind,
wobei die Enzymquelle eine Mikroorganismenkultur ist, in der die Aktivitäten von drei oder mehr Arten von Peptidasen im Vergleich zum parentalen Stamm reduziert oder verloren gegangen sind, wobei der parentale Stamm zur Gattung *Escherichia, Bacillus, Corynebacterium* oder *Saccharomyces* gehört, wobei der Mikroorganismus ein Dipeptid herstellen kann, und wobei die Fähigkeit zur Herstellung eines Dipeptides die Fähigkeit ist, ein Dipeptid aus einem L-Aminosäureester und einer L-Aminosäure zu bilden; oder ein behandeltes Material der Kultur ist;
Ermöglichen, dass sich das Dipeptid bildet und in dem wässrigen Medium ansammelt; und
Gewinnen des Dipeptides aus dem Medium.

8. Verfahren nach Anspruch 7, wobei die Peptidase ein Protein ist, das die Aminosäuresequenz wie in einer der SEQ ID Nummern: 1 bis 4 gezeigt hat, oder ein Protein ist, das eine Aminosäuresequenz hat, die 80% oder eine höhrere Homologie zu der Aminosäuresequenz hat, die in einer der SEQ ID Nummern: 1 bis 4 gezeigt ist, und Peptidase-Aktivität hat.

9. Verfahren nach Anspruch 7 oder 8, wobei der Mikroorganismus, der ein Dipeptid aus einem L-Aminosäureester und einer L-Aminosäure bilden kann, ein Mikroorganismus ist, der Prolin-Iminopeptidase herstellt.

10. Verfahren nach einem der Ansprüche 7 bis 9, wobei der Mikroorganismus, der ein Dipeptid aus einem L-Aminosäureester und einer L-Aminosäure bilden kann ein Mikroorganismus ist, der eine rekombinante DNA enthält, in der DNA, die ein Protein mit Prolin-Iminopeptidase-Aktivität kodiert, an eine Vektor-DNA ligiert ist.

11. Verfahren zur Herstellung eines Dipeptides, das umfasst:
Ermöglichen, dass eine Enzymquelle, ein L-Aminosäureamid und eine L-Aminosäure in einem wässrigen Medium vorhanden sind,
wobei die Enzymquelle eine Mikroorganismenkultur ist, in der die Aktivitäten von drei oder mehr Arten von Peptidasen im Vergleich zum parentalen Stamm reduziert oder verloren gegangen sind, wobei der parentale Stamm zur Gattung *Escherichia, Bacillus, Corynebacterium* oder *Saccharomyces* gehört, wobei der Mikroorganismus ein Dipeptid herstellen kann, und wobei die Fähigkeit zur Herstellung eines Dipeptides die Fähigkeit ist, ein Dipeptid aus einem L-Aminosäureamid und einer L-Aminosäure zu bilden; oder ein behandeltes Material der Kultur ist;
Ermöglichen, dass sich das Dipeptid bildet und in dem wässrigen Medium ansammelt; und
Gewinnen des Dipeptides aus dem Medium.

12. Verfahren nach Anspruch 11, wobei die Peptidase ein Protein ist, das die Aminosäuresequenz wie in einer der SEQ ID Nummern: 1 bis 4 gezeigt hat, oder ein Protein ist, das eine Aminosäuresequenz hat, die 80% oder eine höhere Homologie zu der Aminosäuresequenz hat, die in einer der SEQ ID Nummern: 1 bis 4 gezeigt ist, und Peptidase-Aktivität hat.

13. Verfahren nach Anspruch 11 oder 12, wobei der Mikroorganismus, der ein Dipeptid aus einem L-Aminosäureamid und einer L-Aminosäure bilden kann ein Mikroorganismus ist, der ein Protein herstellt, dass L-Aminosäureamidhydrolase-Aktivität hat.

14. Verfahren nach einem der Ansprüche 11 bis 13, wobei der Mikroorganismus, der ein Dipeptid aus einem L-Aminosäureamid und einer L-Aminosäure bilden kann ein Mikroorganismus ist, der eine rekombinante DNA enthält, in der DNA, die ein Protein mit L-Aminosäureamidhydrolase-Aktivität kodiert, an eine Vektor-DNA ligiert ist.

15. Verfahren nach einem der Ansprüche 1 bis 14, wobei das behandelte Material der Kultur eine konzentrierte Kultur, getrocknete Kultur, Zellen, die durch Zentrifugation der Kultur erhalten wurden oder ein Produkt ist, das dadurch erhalten wurde, dass die Zellen einer Trocknung, Gefriertrocknung, Behandlung mit einem oberflächenaktivem Stoff, Ultraschall, mechanischer Reibung, Behandlung mit einem Lösungsmittel, enzymatischer Behandlung oder Immobilisierung unterworfen wurden.

16. Verfahren nach einem der Ansprüche 1 bis 15, des weiteren umfassend die Formulierung des hergestellten Dipeptides in einen Nahrungsmittelzusatz, eine pharmazeutische Zusammensetzung oder ein Kosmetikum.

## Revendications

1. Procédé de production d'un dipeptide, qui comprend :
l'étape permettant à une source enzymatique et à un ou plusieurs types d'acides aminés d'être présents dans un milieu aqueux,
ladite source enzymatique étant une culture d'un micro-organisme dans lequel les activités de trois types ou plus de peptidases sont réduites ou perdues comparativement à la souche parente, ladite souche parente appartenant au genre Escherichia, Bacillus, Corynebacterium ou Saccharomyces, dans lequel ledit micro-organisme a la capacité de produire un dipeptide, et dans lequel ladite capacité de produire un dipeptide est la capacité de former un dipeptide par condensation et ligature d'un ou de plusieurs types d'acides aminés ; ou une matière traitée de la culture ;
l'étape permettant au dipeptide de se former et de s'accumuler dans le milieu aqueux ; et
la récupération du dipeptide à partir du milieu.

2. Procédé selon la revendication 1, dans lequel la peptidase est une protéine ayant la séquence d'acides aminés représentée dans l'une quelconque de SEQ ID NO : 1 à 4, ou une protéine ayant une séquence d'acides aminés qui présente 80 % d'homologie ou plus avec la séquence d'acides aminés représentée dans l'une quelconque de SEQ ID NO : 1 à 4 et dotée d'une activité peptidase.

3. Procédé selon la revendication 1 ou 2, dans lequel le micro-organisme qui a la capacité de produire un dipeptide par condensation et ligature d'un ou de plusieurs types d'acides aminés est un micro-organisme ayant la capacité de produire une protéine selon l'un quelconque de [1] à [4] ci-dessous :
[1] une protéine ayant la séquence d'acides aminés représentée dans l'une quelconque de SEQ ID NO : 19 à 25 et 68 ;
[2] une protéine constituée d'une séquence d'acides aminés dans laquelle un ou plusieurs résidus d'acides aminés sont délétés, substitués ou ajoutés dans la séquence d'acides aminés représentée dans l'une quelconque de SEQ ID NO : 19 à 25 et 68 et dotée de l'activité de synthétiser un dipeptide ;
[3] une protéine constituée d'une séquence d'acides aminés qui présente 65 % d'homologie ou plus avec la séquence d'acides aminés représentée dans l'une quelconque de SEQ ID NO : 19 à 25 et 68 et dotée de l'activité de synthétiser un dipeptide ; et
[4] une protéine ayant une séquence d'acides aminés qui présente 80 % d'homologie ou plus avec la séquence d'acides aminés représentée dans SEQ ID NO : 33 et dotée de l'activité de synthétiser un dipeptide.

4. Procédé selon l'une quelconque des revendications 1 à 3, dans lequel le micro-organisme qui a la capacité de produire un dipeptide par condensation et ligature d'un ou de plusieurs types d'acides aminés est un micro-organisme portant un ADN selon l'un quelconque de [1] à [4] ci-dessous :
[1] un ADN codant pour la protéine selon l'un quelconque de [1] à [4] de la revendication 3 ;
[2] un ADN ayant la séquence nucléotidique représentée dans l'une quelconque de SEQ ID NO : 26 à 32, 64 et 65 ;
[3] un ADN qui s'hybride avec l'ADN ayant la séquence nucléotidique représentée dans l'une quelconque de SEQ ID NO : 26 à 32, 64 et 65 dans des conditions stringentes et qui code pour une protéine dotée de l'activité de synthétiser un dipeptide ; et
[4] un ADN ayant une séquence nucléotidique qui présente 80 % d'homologie ou plus avec la séquence nucléotidique représentée dans SEQ ID NO : 34 et codant pour une protéine dotée de l'activité de synthétiser un dipeptide.

5. Procédé selon l'une quelconque des revendications 1 à 4, dans lequel le micro-organisme qui a la capacité de produire un dipeptide par condensation et ligature d'un ou de plusieurs types d'acides aminés est un micro-organisme portant un ADN recombinant dans lequel l'ADN selon l'un quelconque de [1] à [4] de la revendication 4 est ligaturé à un ADN de vecteur.

6. Procédé selon l'une quelconque des revendications 1 à 5, dans lequel le dipeptide est un dipeptide représenté par la formule (I) suivante :
R1 - R2 (I)
(dans laquelle R1 et R2, qui peuvent être identiques ou différents, représentent chacun un acide aminé).

7. Procédé de production d'un dipeptide, qui comprend :
l'étape permettant à une source enzymatique, à un ester d'acide aminé L et à un acide aminé L d'être présents dans un milieu aqueux,
ladite source enzymatique étant une culture d'un micro-organisme dans lequel les activités de trois types ou plus de peptidases sont réduites ou perdues comparativement à la souche parente, ladite souche parente appartenant au genre Escherichia, Bacillus, Corynebacterium ou Saccharomyces, dans lequel ledit micro-organisme a la capacité de produire un dipeptide, et dans lequel ladite capacité de produire un dipeptide est la capacité de former un dipeptide à partir d'un ester d'acide aminé L et d'un acide aminé L ; ou une matière traitée de la culture ;
l'étape permettant au dipeptide de se former et de s'accumuler dans le milieu aqueux ; et
la récupération du dipeptide à partir du milieu.

8. Procédé selon la revendication 7, dans lequel la peptidase est une protéine ayant la séquence d'acides aminés représentée dans l'une quelconque de SEQ ID NO : 1 à 4, ou une protéine ayant une séquence d'acides aminés qui présente 80 % d'homologie ou plus avec la séquence d'acides aminés représentée dans l'une quelconque de SEQ ID NO : 1 à 4 et dotée d'une activité peptidase.

9. Procédé selon la revendication 7 ou 8, dans lequel le micro-organisme ayant la capacité de former un dipeptide à partir d'un ester d'acide aminé L et d'un acide aminé L est un micro-organisme produisant de la proline iminopeptidase.

10. Procédé selon l'une quelconque des revendications 7 à 9, dans lequel le micro-organisme ayant la capacité de former un dipeptide à partir d'un ester d'acide aminé L et d'un acide aminé L est un micro-organisme portant un ADN recombinant dans lequel l'ADN codant pour une protéine dotée d'une activité proline iminopeptidase est ligaturé à un ADN de vecteur.

11. Procédé de production d'un dipeptide, qui comprend :
l'étape permettant à une source enzymatique, à un amide d'acide aminé L et à un acide aminé L d'être présents dans un milieu aqueux,
ladite source enzymatique étant une culture d'un micro-organisme dans lequel les activités de trois types ou plus de peptidases sont réduites ou perdues comparativement à la souche parente, ladite souche parente appartenant au genre Escherichia, Bacillus, Corynebacterium ou Saccharomyces, dans lequel ledit micro-organisme a la capacité de produire un dipeptide, et dans lequel ladite capacité de produire un dipeptide est la capacité de former un dipeptide à partir d'un amide d'acide aminé L et d'un acide aminé L ; ou une matière traitée de la culture ;
l'étape permettant au dipeptide de se former et de s'accumuler dans le milieu aqueux ; et
la récupération du dipeptide à partir du milieu.

12. Procédé selon la revendication 11, dans lequel la peptidase est une protéine ayant la séquence d'acides aminés représentée dans l'une quelconque de SEQ ID NO : 1 à 4, ou une protéine ayant une séquence d'acides aminés qui présente 80 % d'homologie ou plus avec la séquence d'acides aminés représentée dans l'une quelconque de SEQ ID NO : 1 à 4 et dotée d'une activité peptidase.

13. Procédé selon la revendication 11 ou 12, dans lequel le micro-organisme ayant la capacité de former un dipeptide à partir d'un amide d'acide aminé L et d'un acide aminé L est un micro-organisme produisant une protéine dotée d'une activité amide d'acide aminé L hydrolase.

14. Procédé selon l'une quelconque des revendications 11 à 13, dans lequel le micro-organisme ayant la capacité de former un dipeptide à partir d'un amide d'acide aminé L et d'un acide aminé L est un micro-organisme portant un ADN recombinant dans lequel l'ADN codant pour une protéine dotée d'une activité amide d'acide aminé L hydrolase est ligaturé à un ADN de vecteur.

15. Procédé selon l'une quelconque des revendications 1 à 14, dans lequel la matière traitée de la culture est une culture concentrée, une culture séchée, des cellules obtenues par centrifugation de la culture, ou un produit obtenu par soumission des cellules à un séchage, une cryodessiccation, un traitement avec un tensioactif, des ultrasons, une friction mécanique, un traitement avec un solvant, un traitement enzymatique ou une immobilisation.

16. Procédé selon l'une quelconque des revendications 1 à 15, comprenant en outre la formulation du dipeptide produit dans un additif alimentaire, une composition pharmaceutique ou un produit cosmétique.


### SEQUENCE LISTING

<110> KYOWA HAKKO KOGYO CO., LTD.
<120> Microorganisms producing dipeptides and process for producing dipeptides
   using the microorganisms
<130> K 2817 EP/1
<150> JP 2003-375823
   <151> 2003-11-5
<150> JP 2004-189010
   <151> 2004-06-25
<160> 72
<170> PatentIn Ver. 2.1
<210> 1
   <211> 503
   <212> PRT
   <213> Escherichia coli
<400> 1
   Met Glu Phe Ser Val Lys Ser Gly Ser Pro Glu Lys Gln Arg Ser Ala
    1               5                  10                  15
   Cys Ile Val Val Gly Val Phe Glu Pro Arg Arg Leu Ser Pro Ile Ala
               20                  25                  30
   Glu Gln Leu Asp Lys Ile Ser Asp Gly Tyr Ile Ser Ala Leu Leu Arg
           35                  40                  45
   Arg Gly Glu Leu Glu Gly Lys Pro Gly Gln Thr Leu Leu Leu His His
       50                  55                  60
   Val Pro Asn Val Leu Ser Glu Arg Ile Leu Leu Ile Gly Cys Gly Lys
   65                  70                  75                  80
   Glu Arg Glu Leu Asp Glu Arg Gln Tyr Lys Gln Val Ile Gln Lys Thr
                   85                  90                  95
   Ile Asn Thr Leu Asn Asp Thr Gly Ser Met Glu Ala Val Cys Phe Leu
               100                 105                 110
   Thr Glu Leu His Val Lys Gly Arg Asn Asn Tyr Trp Lys Val Arg Gln
           115                 120                 125
   Ala Val Glu Thr Ala Lys Glu Thr Leu Tyr Ser Phe Asp Gln Leu Lys
       130                 135                 140
   Thr Asn Lys Ser Glu Pro Arg Arg Pro Leu Arg Lys Met Val Phe Asn
   145                 150                 155                 160
   Val Pro Thr Arg Arg Glu Leu Thr Ser Gly Glu Arg Ala Ile Gln His
                   165                 170                 175
   Gly Leu Ala Ile Ala Ala Gly Ile Lys Ala Ala Lys Asp Leu Gly Asn
               180                 185                 190
   Met Pro Pro Asn Ile Cys Asn Ala Ala Tyr Leu Ala Ser Gln Ala Arg
           195                 200                 205
   Gln Leu Ala Asp Ser Tyr Ser Lys Asn Val Ile Thr Arg Val Ile Gly
       210                 215                 220
   Glu Gln Gln Met Lys Glu Leu Gly Met His Ser Tyr Leu Ala Val Gly
   225                 230                 235                 240
   Gln Gly Ser Gln Asn Glu Ser Leu Met Ser Val Ile Glu Tyr Lys Gly
   245 250 255
   Asn Ala Ser Glu Asp Ala Arg Pro Ile Val Leu Val Gly Lys Gly Leu
   260 265 270
   Thr Phe Asp Ser Gly Gly Ile Ser Ile Lys Pro Ser Glu Gly Met Asp
   275 280 285
   Glu Met Lys Tyr Asp Met Cys Gly Ala Ala Ala Val Tyr Gly Val Met
   290 295 300
   Arg Met Val Ala Glu Leu Gln Leu Pro Ile Asn Val Ile Gly Val Leu
   305 310 315 320
   Ala Gly Cys Glu Asn Met Pro Gly Gly Arg Ala Tyr Arg Pro Gly Asp
   325 330 335
   Val Leu Thr Thr Met Ser Gly Gln Thr Val Glu Val Leu Asn Thr Asp
   340 345 350
   Ala Glu Gly Arg Leu Val Leu Cys Asp Val Leu Thr Tyr Val Glu Arg
   355 360 365
   Phe Glu Pro Glu Ala Val Ile Asp Val Ala Thr Leu Thr Gly Ala Cys
   370 375 380
   Val Ile Ala Leu Gly His His Ile Thr Gly Leu Met Ala Asn His Asn
   385 390 395 400
   Pro Leu Ala His Glu Leu Ile Ala Ala Ser Glu Gln Ser Gly Asp Arg
   405 410 415
   Ala Trp Arg Leu Pro Leu Gly Asp Glu Tyr Gln Glu Gln Leu Glu Ser
   420 425 430
   Asn Phe Ala Asp Met Ala Asn Ile Gly Gly Arg Pro Gly Gly Ala Ile
   435 440 445
   Thr Ala Gly Cys Phe Leu Ser Arg Phe Thr Arg Lys Tyr Asn Trp Ala
   450 455 460
   His Leu Asp Ile Ala Gly Thr Ala Trp Arg Ser Gly Lys Ala Lys Gly
   465 470 475 480
   Ala Thr Gly Arg Pro Val Ala Leu Leu Ala Gln Phe Leu Leu Asn Arg
   485 490 495
   Ala Gly Phe Asn Gly Glu Glu
   500
<210> 2
   <211> 427
   <212> PRT
   <213> Escherichia coli
<400> 2
   Met Thr Glu Ala Met Lys Ile Thr Leu Ser Thr Gln Pro Ala Asp Ala
   1 5 10 15
   Arg Trp Gly Glu Lys Ala Thr Tyr Ser Ile Asn Asn Asp Gly Ile Thr
   20 25 30
   Leu His Leu Asn Gly Ala Asp Asp Leu Gly Leu Ile Gln Arg Ala Ala
   35 40 45
   Arg Lys Ile Asp Gly Leu Gly Ile Lys His Val Gln Leu Ser Gly Glu
       50                  55                  60
   Gly Trp Asp Ala Asp Arg Cys Trp Ala Phe Trp Gln Gly Tyr Lys Ala
   65                  70                  75                  80
   Pro Lys Gly Thr Arg Lys Val Val Trp Pro Asp Leu Asp Asp Ala Gln
                   85                  90                  95
   Arg Gln Glu Leu Asp Asn Arg Leu Met Ile Ile Asp Trp Val Arg Asp
               100                 105                 110
   Thr Ile Asn Ala Pro Ala Glu Glu Leu Gly Pro Ser Gln Leu Ala Gln
           115                 120                 125
   Arg Ala Val Asp Leu Ile Ser Asn Val Ala Gly Asp Arg Val Thr Tyr
       130                 135                 140
   Arg Ile Thr Lys Gly Glu Asp Leu Arg Glu Gln Gly Tyr Met Gly Leu
   145                 150                 155                 160
   His Thr Val Gly Arg Gly Ser Glu Arg Ser Pro Val Leu Leu Ala Leu
                   165                 170                 175
   Asp Tyr Asn Pro Thr Gly Asp Lys Glu Ala Pro Val Tyr Ala Cys Leu
               180                 185                 190
   Val Gly Lys Gly Ile Thr Phe Asp Ser Gly Gly Tyr Ser Ile Lys Gln
           195                 200                 205
   Thr Ala Phe Met Asp Ser Met Lys Ser Asp Met Gly Gly Ala Ala Thr
       210                 215                 220
   Val Thr Gly Ala Leu Ala Phe Ala Ile Thr Arg Gly Leu Asn Lys Arg
   225                 230                 235                 240
   Val Lys Leu Phe Leu Cys Cys Ala Asp Asn Leu Ile Ser Gly Asn Ala
                   245                 250                 255
   Phe Lys Leu Gly Asp Ile Ile Thr Tyr Arg Asn Gly Lys Lys Val Glu
               260                 265                 270
   Val Met Asn Thr Asp Ala Glu Gly Arg Leu Val Leu Ala Asp Gly Leu
           275                 280                 285
   Ile Asp Ala Ser Ala Gln Lys Pro Glu Met Ile Ile Asp Ala Ala Thr
       290                 295                 300
   Leu Thr Gly Ala Ala Lys Thr Ala Leu Gly Asn Asp Tyr His Ala Leu
   305                 310                 315                 320
   Phe Ser Phe Asp Asp Ala Leu Ala Gly Arg Leu Leu Ala Ser Ala Ala
                   325                 330                 335
   Gln Glu Asn Glu Pro Phe Trp Arg Leu Pro Leu Ala Glu Phe His Arg
               340                 345                 350
   Ser Gln Leu Pro Ser Asn Phe Ala Glu Leu Asn Asn Thr Gly Ser Ala
           355                 360                 365
   Ala Tyr Pro Ala Gly Ala Ser Thr Ala Ala Gly Phe Leu Ser His Phe
       370                 375                 380
   Val Glu Asn Tyr Gln Gln Gly Trp Leu His Ile Asp Cys Ser Ala Thr
   385                 390                 395                 400
   Tyr Arg Lys Ala Pro Val Glu Gln Trp Ser Ala Gly Ala Thr Gly Leu
                   405                 410                 415
   Gly Val Arg Thr Ile Ala Asn Leu Leu Thr Ala
               420                 425
<210> 3
   <211> 485
   <212> PRT
   <213> Escherichia coli
<400> 3
   Met Ser Glu Leu Ser Gln Leu Ser Pro Gln Pro Leu Trp Asp Ile Phe
   1 5 10 15
   Ala Lys Ile Cys Ser Ile Pro His Pro Ser Tyr His Glu Glu Gln Leu
   20 25 30
   Ala Glu Tyr Ile Val Gly Trp Ala Lys Glu Lys Gly Phe His Val Glu
   35 40 45
   Arg Asp Gln Val Gly Asn Ile Leu Ile Arg Lys Pro Ala Thr Ala Gly
   50 55 60
   Met Glu Asn Arg Lys Pro Val Val Leu Gln Ala His Leu Asp Met Val
   65 70 75 80
   Pro Gln Lys Asn Asn Asp Thr Val His Asp Phe Thr Lys Asp Pro Ile
   85 90 95
   Gln Pro Tyr Ile Asp Gly Glu Trp Val Lys Ala Arg Gly Thr Thr Leu
   100 105 110
   Gly Ala Asp Asn Gly Ile Gly Met Ala Ser Ala Leu Ala Val Leu Ala
   115 120 125
   Asp Glu Asn Val Val His Gly Pro Leu Glu Val Leu Leu Thr Met Thr
   130 135 140
   Glu Glu Ala Gly Met Asp Gly Ala Phe Gly Leu Gln Gly Asn Trp Leu
   145 150 155 160
   Gln Ala Asp Ile Leu Ile Asn Thr Asp Ser Glu Glu Glu Gly Glu Ile
   165 170 175
   Tyr Met Gly Cys Ala Gly Gly Ile Asp Phe Thr Ser Asn Leu His Leu
   180 185 190
   Asp Arg Glu Ala Val Pro Ala Gly Phe Glu Thr Phe Lys Leu Thr Leu
   195 200 205
   Lys Gly Leu Lys Gly Gly His Ser Gly Gly Glu Ile His Val Gly Leu
   210 215 220
   Gly Asn Ala Asn Lys Leu Leu Val Arg Phe Leu Ala Gly His Ala Glu
   225 230 235 240
   Glu Leu Asp Leu Arg Leu Ile Asp Phe Asn Gly Gly Thr Leu Arg Asn
   245 250 255
   Ala Ile Pro Arg Glu Ala Phe Ala Thr Ile Ala Val Ala Ala Asp Lys
   260 265 270
   Val Asp Val Leu Lys Ser Leu Val Asn Thr Tyr Gln Glu Ile Leu Lys
   275 280 285
   Asn Glu Leu Ala Glu Lys Glu Lys Asn Leu Ala Leu Leu Leu Asp Ser
   290 295 300
   Val Ala Asn Asp Lys Ala Ala Leu Ile Ala Lys Ser Arg Asp Thr Phe
   305 310 315 320
   Ile Arg Leu Leu Asn Ala Thr Pro Asn Gly Val Ile Arg Asn Ser Asp
   325 330 335
   Val Ala Lys Gly Val Val Glu Thr Ser Leu Asn Val Gly Val Val Thr
   340 345 350
   Met Thr Asp Asn Asn Val Glu Ile His Cys Leu Ile Arg Ser Leu Ile
   355 360 365
   Asp Ser Gly Lys Asp Tyr Val Val Ser Met Leu Asp Ser Leu Gly Lys
   370 375 380
   Leu Ala Gly Ala Lys Thr Glu Ala Lys Gly Ala Tyr Pro Gly Trp Gln
   385 390 395 400
   Pro Asp Ala Asn Ser Pro Val Met His Leu Val Arg Glu Thr Tyr Gln
   405 410 415
   Arg Leu Phe Asn Lys Thr Pro Asn Ile Gln Ile Ile His Ala Gly Leu
   420 425 430
   Glu Cys Gly Leu Phe Lys Lys Pro Tyr Pro Glu Met Asp Met Val Ser
   435 440 445
   Ile Gly Pro Thr Ile Thr Gly Pro His Ser Pro Asp Glu Gln Val His
   450 455 460
   Ile Glu Ser Val Gly His Tyr Trp Thr Leu Leu Thr Glu Leu Leu Lys
   465 470 475 480
   Glu Ile Pro Ala Lys
   485
<210> 4
   <211> 870
   <212> PRT
   <213> Escherichia coli
<400> 4
   Met Thr Gln Gln Pro Gln Ala Lys Tyr Arg His Asp Tyr Arg Ala Pro
   1               5                   10                  15
   Asp Tyr Gln Ile Thr Asp Ile Asp Leu Thr Phe Asp Leu Asp Ala Gln
               20                  25                  30
   Lys Thr Val Val Thr Ala Val Ser Gln Ala Val Arg His Gly Ala Ser
           35                  40                  45
   Asp Ala Pro Leu Arg Leu Asn Gly Glu Asp Leu Lys Leu Val Ser Val
       50                  55                  60
   His Ile Asn Asp Glu Pro Trp Thr Ala Trp Lys Glu Glu Glu Gly Ala
   65                  70                  75                  80
   Leu Val Ile Ser Asn Leu Pro Glu Arg Phe Thr Leu Lys Ile Ile Asn
                   85                  90                  95
   Glu Ile Ser Pro Ala Ala Asn Thr Ala Leu Glu Gly Leu Tyr Gln Ser
               100                 105                 110
   Gly Asp Ala Leu Cys Thr Gln Cys Glu Ala Glu Gly Phe Arg His Ile
           115                 120                 125
   Thr Tyr Tyr Leu Asp Arg Pro Asp Val Leu Ala Arg Phe Thr Thr Lys
       130                 135                 140
   Ile Ile Ala Asp Lys Ile Lys Tyr Pro Phe Leu Leu Ser Asn Gly Asn
   145 150 155 160
   Arg Val Ala Gln Gly Glu Leu Glu Asn Gly Arg His Trp Val Gln Trp
   165 170 175
   Gln Asp Pro Phe Pro Lys Pro Cys Tyr Leu Phe Ala Leu Val Ala Gly
   180 185 190
   Asp Phe Asp Val Leu Arg Asp Thr Phe Thr Thr Arg Ser Gly Arg Glu
   195 200 205
   Val Ala Leu Glu Leu Tyr Val Asp Arg Gly Asn Leu Asp Arg Ala Pro
   210 215 220
   Trp Ala Met Thr Ser Leu Lys Asn Ser Met Lys Trp Asp Glu Glu Arg
   225 230 235 240
   Phe Gly Leu Glu Tyr Asp Leu Asp Ile Tyr Met Ile Val Ala Val Asp
   245 250 255
   Phe Phe Asn Met Gly Ala Met Glu Asn Lys Gly Leu Asn Ile Phe Asn
   260 265 270
   Ser Lys Tyr Val Leu Ala Arg Thr Asp Thr Ala Thr Asp Lys Asp Tyr
   275 280 285
   Leu Asp Ile Glu Arg Val Ile Gly His Glu Tyr Phe His Asn Trp Thr
   290 295 300
   Gly Asn Arg Val Thr Cys Arg Asp Trp Phe Gln Leu Ser Leu Lys Glu
   305 310 315 320
   Gly Leu Thr Val Phe Arg Asp Gln Glu Phe Ser Ser Asp Leu Gly Ser
   325 330 335
   Arg Ala Val Asn Arg Ile Asn Asn Val Arg Thr Met Arg Gly Leu Gln
   340 345 350
   Phe Ala Glu Asp Ala Ser Pro Met Ala His Pro Ile Arg Pro Asp Met
   355 360 365
   Val Ile Glu Met Asn Asn Phe Tyr Thr Leu Thr Val Tyr Glu Lys Gly
   370 375 380
   Ala Glu Val Ile Arg Met Ile His Thr Leu Leu Gly Glu Glu Asn Phe
   385 390 395 400
   Gln Lys Gly Met Gln Leu Tyr Phe Glu Arg His Asp Gly Ser Ala Ala
   405 410 415
   Thr Cys Asp Asp Phe Val Gln Ala Met Glu Asp Ala Ser Asn Val Asp
   420 425 430
   Leu Ser His Phe Arg Arg Trp Tyr Ser Gln Ser Gly Thr Pro Ile Val
   435 440 445
   Thr Val Lys Asp Asp Tyr Asn Pro Glu Thr Glu Gln Tyr Thr Leu Thr
   450 455 460
   Ile Ser Gln Arg Thr Pro Ala Thr Pro Asp Gln Ala Glu Lys Gln Pro
   465 470 475 480
   Leu His Ile Pro Phe Ala Ile Glu Leu Tyr Asp Asn Glu Gly Lys Val
   485 490 495
   Ile Pro Leu Gln Lys Gly Gly His Pro Val Asn Ser Val Leu Asn Val
   500 505 510
   Thr Gln Ala Glu Gln Thr Phe Val Phe Asp Asn Val Tyr Phe Gln Pro
   515 520 525
   Val Pro Ala Leu Leu Cys Glu Phe Ser Ala Pro Val Lys Leu Glu Tyr
   530 535 540
   Lys Trp Ser Asp Gln Gln Leu Thr Phe Leu Met Arg His Ala Arg Asn
   545 550 555 560
   Asp Phe Ser Arg Trp Asp Ala Ala Gln Ser Leu Leu Ala Thr Tyr Ile
   565 570 575
   Lys Leu Asn Val Ala Arg His Gln Gln Gly Gln Pro Leu Ser Leu Pro
   580 585 590
   Val His Val Ala Asp Ala Phe Arg Ala Val Leu Leu Asp Glu Lys Ile
   595 600 605
   Asp Pro Ala Leu Ala Ala Glu Ile Leu Thr Leu Pro Ser Val Asn Glu
   610 615 620
   Met Ala Glu Leu Phe Asp Ile Ile Asp Pro Ile Ala Ile Ala Glu Val
   625 630 635 640
   Arg Glu Ala Leu Thr Arg Thr Leu Ala Thr Glu Leu Ala Asp Glu Leu
   645 650 655
   Leu Ala Ile Tyr Asn Ala Asn Tyr Gln Ser Glu Tyr Arg Val Glu His
   660 665 670
   Glu Asp Ile Ala Lys Arg Thr Leu Arg Asn Ala Cys Leu Arg Phe Leu
   675 680 685
   Ala Phe Gly Glu Thr His Leu Ala Asp Val Leu Val Ser Lys Gln Phe
   690 695 700
   His Glu Ala Asn Asn Met Thr Asp Ala Leu Ala Ala Leu Ser Ala Ala
   705 710 715 720
   Val Ala Ala Gln Leu Pro Cys Arg Asp Ala Leu Met Gln Glu Tyr Asp
   725 730 735
   Asp Lys Trp His Gln Asn Gly Leu Val Met Asp Lys Trp Phe Ile Leu
   740 745 750
   Gln Ala Thr Ser Pro Ala Ala Asn Val Leu Glu Thr Val Arg Gly Leu
   755 760 765
   Leu Gln His Arg Ser Phe Thr Met Ser Asn Pro Asn Arg Ile Arg Ser
   770 775 780
   Leu Ile Gly Ala Phe Ala Gly Ser Asn Pro Ala Ala Phe His Ala Glu
   785 790 795 800
   Asp Gly Ser Gly Tyr Leu Phe Leu Val Glu Met Leu Thr Asp Leu Asn
   805 810 815
   Ser Arg Asn Pro Gln Val Ala Ser Arg Leu Ile Glu Pro Leu Ile Arg
   820 825 830
   Leu Lys Arg Tyr Asp Ala Lys Arg Gln Glu Lys Met Arg Ala Ala Leu
   835 840 845
   Glu Gln Leu Lys Gly Leu Glu Asn Leu Ser Gly Asp Leu Tyr Glu Lys
   850 855 860
   Ile Thr Lys Ala Leu Ala
   865 870
<210> 5
   <211> 535
   <212> PRT
   <213> Escherichia coli
<400> 5
   Met Arg Ile Ser Leu Lys Lys Ser Gly Met Leu Lys Leu Gly Leu Ser
   1               5                   10                  15
   Leu Val Ala Met Thr Val Ala Ala Ser Val Gln Ala Lys Thr Leu Val
               20                  25                  30
   Tyr Cys Ser Glu Gly Ser Pro Glu Gly Phe Asn Pro Gln Leu Phe Thr
           35                  40                  45
   Ser Gly Thr Thr Tyr Asp Ala Ser Ser Val Pro Leu Tyr Asn Arg Leu
       50                  55                  60
   Val Glu Phe Lys Ile Gly Thr Thr Glu Val Ile Pro Gly Leu Ala Glu
   65                  70                  75                  80
   Lys Trp Glu Val Ser Glu Asp Gly Lys Thr Tyr Thr Phe His Leu Arg
                   85                  90                  95
   Lys Gly Val Lys Trp His Asp Asn Lys Glu Phe Lys Pro Thr Arg Glu
               100                 105                 110
   Leu Asn Ala Asp Asp Val Val Phe Ser Phe Asp Arg Gln Lys Asn Ala
           115                 120                 125
   Gln Asn Pro Tyr His Lys Val Ser Gly Gly Ser Tyr Glu Tyr Phe Glu
       130                 135                 140
   Gly Met Gly Leu Pro Glu Leu Ile Ser Glu Val Lys Lys Val Asp Asp
   145                 150                 155                 160
   Asn Thr Val Gln Phe Val Leu Thr Arg Pro Glu Ala Pro Phe Leu Ala
                   165                 170                 175
   Asp Leu Ala Met Asp Phe Ala Ser Ile Leu Ser Lys Glu Tyr Ala Asp
               180                 185                 190
   Ala Met Met Lys Ala Gly Thr Pro Glu Lys Leu Asp Leu Asn Pro Ile
           195                 200                 205
   Gly Thr Gly Pro Phe Gln Leu Gln Gln Tyr Gln Lys Asp Ser Arg Ile
       210                 215                 220
   Arg Tyr Lys Ala Phe Asp Gly Tyr Trp Gly Thr Lys Pro Gln Ile Asp
   225                 230                 235                 240
   Thr Leu Val Phe Ser Ile Thr Pro Asp Ala Ser Val Arg Tyr Ala Lys
                   245                 250                 255
   Leu Gln Lys Asn Glu Cys Gln Val Met Pro Tyr Pro Asn Pro Ala Asp
               260                 265                 270
   Ile Ala Arg Met Lys Gln Asp Lys Ser Ile Asn Leu Met Glu Met Pro
           275                 280                 285
   Gly Leu Asn Val Gly Tyr Leu Ser Tyr Asn Val Gln Lys Lys Pro Leu
       290                 295                 300
   Asp Asp Val Lys Val Arg Gln Ala Leu Thr Tyr Ala Val Asn Lys Asp
   305 310 315 320
   Ala Ile Ile Lys Ala Val Tyr Gln Gly Ala Gly Val Ser Ala Lys Asn
   325 330 335
   Leu Ile Pro Pro Thr Met Trp Gly Tyr Asn Asp Asp Val Gln Asp Tyr
   340 345 350
   Thr Tyr Asp Pro Glu Lys Ala Lys Ala Leu Leu Lys Glu Ala Gly Leu
   355 360 365
   Glu Lys Gly Phe Ser Ile Asp Leu Trp Ala Met Pro Val Gln Arg Pro
   370 375 380
   Tyr Asn Pro Asn Ala Arg Arg Met Ala Glu Met Ile Gln Ala Asp Trp
   385 390 395 400
   Ala Lys Val Gly Val Gln Ala Lys Ile Val Thr Tyr Glu Trp Gly Glu
   405 410 415
   Tyr Leu Lys Arg Ala Lys Asp Gly Glu His Gln Thr Val Met Met Gly
   420 425 430
   Trp Thr Gly Asp Asn Gly Asp Pro Asp Asn Phe Phe Ala Thr Leu Phe
   435 440 445
   Ser Cys Ala Ala Ser Glu Gln Gly Ser Asn Tyr Ser Lys Trp Cys Tyr
   450 455 460
   Lys Pro Phe Glu Asp Leu Ile Gln Pro Ala Arg Ala Thr Asp Asp His
   465 470 475 480
   Asn Lys Arg Val Glu Leu Tyr Lys Gln Ala Gln Val Val Met His Asp
   485 490 495
   Gln Ala Pro Ala Leu Ile Ile Ala His Ser Thr Val Phe Glu Pro Val
   500 505 510
   Arg Lys Glu Val Lys Gly Tyr Val Val Asp Pro Leu Gly Lys His His
   515 520 525
   Phe Glu Asn Val Ser Ile Glu
   530 535
<210> 6
   <211> 339
   <212> PRT
   <213> Escherichia coli
<400> 6
   Met Leu Gln Phe Ile Leu Arg Arg Leu Gly Leu Val Ile Pro Thr Phe
   1               5                   10                  15
   Ile Gly Ile Thr Leu Leu Thr Phe Ala Phe Val His Met Ile Pro Gly
               20                  25                  30
   Asp Pro Val Met Ile Met Ala Gly Glu Arg Gly Ile Ser Pro Glu Arg
           35                  40                  45
   His Ala Gln Leu Leu Ala Glu Leu Gly Leu Asp Lys Pro Met Trp Gln
       50                  55                  60
   Gln Tyr Leu His Tyr Ile Trp Gly Val Met His Gly Asp Leu Gly Ile
   65                  70                  75                  80
   Ser Met Lys Ser Arg Ile Pro Val Trp Glu Glu Phe Val Pro Arg Phe
                   85                  90                  95
   Gln Ala Thr Leu Glu Leu Gly Val Cys Ala Met Ile Phe Ala Thr Ala
               100                 105                 110
   Val Gly Ile Pro Val Gly Val Leu Ala Ala Val Lys Arg Gly Ser Ile
           115                 120                 125
   Phe Asp His Thr Ala Val Gly Leu Ala Leu Thr Gly Tyr Ser Met Pro
       130                 135                 140
   Ile Phe Trp Trp Gly Met Met Leu Ile Met Leu Val Ser Val His Trp
   145                 150                 155                 160
   Asn Leu Thr Pro Val Ser Gly Arg Val Ser Asp Met Val Phe Leu Asp
                   165                 170                 175
   Asp Ser Asn Pro Leu Thr Gly Phe Met Leu Ile Asp Thr Ala Ile Trp
               180                 185                 190
   Gly Glu Asp Gly Asn Phe Ile Asp Ala Val Ala His Met Ile Leu Pro
           195                 200                 205
   Ala Ile Val Leu Gly Thr Ile Pro Leu Ala Val Ile Val Arg Met Thr
       210                 215                 220
   Arg Ser Ser Met Leu Glu Val Leu Gly Glu Asp Tyr Ile Arg Thr Ala
   225                 230                 235                 240
   Arg Ala Lys Gly Leu Thr Arg Met Arg Val Ile Ile Val His Ala Leu
                   245                 250                 255
   Arg Asn Ala Met Leu Pro Val Val Thr Val Ile Gly Leu Gln Val Gly
               260                 265                 270
   Thr Leu Leu Ala Gly Ala Ile Leu Thr Glu Thr Ile Phe Ser Trp Pro
           275                 280                 285
   Gly Leu Gly Arg Trp Leu Ile Asp Ala Leu Gln Arg Arg Asp Tyr Pro
       290                 295                 300
   Val Val Gln Gly Gly Val Leu Leu Val Ala Thr Met Ile Ile Leu Val
   305                 310                 315                 320
   Asn Leu Leu Val Asp Leu Leu Tyr Gly Val Val Asn Pro Arg Ile Arg
                   325                 330                 335
   His Lys Lys
<210> 7
   <211> 300
   <212> PRT
   <213> Escherichia coli
<400> 7
   Met Ser Gln Val Thr Glu Asn Lys Val Ile Ser Ala Pro Val Pro Met
   1               5                   10                  15
   Thr Pro Leu Gln Glu Phe Trp His Tyr Phe Lys Arg Asn Lys Gly Ala
               20                  25                  30
   Val Val Gly Leu Val Tyr Val Val Ile Val Leu Phe Ile Ala Ile Phe
           35                  40                  45
   Ala Asn Trp Ile Ala Pro Tyr Asn Pro Ala Glu Gln Phe Arg Asp Ala
       50                  55                  60
   Leu Leu Ala Pro Pro Ala Trp Gln Glu Gly Gly Ser Met Ala His Leu
   65                  70                  75                  80
   Leu Gly Thr Asp Asp Val Gly Arg Asp Val Leu Ser Arg Leu Met Tyr
                   85                  90                  95
   Gly Ala Arg Leu Ser Leu Leu Val Gly Cys Leu Val Val Val Leu Ser
               100                 105                 110
   Leu Ile Met Gly Val Ile Leu Gly Leu Ile Ala Gly Tyr Phe Gly Gly
           115                 120                 125
   Leu Val Asp Asn Ile Ile Met Arg Val Val Asp Ile Met Leu Ala Leu
       130                 135                 140
   Pro Ser Leu Leu Leu Ala Leu Val Leu Val Ala Ile Phe Gly Pro Ser
   145                 150                 155                 160
   Ile Gly Asn Ala Ala Leu Ala Leu Thr Phe Val Ala Leu Pro His Tyr
                   165                 170                 175
   Val Arg Leu Thr Arg Ala Ala Val Leu Val Glu Val Asn Arg Asp Tyr
               180                 185                 190
   Val Thr Ala Ser Arg Val Ala Gly Ala Gly Ala Met Arg Gln Met Phe
           195                 200                 205
   Ile Asn Ile Phe Pro Asn Cys Leu Ala Pro Leu Ile Val Gln Ala Ser
       210                 215                 220
   Leu Gly Phe Ser Asn Ala Ile Leu Asp Met Ala Ala Leu Gly Phe Leu
   225                 230                 235                 240
   Gly Met Gly Ala Gln Pro Pro Thr Pro Glu Trp Gly Thr Met Leu Ser
                   245                 250                 255
   Asp Val Leu Gln Phe Ala Gln Ser Ala Trp Trp Val Val Thr Phe Pro
               260                 265                 270
   Gly Leu Ala Ile Leu Leu Thr Val Leu Ala Phe Asn Leu Met Gly Asp
           275                 280                 285
   Gly Leu Arg Asp Ala Leu Asp Pro Lys Leu Lys Gln
       290                 295                 300
<210> 8
   <211> 327
   <212> PRT
   <213> Escherichia coli
<400> 8
   Met Ala Leu Leu Asn Val Asp Lys Leu Ser Val His Phe Gly Asp Glu
   1               5                   10                  15
   Ser Ala Pro Phe Arg Ala Val Asp Arg Ile Ser Tyr Ser Val Lys Gln
               20                  25                  30
   Gly Glu Val Val Gly Ile Val Gly Glu Ser Gly Ser Gly Lys Ser Val
           35                  40                  45
   Ser Ser Leu Ala Ile Met Gly Leu Ile Asp Tyr Pro Gly Arg Val Met
       50                  55                  60
   Ala Glu Lys Leu Glu Phe Asn Gly Gln Asp Leu Gln Arg Ile Ser Glu
   65                  70                  75                  80
   Lys Glu Arg Arg Asn Leu Val Gly Ala Glu Val Ala Met Ile Phe Gln
                   85                  90                  95
   Asp Pro Met Thr Ser Leu Asn Pro Cys Tyr Thr Val Gly Phe Gln Ile
   100 105 110
   Met Glu Ala Ile Lys Val His Gln Gly Gly Asn Lys Ser Thr Arg Arg
   115 120 125
   Gln Arg Ala Ile Asp Leu Leu Asn Gln Val Gly Ile Pro Asp Pro Ala
   130 135 140
   Ser Arg Leu Asp Val Tyr Pro His Gln Leu Ser Gly Gly Met Ser Gln
   145 150 155 160
   Arg Val Met Ile Ala Met Ala Ile Ala Cys Arg Pro Lys Leu Leu Ile
   165 170 175
   Ala Asp Glu Pro Thr Thr Ala Leu Asp Val Thr Ile Gln Ala Gln Ile
   180 185 190
   Ile Glu Leu Leu Leu Glu Leu Gln Gln Lys Glu Asn Met Ala Leu Val
   195 200 205
   Leu Ile Thr His Asp Leu Ala Leu Val Ala Glu Ala Ala His Lys Ile
   210 215 220
   Ile Val Met Tyr Ala Gly Gln Val Val Glu Thr Gly Asp Ala His Ala
   225 230 235 240
   Ile Phe His Ala Pro Arg His Pro Tyr Thr Gln Ala Leu Leu Arg Ala
   245 250 255
   Leu Pro Glu Phe Ala Gln Asp Lys Glu Arg Leu Ala Ser Leu Pro Gly
   260 265 270
   Val Val Pro Gly Lys Tyr Asp Arg Pro Asn Gly Cys Leu Leu Asn Pro
   275 280 285
   Arg Cys Pro Tyr Ala Thr Asp Arg Cys Arg Ala Glu Glu Pro Ala Leu
   290 295 300
   Asn Met Leu Ala Asp Gly Arg Gln Ser Lys Cys His Tyr Pro Leu Asp
   305 310 315 320
   Asp Ala Gly Arg Pro Thr Leu
   325
<210> 9
   <211> 334
   <212> PRT
   <213> Escherichia coli
<400> 9
   Met Ser Thr Gln Glu Ala Thr Leu Gln Gln Pro Leu Leu Gln Ala Ile
    1               5                  10                  15
   Asp Leu Lys Lys His Tyr Pro Val Lys Lys Gly Met Phe Ala Pro Glu
               20                  25                  30
   Arg Leu Val Lys Ala Leu Asp Gly Val Ser Phe Asn Leu Glu Arg Gly
           35                  40                  45
   Lys Thr Leu Ala Val Val Gly Glu Ser Gly Cys Gly Lys Ser Thr Leu
       50                  55                  60
   Gly Arg Leu Leu Thr Met Ile Glu Met Pro Thr Gly Gly Glu Leu Tyr
   65                  70                  75                  80
   Tyr Gln Gly Gln Asp Leu Leu Lys His Asp Pro Gln Ala Gln Lys Leu
                   85                  90                  95
   Arg Arg Gln Lys Ile Gln Ile Val Phe Gln Asn Pro Tyr Gly Ser Leu
               100                 105                 110
   Asn Pro Arg Lys Lys Val Gly Gln Ile Leu Glu Glu Pro Leu Leu Ile
           115                 120                 125
   Asn Thr Ser Leu Ser Lys Glu Gln Arg Arg Glu Lys Ala Leu Ser Met
       130                 135                 140
   Met Ala Lys Val Gly Leu Lys Thr Glu His Tyr Asp Arg Tyr Pro His
   145                 150                 155                 160
   Met Phe Ser Gly Gly Gln Arg Gln Arg Ile Ala Ile Ala Arg Gly Leu
                   165                 170                 175
   Met Leu Asp Pro Asp Val Val Ile Ala Asp Glu Pro Val Ser Ala Leu
               180                 185                 190
   Asp Val Ser Val Arg Ala Gln Val Leu Asn Leu Met Met Asp Leu Gln
           195                 200                 205
   Gln Glu Leu Gly Leu Ser Tyr Val Phe Ile Ser His Asp Leu Ser Val
       210                 215                 220
   Val Glu His Ile Ala Asp Glu Val Met Val Met Tyr Leu Gly Arg Cys
   225                 230                 235                 240
   Val Glu Lys Gly Thr Lys Asp Gln Ile Phe Asn Asn Pro Arg His Pro
                   245                 250                 255
   Tyr Thr Gln Ala Leu Leu Ser Ala Thr Pro Arg Leu Asn Pro Asp Asp
               260                 265                 270
   Arg Arg Glu Arg Ile Lys Leu Ser Gly Glu Leu Pro Ser Pro Leu Asn
           275                 280                 285
   Pro Pro Pro Gly Cys Ala Phe Asn Ala Arg Cys Arg Arg Arg Phe Gly
       290                 295                 300
   Pro Cys Thr Gln Leu Gln Pro Gln Leu Lys Asp Tyr Gly Gly Gln Leu
   305                 310                 315                 320
   Val Ala Cys Phe Ala Val Asp Gln Asp Glu Asn Pro Gln Arg
                   325                 330
<210> 10
   <211> 1509
   <212> DNA
   <213> Escherichia coli
<400> 10
   atg gag ttt agt gta aaa agc ggt agc ccg gag aaa cag cgg agt gcc    48
   Met Glu Phe Ser Val Lys Ser Gly Ser Pro Glu Lys Gln Arg Ser Ala
    1               5                  10                  15
   tgc atc gtc gtg ggc gtc ttc gaa cca cgt cgc ctt tct ccg att gca    96
   Cys Ile Val Val Gly Val Phe Glu Pro Arg Arg Leu Ser Pro Ile Ala
               20                  25                  30
   gaa cag ctc gat aaa atc agc gat ggg tac atc agc gcc ctg cta cgt    144
   Glu Gln Leu Asp Lys Ile Ser Asp Gly Tyr Ile Ser Ala Leu Leu Arg
           35                  40                  45
   cgg ggc gaa ctg gaa gga aaa ccg ggg cag aca ttg ttg ctg cac cat    192
   Arg Gly Glu Leu Glu Gly Lys Pro Gly Gln Thr Leu Leu Leu His His
       50                  55                  60
   gtt ccg aat gta ctt tcc gag cga att ctc ctt att ggt tgc ggc aaa     240
   Val Pro Asn Val Leu Ser Glu Arg Ile Leu Leu Ile Gly Cys Gly Lys
   65                  70                  75                  80
   gaa cgt gag ctg gat gag cgt cag tac aag cag gtt att cag aaa acc     288
   Glu Arg Glu Leu Asp Glu Arg Gln Tyr Lys Gln Val Ile Gln Lys Thr
                   85                  90                  95
   att aat acg ctg aat gat act ggc tca atg gaa gcg gtc tgc ttt ctg     336
   Ile Asn Thr Leu Asn Asp Thr Gly Ser Met Glu Ala Val Cys Phe Leu
               100                 105                 110
   act gag ctg cac gtt aaa ggc cgt aac aac tac tgg aaa gtg cgt cag     384
   Thr Glu Leu His Val Lys Gly Arg Asn Asn Tyr Trp Lys Val Arg Gln
           115                 120                 125
   gct gtc gag acg gca aaa gag acg ctc tac agt ttc gat cag ctg aaa     432
   Ala Val Glu Thr Ala Lys Glu Thr Leu Tyr Ser Phe Asp Gln Leu Lys
       130                 135                 140
   acg aac aag agc gaa ccg cgt cgt ccg ctg cgt aag atg gtg ttc aac     480
   Thr Asn Lys Ser Glu Pro Arg Arg Pro Leu Arg Lys Met Val Phe Asn
   145                 150                 155                 160
   gtg ccg acc cgc cgt gaa ctg acc agc ggt gag cgc gcg atc cag cac     528
   Val Pro Thr Arg Arg Glu Leu Thr Ser Gly Glu Arg Ala Ile Gln His
                   165                 170                 175
   ggt ctg gcg att gcc gcc ggg att aaa gca gca aaa gat ctc ggc aat     576
   Gly Leu Ala Ile Ala Ala Gly Ile Lys Ala Ala Lys Asp Leu Gly Asn
               180                 185                 190
   atg ccg ccg aat atc tgt aac gcc gct tac ctc gct tca caa gcg cgc     624
   Met Pro Pro Asn Ile Cys Asn Ala Ala Tyr Leu Ala Ser Gln Ala Arg
           195                 200                 205
   cag ctg gct gac agc tac agc aag aat gtc atc acc cgc gtt atc ggc     672
   Gln Leu Ala Asp Ser Tyr Ser Lys Asn Val Ile Thr Arg Val Ile Gly
       210                 215                 220
   gaa cag cag atg aaa gag ctg ggg atg cat tcc tat ctg gcg gtc ggt     720
   Glu Gln Gln Met Lys Glu Leu Gly Met His Ser Tyr Leu Ala Val Gly
   225                 230                 235                 240
   cag ggt tcg caa aac gaa tcg ctg atg tcg gtg att gag tac aaa ggc     768
   Gln Gly Ser Gln Asn Glu Ser Leu Met Ser Val Ile Glu Tyr Lys Gly
                   245                 250                 255
   aac gcg tcg gaa gat gca cgc cca atc gtg ctg gtg ggt aaa ggt tta     816
   Asn Ala Ser Glu Asp Ala Arg Pro Ile Val Leu Val Gly Lys Gly Leu
               260                 265                 270
   acc ttc gac tcc ggc ggt atc tcg atc aag cct tca gaa ggc atg gat     864
   Thr Phe Asp Ser Gly Gly Ile Ser Ile Lys Pro Ser Glu Gly Met Asp
           275                 280                 285
   gag atg aag tac gat atg tgc ggt gcg gca gcg gtt tac ggc gtg atg     912
   Glu Met Lys Tyr Asp Met Cys Gly Ala Ala Ala Val Tyr Gly Val Met
       290                 295                 300
   cgg atg gtc gcg gag cta caa ctg ccg att aac gtt atc ggc gtg ttg     960
   Arg Met Val Ala Glu Leu Gln Leu Pro Ile Asn Val Ile Gly Val Leu
   305                 310                 315                 320
   gca ggc tgc gaa aac atg cct ggc gga cga gcc tat cgt ccg ggc gat    1008
   Ala Gly Cys Glu Asn Met Pro Gly Gly Arg Ala Tyr Arg Pro Gly Asp
                   325                 330                 335
   gtg tta acc acc atg tcc ggt caa acc gtt gaa gtg ctg aac acc gac      1056
   Val Leu Thr Thr Met Ser Gly Gln Thr Val Glu Val Leu Asn Thr Asp
               340                 345                 350
   gct gaa ggc cgc ctg gta ctg tgc gac gtg tta act tac gtt gag cgt      1104
   Ala Glu Gly Arg Leu Val Leu Cys Asp Val Leu Thr Tyr Val Glu Arg
           355                 360                 365
   ttt gag ccg gaa gcg gtg att gac gtg gcg acg ctg acc ggt gcc tgc      1152
   Phe Glu Pro Glu Ala Val Ile Asp Val Ala Thr Leu Thr Gly Ala Cys
       370                 375                 380
   gtg atc gcg ctg ggt cat cat att act ggt ctg atg gcg aac cat aat      1200
   Val Ile Ala Leu Gly His His Ile Thr Gly Leu Met Ala Asn His Asn
   385                 390                 395                 400
   ccg ctg gcc cat gaa ctg att gcc gcg tct gaa caa tcc ggt gac cgc      1248
   Pro Leu Ala His Glu Leu Ile Ala Ala Ser Glu Gln Ser Gly Asp Arg
                   405                 410                 415
   gca tgg cgc tta ccg ctg ggt gac gag tat cag gaa caa ctg gag tcc      1296
   Ala Trp Arg Leu Pro Leu Gly Asp Glu Tyr Gln Glu Gln Leu Glu Ser
               420                 425                 430
   aat ttt gcc gat atg gcg aac att ggc ggt cgt cct ggt ggg gcg att      1344
   Asn Phe Ala Asp Met Ala Asn Ile Gly Gly Arg Pro Gly Gly Ala Ile
           435                 440                 445
   acc gca ggt tgc ttc ctg tca cgc ttt acc cgt aag tac aac tgg gcg      1392
   Thr Ala Gly Cys Phe Leu Ser Arg Phe Thr Arg Lys Tyr Asn Trp Ala
       450                 455                 460
   cac ctg gat atc gcc ggt acc gcc tgg cgt tct ggt aaa gca aaa ggc      1440
   His Leu Asp Ile Ala Gly Thr Ala Trp Arg Ser Gly Lys Ala Lys Gly
   465                 470                 475                 480
   gcc acc ggt cgt ccg gta gcg ttg ctg gca cag ttc ctg tta aac cgc      1488
   Ala Thr Gly Arg Pro Val Ala Leu Leu Ala Gln Phe Leu Leu Asn Arg
                   485                 490                 495
   gct ggg ttt aac ggc gaa gag                                          1509
   Ala Gly Phe Asn Gly Glu Glu
               500
<210> 11
   <211> 1281
   <212> DNA
   <213> Escherichia coli
<400> 11
   atg aca gaa gcg atg aag att acc ctc tct acc caa cct gcc gac gcg      48
   Met Thr Glu Ala Met Lys Ile Thr Leu Ser Thr Gln Pro Ala Asp Ala
    1               5                  10                  15
   cgc tgg gga gaa aaa gca act tac agc att aat aat gac ggc att acc      96
   Arg Trp Gly Glu Lys Ala Thr Tyr Ser Ile Asn Asn Asp Gly Ile Thr
               20                  25                  30
   ctg cat ttg aac ggg gca gac gat ctg ggg ctg atc cag cgt gcg gcg     144
   Leu His Leu Asn Gly Ala Asp Asp Leu Gly Leu Ile Gln Arg Ala Ala
           35                  40                  45
   cgc aag att gac ggt ctg ggc atc aag cat gtt cag tta agc ggt gaa     192
   Arg Lys Ile Asp Gly Leu Gly Ile Lys His Val Gln Leu Ser Gly Glu
   50 55 60
   ggc tgg gat gcg gat cgc tgc tgg gca ttc tgg caa ggt tac aaa gcc 240
   Gly Trp Asp Ala Asp Arg Cys Trp Ala Phe Trp Gln Gly Tyr Lys Ala
   65 70 75 80
   ccg aaa ggc acg cgt aaa gtg gtg tgg ccg gat ctg gac gat gcc cag 288
   Pro Lys Gly Thr Arg Lys Val Val Trp Pro Asp Leu Asp Asp Ala Gln
   85 90 95
   cgc cag gaa ctg gat aac cgc ctg atg atc atc gac tgg gtg cgt gac 336
   Arg Gln Glu Leu Asp Asn Arg Leu Met Ile Ile Asp Trp Val Arg Asp
   100 105 110
   acc atc aac gca ccg gca gaa gaa ttg gga cca tcg caa ctg gca cag 384
   Thr Ile Asn Ala Pro Ala Glu Glu Leu Gly Pro Ser Gln Leu Ala Gln
   115 120 125
   cgt gct gtt gat ctg atc agc aac gtc gcg ggc gat cgt gtg act tat 432
   Arg Ala Val Asp Leu Ile Ser Asn Val Ala Gly Asp Arg Val Thr Tyr
   130 135 140
   cgg atc acc aaa ggc gaa gat ctg cgt gag caa ggt tat atg ggg ctg 480
   Arg Ile Thr Lys Gly Glu Asp Leu Arg Glu Gln Gly Tyr Met Gly Leu
   145 150 155 160
   cac aca gtc gga cgc ggt tca gaa cgt tct ccg gta ttg ctg gcg ctg 528
   His Thr Val Gly Arg Gly Ser Glu Arg Ser Pro Val Leu Leu Ala Leu
   165 170 175
   gat tac aac cca act ggc gat aaa gaa gcg cca gtg tac gcg tgc ctg 576
   Asp Tyr Asn Pro Thr Gly Asp Lys Glu Ala Pro Val Tyr Ala Cys Leu
   180 185 190
   gta ggt aaa ggt atc act ttt gac tcc ggc ggc tac agc atc aaa cag 624
   Val Gly Lys Gly Ile Thr Phe Asp Ser Gly Gly Tyr Ser Ile Lys Gln
   195 200 205
   act gcg ttt atg gac tcg atg aag tcg gac atg ggc ggc gcg gca acg 672
   Thr Ala Phe Met Asp Ser Met Lys Ser Asp Met Gly Gly Ala Ala Thr
   210 215 220
   gtt acc ggg gcg ctg gca ttt gcc att acg cgc gga ctg aac aag cgc 720
   Val Thr Gly Ala Leu Ala Phe Ala Ile Thr Arg Gly Leu Asn Lys Arg
   225 230 235 240
   gtg aag ctg ttc ctc tgc tgt gcg gat aac ctg att agc ggc aat gcg 768
   Val Lys Leu Phe Leu Cys Cys Ala Asp Asn Leu Ile Ser Gly Asn Ala
   245 250 255
   ttc aag ctg ggc gat atc atc acc tat cgc aac ggt aaa aaa gtt gaa 816
   Phe Lys Leu Gly Asp Ile Ile Thr Tyr Arg Asn Gly Lys Lys Val Glu
   260 265 270
   gtg atg aac act gat gcc gaa ggg cgt ctg gtg ctt gcc gat ggt ctg 864
   Val Met Asn Thr Asp Ala Glu Gly Arg Leu Val Leu Ala Asp Gly Leu
   275 280 285
   att gat gcc agt gcg cag aaa ccg gaa atg atc att gat gcg gcg acc 912
   Ile Asp Ala Ser Ala Gln Lys Pro Glu Met Ile Ile Asp Ala Ala Thr
   290 295 300
   ctc acc ggg gcg gcg aaa act gcg ctg ggt aat gat tat cac gcg ctg 960
   Leu Thr Gly Ala Ala Lys Thr Ala Leu Gly Asn Asp Tyr His Ala Leu
   305 310 315 320
   ttc agt ttt gac gat gcg ctg gcc ggt cgc ttg ctg gcg agt gcc gcg 1008
   Phe Ser Phe Asp Asp Ala Leu Ala Gly Arg Leu Leu Ala Ser Ala Ala
                   325                 330                 335
   cag gag aac gaa ccg ttc tgg cgt ctg ccg ctg gcg gag ttc cac cgc      1056
   Gln Glu Asn Glu Pro Phe Trp Arg Leu Pro Leu Ala Glu Phe His Arg
               340                 345                 350
   agc cag ctg ccg tct aac ttt gcc gaa ctg aac aat acc gga agc gcg      1104
   Ser Gln Leu Pro Ser Asn Phe Ala Glu Leu Asn Asn Thr Gly Ser Ala
           355                 360                 365
   gcg tat ccg gca ggc gcg agc acg gcg gcg ggc ttc ctg tcg cac ttt      1152
   Ala Tyr Pro Ala Gly Ala Ser Thr Ala Ala Gly Phe Leu Ser His Phe
       370                 375                 380
   gtt gag aac tat cag caa ggc tgg ctg cat atc gac tgc tcg gcg act      1200
   Val Glu Asn Tyr Gln Gln Gly Trp Leu His Ile Asp Cys Ser Ala Thr
   385                 390                 395                 400
   tac cgt aaa gcg ccg gtt gaa cag tgg tct gcg ggc gct acg gga ctt      1248
   Tyr Arg Lys Ala Pro Val Glu Gln Trp Ser Ala Gly Ala Thr Gly Leu
                   405                 410                 415
   ggt gtg cgc acg ata gct aat ctg tta acg gcg                          1281
   Gly Val Arg Thr Ile Ala Asn Leu Leu Thr Ala
               420                 425
<210> 12
   <211> 1455
   <212> DNA
   <213> Escherichia coli
<400> 12
   gtg tct gaa ctg tct caa tta tct cca cag ccg ctg tgg gat att ttt      48
   Val Ser Glu Leu Ser Gln Leu Ser Pro Gln Pro Leu Trp Asp Ile Phe
   1               5                   10                  15
   gcc aaa atc tgt tct att cct cac ccg tcc tat cat gaa gag caa ctc      96
   Ala Lys Ile Cys Ser Ile Pro His Pro Ser Tyr His Glu Glu Gln Leu
               20                  25                  30
   gct gaa tac att gtt ggt tgg gca aaa gag aaa ggt ttc cat gtc gaa     144
   Ala Glu Tyr Ile Val Gly Trp Ala Lys Glu Lys Gly Phe His Val Glu
           35                  40                  45
   cgc gat cag gta ggt aat atc ctg att cgt aaa cct gct acc gca ggt     192
   Arg Asp Gln Val Gly Asn Ile Leu Ile Arg Lys Pro Ala Thr Ala Gly
       50                  55                  60
   atg gaa aat cgt aaa ccg gtc gtc tta cag gcc cac ctc gat atg gtg     240
   Met Glu Asn Arg Lys Pro Val Val Leu Gln Ala His Leu Asp Met Val
   65                  70                  75                  80
   ccg cag aaa aat aac gac acc gtg cat gac ttc acg aaa gat cct atc     288
   Pro Gln Lys Asn Asn Asp Thr Val His Asp Phe Thr Lys Asp Pro Ile
                   85                  90                  95
   cag cct tat att gat ggc gaa tgg gtt aaa gcg cgc ggc acc acg ctg     336
   Gln Pro Tyr Ile Asp Gly Glu Trp Val Lys Ala Arg Gly Thr Thr Leu
               100                 105                 110
   ggt gcg gat aac ggc att ggt atg gcc tct gcg ctg gcg gtt ctg gct     384
   Gly Ala Asp Asn Gly Ile Gly Met Ala Ser Ala Leu Ala Val Leu Ala
           115                 120                 125
   gac gaa aac gtg gtt cac ggc ccg ctg gaa gtg ctg ctg acc atg acc     432
   Asp Glu Asn Val Val His Gly Pro Leu Glu Val Leu Leu Thr Met Thr
       130                 135                 140
   gaa gaa gcc ggt atg gac ggt gcg ttc ggc tta cag ggc aac tgg ttg     480
   Glu Glu Ala Gly Met Asp Gly Ala Phe Gly Leu Gln Gly Asn Trp Leu
   145                 150                 155                 160
   cag gct gat att ctg att aac acc gac tcc gaa gaa gaa ggt gaa atc     528
   Gln Ala Asp Ile Leu Ile Asn Thr Asp Ser Glu Glu Glu Gly Glu Ile
                   165                 170                 175
   tac atg ggt tgt gcg ggg ggt atc gac ttc acc tcc aac ctg cat tta     576
   Tyr Met Gly Cys Ala Gly Gly Ile Asp Phe Thr Ser Asn Leu His Leu
               180                 185                 190
   gat cgt gaa gcg gtt cca gct ggt ttt gaa acc ttc aag tta acc tta     624
   Asp Arg Glu Ala Val Pro Ala Gly Phe Glu Thr Phe Lys Leu Thr Leu
           195                 200                 205
   aaa ggt ctg aaa ggc ggt cac tcc ggc ggg gaa atc cac gtt ggg ctg     672
   Lys Gly Leu Lys Gly Gly His Ser Gly Gly Glu Ile His Val Gly Leu
       210                 215                 220
   ggt aat gcc aac aaa ctg ctg gtg cgc ttc ctg gcg ggt cat gcg gaa     720
   Gly Asn Ala Asn Lys Leu Leu Val Arg Phe Leu Ala Gly His Ala Glu
   225                 230                 235                 240
   gaa ctg gat ctg cgc ctt atc gat ttc aac ggc ggc aca ctg cgt aac     768
   Glu Leu Asp Leu Arg Leu Ile Asp Phe Asn Gly Gly Thr Leu Arg Asn
                   245                 250                 255
   gcc atc ccg cgt gaa gcc ttt gcg acc att gct gtc gca gct gat aaa     816
   Ala Ile Pro Arg Glu Ala Phe Ala Thr Ile Ala Val Ala Ala Asp Lys
               260                 265                 270
   gtc gac gtc ctg aaa tct ctg gtg aat acc tat cag gag atc ctg aaa     864
   Val Asp Val Leu Lys Ser Leu Val Asn Thr Tyr Gln Glu Ile Leu Lys
           275                 280                 285
   aac gag ctg gca gaa aaa gag aaa aat ctg gcc ttg ttg ctg gac tct     912
   Asn Glu Leu Ala Glu Lys Glu Lys Asn Leu Ala Leu Leu Leu Asp Ser
       290                 295                 300
   gta gcg aac gat aaa gct gcc ctg att gcg aaa tct cgc gat acc ttt     960
   Val Ala Asn Asp Lys Ala Ala Leu Ile Ala Lys Ser Arg Asp Thr Phe
   305                 310                 315                 320
   att cgt ctg ctg aac gcc acc ccg aac ggt gtg att cgt aac tcc gat     1008
   Ile Arg Leu Leu Asn Ala Thr Pro Asn Gly Val Ile Arg Asn Ser Asp
                   325                 330                 335
   gta gcc aaa ggt gtg gtt gaa acc tcc ctg aac gtc ggt gtg gtg acc     1056
   Val Ala Lys Gly Val Val Glu Thr Ser Leu Asn Val Gly Val Val Thr
               340                 345                 350
   atg act gac aat aac gta gaa att cac tgc ctg atc cgt tca ctg atc     1104
   Met Thr Asp Asn Asn Val Glu Ile His Cys Leu Ile Arg Ser Leu Ile
           355                 360                 365
   gac agc ggt aaa gac tac gtg gtg agc atg ctg gat tcg ctg ggt aaa     1152
   Asp Ser Gly Lys Asp Tyr Val Val Ser Met Leu Asp Ser Leu Gly Lys
       370                 375                 380
   ctg gct ggc gcg aaa acc gaa gcg aaa ggc gca tat cct ggc tgg cag     1200
   Leu Ala Gly Ala Lys Thr Glu Ala Lys Gly Ala Tyr Pro Gly Trp Gln
   385                 390                 395                 400
   ccg gac gct aat tct ccg gtg atg cat ctg gta cgt gaa acc tat cag     1248
   Pro Asp Ala Asn Ser Pro Val Met His Leu Val Arg Glu Thr Tyr Gln
                   405                 410                 415
   cgc ctg ttc aac aag acg ccg aac atc cag att atc cac gcg ggc ctg     1296
   Arg Leu Phe Asn Lys Thr Pro Asn Ile Gln Ile Ile His Ala Gly Leu
               420                 425                 430
   gaa tgt ggt ctg ttc aaa aaa ccg tat ccg gaa atg gac atg gtt tct     1344
   Glu Cys Gly Leu Phe Lys Lys Pro Tyr Pro Glu Met Asp Met Val Ser
           435                 440                 445
   atc ggg cca act atc acc ggt cca cac tct ccg gat gag caa gtt cac     1392
   Ile Gly Pro Thr Ile Thr Gly Pro His Ser Pro Asp Glu Gln Val His
       450                 455                 460
   atc gaa agc gta ggt cat tac tgg aca ctg ctg act gaa ctg ctg aaa     1440
   Ile Glu Ser Val Gly His Tyr Trp Thr Leu Leu Thr Glu Leu Leu Lys
   465                 470                 475                 480
   gaa att ccg gcg aag                                                 1455
   Glu Ile Pro Ala Lys
                   485
<210> 13
   <211> 2610
   <212> DNA
   <213> Escherichia coli
<400> 13
   atg act caa cag cca caa gcc aaa tac cgt cac gat tat cgt gcg ccg 48
   Met Thr Gln Gln Pro Gln Ala Lys Tyr Arg His Asp Tyr Arg Ala Pro
   1 5 10 15
   gat tac cag att act gat att gac ttg acc ttt gac ctc gac gcg caa 96
   Asp Tyr Gln Ile Thr Asp Ile Asp Leu Thr Phe Asp Leu Asp Ala Gln
   20 25 30
   aag acg gtc gtt acc gcg gtc agc cag gct gtc cgt cat ggt gca tca 144
   Lys Thr Val Val Thr Ala Val Ser Gln Ala Val Arg His Gly Ala Ser
   35 40 45
   gat gct ccc ctt cgt ctc aac ggc gaa gac ctc aaa ctg gtt tct gtt 192
   Asp Ala Pro Leu Arg Leu Asn Gly Glu Asp Leu Lys Leu Val Ser Val
   50 55 60
   cat att aat gat gag ccg tgg acc gcc tgg aaa gaa gaa gag ggc gca 240
   His Ile Asn Asp Glu Pro Trp Thr Ala Trp Lys Glu Glu Glu Gly Ala
   65 70 75 80
   ctg gtt atc agt aat ttg ccg gag cgt ttt acg ctt aag atc att aat 288
   Leu Val Ile Ser Asn Leu Pro Glu Arg Phe Thr Leu Lys Ile Ile Asn
   85 90 95
   gaa ata agc ccg gcg gcg aat acc gcg ctg gaa ggg ctt tat cag tca 336
   Glu Ile Ser Pro Ala Ala Asn Thr Ala Leu Glu Gly Leu Tyr Gln Ser
   100 105 110
   ggc gat gcg ctt tgc acc cag tgt gaa gcc gaa ggt ttc cgc cat att 384
   Gly Asp Ala Leu Cys Thr Gln Cys Glu Ala Glu Gly Phe Arg His Ile
   115 120 125
   acg tat tat ctc gac cgc ccg gac gtg ctg gcg cgt ttt acc acc aaa 432
   Thr Tyr Tyr Leu Asp Arg Pro Asp Val Leu Ala Arg Phe Thr Thr Lys
   130 135 140
   att att gcc gat aaa atc aaa tat ccc ttc ctg ctt tcc aat ggt aac 480
   Ile Ile Ala Asp Lys Ile Lys Tyr Pro Phe Leu Leu Ser Asn Gly Asn
   145 150 155 160
   cgc gtt gcg caa ggc gaa ctg gaa aac gga cgc cat tgg gta cag tgg     528
   Arg Val Ala Gln Gly Glu Leu Glu Asn Gly Arg His Trp Val Gln Trp
                   165                 170                 175
   cag gac ccg ttc ccg aaa ccg tgc tac ctg ttt gcg ctg gtg gca ggc     576
   Gln Asp Pro Phe Pro Lys Pro Cys Tyr Leu Phe Ala Leu Val Ala Gly
               180                 185                 190
   gac ttt gat gta ctg cgc gat acc ttt acc acg cgt tct ggt cgc gaa     624
   Asp Phe Asp Val Leu Arg Asp Thr Phe Thr Thr Arg Ser Gly Arg Glu
           195                 200                 205
   gta gca ctg gag ctg tac gtc gat cgc ggc aac ctt gat cgc gcg ccg     672
   Val Ala Leu Glu Leu Tyr Val Asp Arg Gly Asn Leu Asp Arg Ala Pro
       210                 215                 220
   tgg gcg atg acc tcg ctg aaa aac tcc atg aaa tgg gat gaa gaa cgc     720
   Trp Ala Met Thr Ser Leu Lys Asn Ser Met Lys Trp Asp Glu Glu Arg
   225                 230                 235                 240
   ttt ggc ctg gag tat gac ctc gac atc tat atg atc gtc gcg gtg gat     768
   Phe Gly Leu Glu Tyr Asp Leu Asp Ile Tyr Met Ile Val Ala Val Asp
                   245                 250                 255
   ttc ttc aat atg ggc gca atg gag aat aag ggg ctg aat atc ttt aac     816
   Phe Phe Asn Met Gly Ala Met Glu Asn Lys Gly Leu Asn Ile Phe Asn
               260                 265                 270
   tcc aaa tat gtg ctg gcc cgc acc gac acc gcc acc gac aaa gat tac     864
   Ser Lys Tyr Val Leu Ala Arg Thr Asp Thr Ala Thr Asp Lys Asp Tyr
           275                 280                 285
   ctc gat att gaa cgc gtt atc ggc cat gaa tat ttc cat aac tgg acc     912
   Leu Asp Ile Glu Arg Val Ile Gly His Glu Tyr Phe His Asn Trp Thr
       290                 295                 300
   ggt aac cga gtg acc tgt cgc gac tgg ttc cag ctc agc ctg aaa gaa     960
   Gly Asn Arg Val Thr Cys Arg Asp Trp Phe Gln Leu Ser Leu Lys Glu
   305                 310                 315                 320
   ggt tta acc gtc ttc cgc gat cag gag ttc agc tct gac ctt ggt tcc     1008
   Gly Leu Thr Val Phe Arg Asp Gln Glu Phe Ser Ser Asp Leu Gly Ser
                   325                 330                 335
   cgc gca gtt aac cgc atc aat aat gta cgc acc atg cgc gga ttg cag     1056
   Arg Ala Val Asn Arg Ile Asn Asn Val Arg Thr Met Arg Gly Leu Gln
               340                 345                 350
   ttt gca gaa gac gcc agc ccg atg gcg cac ccg atc cgc ccg gat atg     1104
   Phe Ala Glu Asp Ala Ser Pro Met Ala His Pro Ile Arg Pro Asp Met
           355                 360                 365
   gtc att gag atg aac aac ttc tac acc ctg acc gtt tac gag aag ggc     1152
   Val Ile Glu Met Asn Asn Phe Tyr Thr Leu Thr Val Tyr Glu Lys Gly
       370                 375                 380
   gcg gaa gtg att cgc atg atc cac acc ctg ctt ggc gaa gaa aac ttc     1200
   Ala Glu Val Ile Arg Met Ile His Thr Leu Leu Gly Glu Glu Asn Phe
   385                 390                 395                 400
   cag aaa ggg atg cag ctt tat ttc gag cgt cat gat ggt agt gca gcg     1248
   Gln Lys Gly Met Gln Leu Tyr Phe Glu Arg His Asp Gly Ser Ala Ala
                   405                 410                 415
   acc tgt gac gac ttt gtg cag gcg atg gaa gat gcg tcg aat gtc gat     1296
   Thr Cys Asp Asp Phe Val Gln Ala Met Glu Asp Ala Ser Asn Val Asp
               420                 425                 430
   ctc tcc cat ttc cgc cgt tgg tac agc cag tcc ggt aca ccg att gtg 1344
   Leu Ser His Phe Arg Arg Trp Tyr Ser Gln Ser Gly Thr Pro Ile Val
   435 440 445
   acc gtc aaa gac gac tac aat ccg gaa acc gag cag tac acc ctg acc 1392
   Thr Val Lys Asp Asp Tyr Asn Pro Glu Thr Glu Gln Tyr Thr Leu Thr
   450 455 460
   atc agc cag cgc acg cca gcc acg ccg gat cag gca gaa aaa cag ccg 1440
   Ile Ser Gln Arg Thr Pro Ala Thr Pro Asp Gln Ala Glu Lys Gln Pro
   465 470 475 480
   ctg cat att ccg ttt gcc atc gaa ctg tat gat aac gaa ggc aaa gtg 1488
   Leu His Ile Pro Phe Ala Ile Glu Leu Tyr Asp Asn Glu Gly Lys Val
   485 490 495
   atc ccg ttg cag aaa ggc ggt cat ccg gtg aat tcc gtg ctg aac gtc 1536
   Ile Pro Leu Gln Lys Gly Gly His Pro Val Asn Ser Val Leu Asn Val
   500 505 510
   act cag gcg gaa cag acc ttt gtc ttt gat aat gtc tac ttc cag ccg 1584
   Thr Gln Ala Glu Gln Thr Phe Val Phe Asp Asn Val Tyr Phe Gln Pro
   515 520 525
   gtg cct gcg ctg ctg tgc gaa ttc tct gcg cca gtg aaa ctg gaa tat 1632
   Val Pro Ala Leu Leu Cys Glu Phe Ser Ala Pro Val Lys Leu Glu Tyr
   530 535 540
   aag tgg agc gat cag caa ctg acc ttc ctg atg cgt cat gcg cgt aat 1680
   Lys Trp Ser Asp Gln Gln Leu Thr Phe Leu Met Arg His Ala Arg Asn
   545 550 555 560
   gat ttc tcc cgc tgg gat gcg gcg caa agt ttg ctg gca acc tac atc 1728
   Asp Phe Ser Arg Trp Asp Ala Ala Gln Ser Leu Leu Ala Thr Tyr Ile
   565 570 575
   aag ctg aac gtc gcg cgt cat cag caa ggt cag ccg ctg tct ctg ccg 1776
   Lys Leu Asn Val Ala Arg His Gln Gln Gly Gln Pro Leu Ser Leu Pro
   580 585 590
   gtg cat gtg gct gat gct ttc cgc gcg gta ctg ctt gat gag aag att 1824
   Val His Val Ala Asp Ala Phe Arg Ala Val Leu Leu Asp Glu Lys Ile
   595 600 605
   gat cca gcg ctg gcg gca gaa atc ctg acg ctg cct tct gtc aat gaa 1872
   Asp Pro Ala Leu Ala Ala Glu Ile Leu Thr Leu Pro Ser Val Asn Glu
   610 615 620
   atg gct gaa ttg ttc gat atc atc gac ccg att gct att gcc gaa gta 1920
   Met Ala Glu Leu Phe Asp Ile Ile Asp Pro Ile Ala Ile Ala Glu Val
   625 630 635 640
   cgc gaa gca ctc act cgt act ctg gcg act gaa ctg gcg gat gag cta 1968
   Arg Glu Ala Leu Thr Arg Thr Leu Ala Thr Glu Leu Ala Asp Glu Leu
   645 650 655
   ctg gct att tac aac gcg aat tac cag agc gag tac cgt gtt gag cat 2016
   Leu Ala Ile Tyr Asn Ala Asn Tyr Gln Ser Glu Tyr Arg Val Glu His
   660 665 670
   gaa gat att gca aaa cgc act ctg cgt aat gcc tgc ctg cgt ttc ctc 2064
   Glu Asp Ile Ala Lys Arg Thr Leu Arg Asn Ala Cys Leu Arg Phe Leu
   675 680 685
   gct ttt ggt gaa acg cat ctg gct gat gtg ctg gtg agc aag cag ttc 2112
   Ala Phe Gly Glu Thr His Leu Ala Asp Val Leu Val Ser Lys Gln Phe
   690 695 700
   cac gaa gca aac aat atg act gat gcg ctg gcg gcg ctt tct gcg gcg     2160
   His Glu Ala Asn Asn Met Thr Asp Ala Leu Ala Ala Leu Ser Ala Ala
   705                 710                 715                 720
   gtt gcc gca cag ctg cct tgc cgt gac gcg ctg atg cag gag tac gac     2208
   Val Ala Ala Gln Leu Pro Cys Arg Asp Ala Leu Met Gln Glu Tyr Asp
                   725                 730                 735
   gac aag tgg cat cag aac ggt ctg gtg atg gat aaa tgg ttt atc ctg     2256
   Asp Lys Trp His Gln Asn Gly Leu Val Met Asp Lys Trp Phe Ile Leu
               740                 745                 750
   caa gcc acc agc ccg gcg gcg aat gtg ctg gag acg gtg cgc ggc ctg     2304
   Gln Ala Thr Ser Pro Ala Ala Asn Val Leu Glu Thr Val Arg Gly Leu
           755                 760                 765
   ttg cag cat cgc tca ttt acc atg agc aac ccg aac cgt att cgt tcg     2352
   Leu Gln His Arg Ser Phe Thr Met Ser Asn Pro Asn Arg Ile Arg Ser
       770                 775                 780
   ttg att ggc gcg ttt gcg ggc agc aat ccg gca gcg ttc cat gcc gaa     2400
   Leu Ile Gly Ala Phe Ala Gly Ser Asn Pro Ala Ala Phe His Ala Glu
   785                 790                 795                 800
   gat ggc agc ggt tac ctg ttc ctg gtg gaa atg ctt acc gac ctc aac     2448
   Asp Gly Ser Gly Tyr Leu Phe Leu Val Glu Met Leu Thr Asp Leu Asn
                   805                 810                 815
   agc cgt aac ccg cag gtg gct tca cgt ctg att gaa ccg ctg att cgc     2496
   Ser Arg Asn Pro Gln Val Ala Ser Arg Leu Ile Glu Pro Leu Ile Arg
               820                 825                 830
   ctg aaa cgt tac gat gcc aaa cgt cag gag aaa atg cgc gcg gcg ctg     2544
   Leu Lys Arg Tyr Asp Ala Lys Arg Gln Glu Lys Met Arg Ala Ala Leu
           835                 840                 845
   gaa cag ttg aaa ggg ctg gaa aat ctc tct ggc gat ctg tac gag aag     2592
   Glu Gln Leu Lys Gly Leu Glu Asn Leu Ser Gly Asp Leu Tyr Glu Lys
       850                 855                 860
   ata act aaa gca ctg gct                                             2610
   Ile Thr Lys Ala Leu Ala
   865                 870
<210> 14
   <211> 1605
   <212> DNA
   <213> Escherichia coli
<400> 14
   atg cgt att tcc ttg aaa aag tca ggg atg ctg aag ctt ggt ctc agc      48
   Met Arg Ile Ser Leu Lys Lys Ser Gly Met Leu Lys Leu Gly Leu Ser
   1               5                   10                  15
   ctg gtg gct atg acc gtc gca gca agt gtt cag gct aaa act ctg gtt      96
   Leu Val Ala Met Thr Val Ala Ala Ser Val Gln Ala Lys Thr Leu Val
               20                  25                  30
   tat tgc tca gaa gga tct ccg gaa ggg ttt aac ccg cag ctg ttt acc      144
   Tyr Cys Ser Glu Gly Ser Pro Glu Gly Phe Asn Pro Gln Leu Phe Thr
           35                  40                  45
   tcc ggc acc acc tat gac gcc tct tcc gtc ccg ctt tat aac cgt ctg      192
   Ser Gly Thr Thr Tyr Asp Ala Ser Ser Val Pro Leu Tyr Asn Arg Leu
       50                  55                  60
   gtt gaa ttt aaa atc ggc acc acc gaa gtg atc ccg ggc ctc gct gaa      240
   Val Glu Phe Lys Ile Gly Thr Thr Glu Val Ile Pro Gly Leu Ala Glu
   65 70 75 80
   aag tgg gaa gtc agc gaa gac ggt aaa acc tat acc ttc cat ctg cgt 288
   Lys Trp Glu Val Ser Glu Asp Gly Lys Thr Tyr Thr Phe His Leu Arg
   85 90 95
   aaa ggt gtg aag tgg cac gac aat aaa gaa ttc aaa ccg acg cgt gaa 336
   Lys Gly Val Lys Trp His Asp Asn Lys Glu Phe Lys Pro Thr Arg Glu
   100 105 110
   ctg aac gcc gat gat gtg gtg ttc tcg ttc gat cgt cag aaa aac gcg 384
   Leu Asn Ala Asp Asp Val Val Phe Ser Phe Asp Arg Gln Lys Asn Ala
   115 120 125
   caa aac ccg tac cat aaa gtt tct ggc ggc agc tac gaa tac ttc gaa 432
   Gln Asn Pro Tyr His Lys Val Ser Gly Gly Ser Tyr Glu Tyr Phe Glu
   130 135 140
   ggc atg ggc ttg cca gag ctg atc agt gaa gtg aaa aag gtg gac gac 480
   Gly Met Gly Leu Pro Glu Leu Ile Ser Glu Val Lys Lys Val Asp Asp
   145 150 155 160
   aac acc gtt cag ttt gtg ctg act cgc ccg gaa gcg ccg ttc ctc gct 528
   Asn Thr Val Gln Phe Val Leu Thr Arg Pro Glu Ala Pro Phe Leu Ala
   165 170 175
   gac ctg gca atg gac ttc gcc tct att ctg tca aaa gaa tat gct gat 576
   Asp Leu Ala Met Asp Phe Ala Ser Ile Leu Ser Lys Glu Tyr Ala Asp
   180 185 190
   gcg atg atg aaa gcc ggt aca ccg gaa aaa ctg gac ctc aac cca atc 624
   Ala Met Met Lys Ala Gly Thr Pro Glu Lys Leu Asp Leu Asn Pro Ile
   195 200 205
   gga acc ggt ccg ttc cag tta cag cag tat caa aaa gat tcc cgt atc 672
   Gly Thr Gly Pro Phe Gln Leu Gln Gln Tyr Gln Lys Asp Ser Arg Ile
   210 215 220
   cgc tac aaa gcg ttt gat ggc tac tgg ggc acc aaa ccg cag atc gat 720
   Arg Tyr Lys Ala Phe Asp Gly Tyr Trp Gly Thr Lys Pro Gln Ile Asp
   225 230 235 240
   acg ctg gtt ttc tct att acc cct gac gct tcc gtg cgt tac gcg aaa 768
   Thr Leu Val Phe Ser Ile Thr Pro Asp Ala Ser Val Arg Tyr Ala Lys
   245 250 255
   ttg cag aag aat gaa tgc cag gtg atg ccg tac ccg aac ccg gca gat 816
   Leu Gln Lys Asn Glu Cys Gln Val Met Pro Tyr Pro Asn Pro Ala Asp
   260 265 270
   atc gct cgc atg aag cag gat aaa tcc atc aat ctg atg gaa atg ccg 864
   Ile Ala Arg Met Lys Gln Asp Lys Ser Ile Asn Leu Met Glu Met Pro
   275 280 285
   ggg ctg aac gtc ggt tat ctc tcg tat aac gtg cag aaa aaa cca ctc 912
   Gly Leu Asn Val Gly Tyr Leu Ser Tyr Asn Val Gln Lys Lys Pro Leu
   290 295 300
   gat gac gtg aaa gtt cgc cag gct ctg acc tac gcg gtg aac aaa gac 960
   Asp Asp Val Lys Val Arg Gln Ala Leu Thr Tyr Ala Val Asn Lys Asp
   305 310 315 320
   gcg atc atc aaa gcg gtt tat cag ggc gcg ggc gta tca gcg aaa aac 1008
   Ala Ile Ile Lys Ala Val Tyr Gln Gly Ala Gly Val Ser Ala Lys Asn
   325 330 335
   ctg atc ccg cca acc atg tgg ggc tat aac gac gac gtt cag gac tac 1056
   Leu Ile Pro Pro Thr Met Trp Gly Tyr Asn Asp Asp Val Gln Asp Tyr
               340                 345                 350
   acc tac gat cct gaa aaa gcg aaa gcc ttg ctg aaa gaa gcg ggt ctg      1104
   Thr Tyr Asp Pro Glu Lys Ala Lys Ala Leu Leu Lys Glu Ala Gly Leu
           355                 360                 365
   gaa aaa ggt ttc tcc atc gac ctg tgg gcg atg ccg gta caa cgt ccg      1152
   Glu Lys Gly Phe Ser Ile Asp Leu Trp Ala Met Pro Val Gln Arg Pro
       370                 375                 380
   tat aac ccg aac gct cgc cgc atg gcg gag atg att cag gca gac tgg      1200
   Tyr Asn Pro Asn Ala Arg Arg Met Ala Glu Met Ile Gln Ala Asp Trp
   385                 390                 395                 400
   gcg aaa gtc ggc gtg cag gcc aaa att gtc acc tac gaa tgg ggt gag      1248
   Ala Lys Val Gly Val Gln Ala Lys Ile Val Thr Tyr Glu Trp Gly Glu
                   405                 410                 415
   tac ctc aag cgt gcg aaa gat ggc gag cac cag acg gta atg atg ggc      1296
   Tyr Leu Lys Arg Ala Lys Asp Gly Glu His Gln Thr Val Met Met Gly
               420                 425                 430
   tgg act ggc gat aac ggg gat ccg gat aac ttc ttc gcc acc ctg ttc      1344
   Trp Thr Gly Asp Asn Gly Asp Pro Asp Asn Phe Phe Ala Thr Leu Phe
           435                 440                 445
   agc tgc gcc gcc tct gaa caa ggc tcc aac tac tca aaa tgg tgc tac      1392
   Ser Cys Ala Ala Ser Glu Gln Gly Ser Asn Tyr Ser Lys Trp Cys Tyr
       450                 455                 460
   aaa ccg ttt gaa gat ctg att caa ccg gcg cgt gct acc gac gac cac      1440
   Lys Pro Phe Glu Asp Leu Ile Gln Pro Ala Arg Ala Thr Asp Asp His
   465                 470                 475                 480
   aat aaa cgc gtt gaa ctg tac aaa caa gcg cag gtg gtg atg cac gat      1488
   Asn Lys Arg Val Glu Leu Tyr Lys Gln Ala Gln Val Val Met His Asp
                   485                 490                 495
   cag gct ccg gca ctg atc atc gct cac tcc acc gtg ttt gaa ccg gta      1536
   Gln Ala Pro Ala Leu Ile Ile Ala His Ser Thr Val Phe Glu Pro Val
               500                 505                 510
   cgt aaa gaa gtt aaa ggc tat gtg gtt gat cca tta ggc aaa cat cac      1584
   Arg Lys Glu Val Lys Gly Tyr Val Val Asp Pro Leu Gly Lys His His
           515                 520                 525
   ttc gaa aac gtc tct atc gaa                                          1605
   Phe Glu Asn Val Ser Ile Glu
       530                 535
<210> 15
   <211> 1017
   <212> DNA
   <213> Escherichia coli
<400> 15
   atg ttg cag ttt att ctc cga cgt ttg gga ctc gtc atc ccc acg ttt    48
   Met Leu Gln Phe Ile Leu Arg Arg Leu Gly Leu Val Ile Pro Thr Phe
    1               5                  10                  15
   atc ggt att acc ctt ctc aca ttt gcc ttt gtc cac atg atc ccg ggc    96
   Ile Gly Ile Thr Leu Leu Thr Phe Ala Phe Val His Met Ile Pro Gly
               20                  25                  30
   gat ccg gtg atg atc atg gcg ggc gaa cgt ggg atc tcc cca gag cgt    144
   Asp Pro Val Met Ile Met Ala Gly Glu Arg Gly Ile Ser Pro Glu Arg
               35                  40                  45
   cac gcg cag ctg ctg gct gaa ctc ggc tta gat aaa ccg atg tgg cag     192
   His Ala Gln Leu Leu Ala Glu Leu Gly Leu Asp Lys Pro Met Trp Gln
       50                  55                  60
   cag tat ctc cat tac att tgg ggc gtt atg cat ggc gat cta ggc att     240
   Gln Tyr Leu His Tyr Ile Trp Gly Val Met His Gly Asp Leu Gly Ile
   65                  70                  75                  80
   tca atg aaa agc cgc atc ccg gtt tgg gaa gag ttc gtg ccg cgc ttc     288
   Ser Met Lys Ser Arg Ile Pro Val Trp Glu Glu Phe Val Pro Arg Phe
                   85                  90                  95
   cag gcc acg ctg gaa ctt ggc gtc tgc gcg atg att ttt gct acg gca     336
   Gln Ala Thr Leu Glu Leu Gly Val Cys Ala Met Ile Phe Ala Thr Ala
               100                 105                 110
   gtc ggt att ccg gtc ggc gtg ctg gct gcg gtt aaa cgc ggt tcc att     384
   Val Gly Ile Pro Val Gly Val Leu Ala Ala Val Lys Arg Gly Ser Ile
           115                 120                 125
   ttc gat cac aca gcg gtt ggc ctg gcg ctg aca ggt tat tca atg cct     432
   Phe Asp His Thr Ala Val Gly Leu Ala Leu Thr Gly Tyr Ser Met Pro
       130                 135                 140
   atc ttc tgg tgg ggc atg atg ctg atc atg ctg gtt tcg gtg cac tgg     480
   Ile Phe Trp Trp Gly Met Met Leu Ile Met Leu Val Ser Val His Trp
   145                 150                 155                 160
   aac ctg acg ccc gtc tcc ggt cgc gtg agc gat atg gtg ttc ctc gat     528
   Asn Leu Thr Pro Val Ser Gly Arg Val Ser Asp Met Val Phe Leu Asp
                   165                 170                 175
   gac tcc aat ccg tta acc ggt ttt atg cta atc gac acc gcc atc tgg     576
   Asp Ser Asn Pro Leu Thr Gly Phe Met Leu Ile Asp Thr Ala Ile Trp
               180                 185                 190
   ggt gaa gac ggc aac ttt atc gat gcc gtc gcc cat atg atc ttg cct     624
   Gly Glu Asp Gly Asn Phe Ile Asp Ala Val Ala His Met Ile Leu Pro
           195                 200                 205
   gcc att gtg ctg ggt act att ccg ctg gcg gtc att gtg cgt atg aca     672
   Ala Ile Val Leu Gly Thr Ile Pro Leu Ala Val Ile Val Arg Met Thr
       210                 215                 220
   cgc tcc tcg atg ctg gaa gtg ctg ggc gag gat tac atc cgc acc gcg     720
   Arg Ser Ser Met Leu Glu Val Leu Gly Glu Asp Tyr Ile Arg Thr Ala
   225                 230                 235                 240
   cgc gcc aaa ggg cta acc cgc atg cgg gtg att atc gtc cat gcg ctg     768
   Arg Ala Lys Gly Leu Thr Arg Met Arg Val Ile Ile Val His Ala Leu
                   245                 250                 255
   cgt aac gcg atg ctg ccg gtg gtg acc gtt atc ggc ctg cag gtg gga     816
   Arg Asn Ala Met Leu Pro Val Val Thr Val Ile Gly Leu Gln Val Gly
               260                 265                 270
   aca ttg ctg gcg ggg gcg att ctg acc gaa acc atc ttc tcg tgg ccc     864
   Thr Leu Leu Ala Gly Ala Ile Leu Thr Glu Thr Ile Phe Ser Trp Pro
           275                 280                 285
   ggt ctg gga cgc tgg ttg att gac gca ctg caa cgc cgc gac tat ccg     912
   Gly Leu Gly Arg Trp Leu Ile Asp Ala Leu Gln Arg Arg Asp Tyr Pro
       290                 295                 300
   gta gtg cag ggc ggc gta ttg ctg gtg gcg acg atg att atc ctc gtc     960
   Val Val Gln Gly Gly Val Leu Leu Val Ala Thr Met Ile Ile Leu Val
   305                 310                 315                 320
   aac ttg ctg gtc gat ctg ctg tac ggc gtg gtg aac ccg cgt att cgt      1008
   Asn Leu Leu Val Asp Leu Leu Tyr Gly Val Val Asn Pro Arg Ile Arg
                   325                 330                 335
   cat aag aag                                                          1017
   His Lys Lys
<210> 16
   <211> 900
   <212> DNA
   <213> Escherichia coli
<400> 16
   atg tca cag gtt act gaa aat aaa gtg att agc gca ccg gtg ccg atg 48
   Met Ser Gln Val Thr Glu Asn Lys Val Ile Ser Ala Pro Val Pro Met
   1 5 10 15
   acc ccg tta cag gag ttc tgg cac tat ttt aaa cgc aac aaa ggc gcg 96
   Thr Pro Leu Gln Glu Phe Trp His Tyr Phe Lys Arg Asn Lys Gly Ala
   20 25 30
   gtc gtc ggg ctg gtt tac gtc gtc atc gtg ctg ttc atc gcg atc ttt 144
   Val Val Gly Leu Val Tyr Val Val Ile Val Leu Phe Ile Ala Ile Phe
   35 40 45
   gcc aac tgg att gca ccc tat aac ccg gcg gaa cag ttc cgc gat gca 192
   Ala Asn Trp Ile Ala Pro Tyr Asn Pro Ala Glu Gln Phe Arg Asp Ala
   50 55 60
   ctg ctc gcc ccg cca gcc tgg cag gaa ggc ggc agc atg gcg cac ttg 240
   Leu Leu Ala Pro Pro Ala Trp Gln Glu Gly Gly Ser Met Ala His Leu
   65 70 75 80
   ctg ggc acc gat gac gta ggc cgt gat gtg ctg tcg cgc ctg atg tac 288
   Leu Gly Thr Asp Asp Val Gly Arg Asp Val Leu Ser Arg Leu Met Tyr
   85 90 95
   ggt gcg cgc ctg tcg ctg ctg gtt ggc tgt ctg gta gtt gtg tta tcg 336
   Gly Ala Arg Leu Ser Leu Leu Val Gly Cys Leu Val Val Val Leu Ser
   100 105 110
   ctg att atg ggc gtt att ctc ggc ctg atc gcc ggt tac ttt ggc ggc 384
   Leu Ile Met Gly Val Ile Leu Gly Leu Ile Ala Gly Tyr Phe Gly Gly
   115 120 125
   ctg gtc gat aac atc att atg cgc gtg gtc gat atc atg ctg gcg ctg 432
   Leu Val Asp Asn Ile Ile Met Arg Val Val Asp Ile Met Leu Ala Leu
   130 135 140
   cca agt ctg ctg ctg gcg ctg gtg ctg gtg gca att ttc ggc ccg tcg 480
   Pro Ser Leu Leu Leu Ala Leu Val Leu Val Ala Ile Phe Gly Pro Ser
   145 150 155 160
   att ggt aac gcc gcg ctg gca ctg acc ttc gtt gcc ttg ccg cac tat 528
   Ile Gly Asn Ala Ala Leu Ala Leu Thr Phe Val Ala Leu Pro His Tyr
   165 170 175
   gtg cgc tta acc cgc gcc gcc gtg ctg gtg gaa gtt aac cgc gat tac 576
   Val Arg Leu Thr Arg Ala Ala Val Leu Val Glu Val Asn Arg Asp Tyr
   180 185 190
   gtc acc gcg tct cgc gtg gcg ggt gcc ggg gcg atg cgt cag atg ttt 624
   Val Thr Ala Ser Arg Val Ala Gly Ala Gly Ala Met Arg Gln Met Phe
   195 200 205
   att aac atc ttc ccg aac tgc ctt gcg ccg ctg att gtt cag gcg tcg      672
   Ile Asn Ile Phe Pro Asn Cys Leu Ala Pro Leu Ile Val Gln Ala Ser
       210                 215                 220
   ctc ggt ttc tct aac gcc att ctc gat atg gct gct ctt ggt ttc ctc      720
   Leu Gly Phe Ser Asn Ala Ile Leu Asp Met Ala Ala Leu Gly Phe Leu
   225                 230                 235                 240
   ggc atg ggg gca cag ccg cca acg cct gag tgg ggc acc atg ctc tcc      768
   Gly Met Gly Ala Gln Pro Pro Thr Pro Glu Trp Gly Thr Met Leu Ser
                   245                 250                 255
   gac gtg ttg cag ttc gcg caa agc gcc tgg tgg gtc gtg acc ttc ccg      816
   Asp Val Leu Gln Phe Ala Gln Ser Ala Trp Trp Val Val Thr Phe Pro
               260                 265                 270
   ggt ctg gcg atc ctg ctg acg gtg ctg gca ttt aac ctg atg ggt gac      864
   Gly Leu Ala Ile Leu Leu Thr Val Leu Ala Phe Asn Leu Met Gly Asp
           275                 280                 285
   ggt ctg cgt gac gcg ctc gat ccc aaa ctg aag cag                      900
   Gly Leu Arg Asp Ala Leu Asp Pro Lys Leu Lys Gln
       290                 295                 300
<210> 17
   <211> 981
   <212> DNA
   <213> Escherichia coli
<400> 17
   atg gcg tta tta aat gta gat aaa tta tcg gtg cat ttc ggc gac gaa    48
   Met Ala Leu Leu Asn Val Asp Lys Leu Ser Val His Phe Gly Asp Glu
    1               5                  10                  15
   agc gcg ccg ttc cgc gcc gta gac cgc atc agc tac agc gta aaa cag    96
   Ser Ala Pro Phe Arg Ala Val Asp Arg Ile Ser Tyr Ser Val Lys Gln
               20                  25                  30
   ggc gaa gtg gtc ggg att gtg ggt gag tcc ggc tcc ggt aag tcg gtc   144
   Gly Glu Val Val Gly Ile Val Gly Glu Ser Gly Ser Gly Lys Ser Val
           35                  40                  45
   agt tca ctg gcg att atg ggg ctg att gat tat ccg ggc cgc gta atg   192
   Ser Ser Leu Ala Ile Met Gly Leu Ile Asp Tyr Pro Gly Arg Val Met
       50                  55                  60
   gca gaa aaa ctg gag ttt aac ggc cag gat ttg cag cgt atc tca gaa   240
   Ala Glu Lys Leu Glu Phe Asn Gly Gln Asp Leu Gln Arg Ile Ser Glu
   65                  70                  75                  80
   aaa gag cgc cgc aac ctg gtg ggt gcc gaa gtg gcg atg atc ttc cag   288
   Lys Glu Arg Arg Asn Leu Val Gly Ala Glu Val Ala Met Ile Phe Gln
                   85                  90                  95
   gac ccg atg acc agc ctt aac ccg tgc tac acc gtg ggt ttc cag att   336
   Asp Pro Met Thr Ser Leu Asn Pro Cys Tyr Thr Val Gly Phe Gln Ile
               100                 105                 110
   atg gaa gcg att aag gtg cat cag ggc ggc aac aaa agt acc cgc cgt   384
   Met Glu Ala Ile Lys Val His Gln Gly Gly Asn Lys Ser Thr Arg Arg
           115                 120                 125
   cag cga gcg att gac ctg ctg aat cag gtc ggt att ccc gat ccg gca   432
   Gln Arg Ala Ile Asp Leu Leu Asn Gln Val Gly Ile Pro Asp Pro Ala
       130                 135                 140
   tcg cgt ctg gat gtt tac ccg cat cag ctt tcc ggc ggc atg agc cag   480
   Ser Arg Leu Asp Val Tyr Pro His Gln Leu Ser Gly Gly Met Ser Gln
   145                 150                 155                 160
   cgc gtg atg atc gcc atg gcg att gcc tgt cgg cca aaa ctg ctg att      528
   Arg Val Met Ile Ala Met Ala Ile Ala Cys Arg Pro Lys Leu Leu Ile
                   165                 170                 175
   gcc gat gaa ccg acc acc gcg ctg gac gtg acc att cag gcg caa atc      576
   Ala Asp Glu Pro Thr Thr Ala Leu Asp Val Thr Ile Gln Ala Gln Ile
               180                 185                 190
   atc gaa cta ctg ctg gag cta cag cag aaa gag aac atg gcg ctg gtg      624
   Ile Glu Leu Leu Leu Glu Leu Gln Gln Lys Glu Asn Met Ala Leu Val
           195                 200                 205
   tta att acc cat gac ctg gcg ctg gtg gcg gaa gcg gca cat aaa atc      672
   Leu Ile Thr His Asp Leu Ala Leu Val Ala Glu Ala Ala His Lys Ile
       210                 215                 220
   atc gtg atg tat gca ggc cag gtg gtg gaa acc ggt gat gcg cac gcc      720
   Ile Val Met Tyr Ala Gly Gln Val Val Glu Thr Gly Asp Ala His Ala
   225                 230                 235                 240
   atc ttc cat gcg ccg cgt cac ccg tat act cag gca ttg ctg cgt gcg      768
   Ile Phe His Ala Pro Arg His Pro Tyr Thr Gln Ala Leu Leu Arg Ala
                   245                 250                 255
   ctg cca gaa ttt gct cag gac aaa gaa cgt ctg gcg tcg ttg cca ggt      816
   Leu Pro Glu Phe Ala Gln Asp Lys Glu Arg Leu Ala Ser Leu Pro Gly
               260                 265                 270
   gtc gtt ccc ggc aag tac gac cgc ccg aac ggc tgc ctg ctt aac ccg      864
   Val Val Pro Gly Lys Tyr Asp Arg Pro Asn Gly Cys Leu Leu Asn Pro
           275                 280                 285
   cgc tgc ccc tat gcc act gac aga tgt cgc gct gaa gaa ccg gcg ctg      912
   Arg Cys Pro Tyr Ala Thr Asp Arg Cys Arg Ala Glu Glu Pro Ala Leu
       290                 295                 300
   aat atg ctc gct gac ggg cgt cag tcc aaa tgc cat tac cca ctt gat      960
   Asn Met Leu Ala Asp Gly Arg Gln Ser Lys Cys His Tyr Pro Leu Asp
   305                 310                 315                 320
   gat gcc ggg agg ccg aca cta                                          981
   Asp Ala Gly Arg Pro Thr Leu
                   325
<210> 18
   <211> 1002
   <212> DNA
   <213> Escherichia coli
<400> 18
   atg agt acg caa gag gcc acc ctg caa caa ccg ctg ttg cag gct atc      48
   Met Ser Thr Gln Glu Ala Thr Leu Gln Gln Pro Leu Leu Gln Ala Ile
    1               5                  10                  15
   gac ctg aaa aaa cat tat ccg gtg aag aaa ggc atg ttc gcg ccg gaa      96
   Asp Leu Lys Lys His Tyr Pro Val Lys Lys Gly Met Phe Ala Pro Glu
               20                  25                  30
   cgt ctg gtt aaa gcg ctg gat ggc gtt tcg ttt aac ctt gaa cgt ggc      144
   Arg Leu Val Lys Ala Leu Asp Gly Val Ser Phe Asn Leu Glu Arg Gly
           35                  40                  45
   aaa acg ctg gca gta gtg ggc gaa tct ggc tgc ggt aaa tcg acc ctc      192
   Lys Thr Leu Ala Val Val Gly Glu Ser Gly Cys Gly Lys Ser Thr Leu
   50 55 60
   ggt cgg ttg ctg acg atg att gaa atg ccc acc ggt ggc gag ctg tat 240
   Gly Arg Leu Leu Thr Met Ile Glu Met Pro Thr Gly Gly Glu Leu Tyr
   65 70 75 80
   tac cag ggg cag gat ctg ctt aag cac gat ccg cag gcg cag aag ctg 288
   Tyr Gln Gly Gln Asp Leu Leu Lys His Asp Pro Gln Ala Gln Lys Leu
   85 90 95
   cgt cgg cag aaa atc cag atc gtc ttc cag aac cct tac ggt tcg ctg 336
   Arg Arg Gln Lys Ile Gln Ile Val Phe Gln Asn Pro Tyr Gly Ser Leu
   100 105 110
   aat ccg cgt aaa aaa gtc ggg caa att ctt gaa gag ccg ctg ctg atc 384
   Asn Pro Arg Lys Lys Val Gly Gln Ile Leu Glu Glu Pro Leu Leu Ile
   115 120 125
   aac acc agc tta agc aaa gaa cag cgt cgg gaa aaa gcc ctg tcg atg 432
   Asn Thr Ser Leu Ser Lys Glu Gln Arg Arg Glu Lys Ala Leu Ser Met
   130 135 140
   atg gcg aaa gtc ggc ctg aaa acc gag cac tat gac cgc tat ccg cat 480
   Met Ala Lys Val Gly Leu Lys Thr Glu His Tyr Asp Arg Tyr Pro His
   145 150 155 160
   atg ttc tcc ggc ggt cag cgt cag cgt atc gcc atc gcc cgt ggt ctg 528
   Met Phe Ser Gly Gly Gln Arg Gln Arg Ile Ala Ile Ala Arg Gly Leu
   165 170 175
   atg ctc gac ccg gat gtg gtg att gcc gat gaa ccg gtt tcc gcg ctg 576
   Met Leu Asp Pro Asp Val Val Ile Ala Asp Glu Pro Val Ser Ala Leu
   180 185 190
   gat gtt tca gtg cgc gcg cag gtg ctg aat ctg atg atg gat ttg cag 624
   Asp Val Ser Val Arg Ala Gln Val Leu Asn Leu Met Met Asp Leu Gln
   195 200 205
   cag gag ttg ggg ctg tct tat gtc ttt atc tcc cac gac ctg tcg gtg 672
   Gln Glu Leu Gly Leu Ser Tyr Val Phe Ile Ser His Asp Leu Ser Val
   210 215 220
   gtg gag cac att gct gat gaa gtg atg gtg atg tac ctg ggc cgc tgc 720
   Val Glu His Ile Ala Asp Glu Val Met Val Met Tyr Leu Gly Arg Cys
   225 230 235 240
   gtg gag aag gga acg aaa gac caa atc ttc aat aac ccg cgc cat ccg 768
   Val Glu Lys Gly Thr Lys Asp Gln Ile Phe Asn Asn Pro Arg His Pro
   245 250 255
   tac act cag gcg cta ctt tcc gcg acg ccg cgc ctg aac ccg gac gat 816
   Tyr Thr Gln Ala Leu Leu Ser Ala Thr Pro Arg Leu Asn Pro Asp Asp
   260 265 270
   cgc cgc gag cgc atc aag ctc agc ggt gaa cta cca agc cca ctg aat 864
   Arg Arg Glu Arg Ile Lys Leu Ser Gly Glu Leu Pro Ser Pro Leu Asn
   275 280 285
   cca ccg ccg ggt tgc gcc ttc aac gcc cgc tgt cgt cgg cgc ttc ggc 912
   Pro Pro Pro Gly Cys Ala Phe Asn Ala Arg Cys Arg Arg Arg Phe Gly
   290 295 300
   ccc tgc acc cag ttg cag ccg cag cta aaa gac tac ggc ggt caa ctg 960
   Pro Cys Thr Gln Leu Gln Pro Gln Leu Lys Asp Tyr Gly Gly Gln Leu
   305 310 315 320
   gta gct tgt ttt gct gtt gat cag gat gaa aat ccg cag cgt 1002
   Val Ala Cys Phe Ala Val Asp Gln Asp Glu Asn Pro Gln Arg
   325 330
<210> 19
   <211> 472
   <212> PRT
   <213> Bacillus subtilis 168
<400> 19
   Met Glu Arg Lys Thr Val Leu Val Ile Ala Asp Leu Gly Gly Cys Pro
   1               5                   10                  15
   Pro His Met Phe Tyr Lys Ser Ala Ala Glu Lys Tyr Asn Leu Val Ser
               20                  25                  30
   Phe Ile Pro Arg Pro Phe Ala Ile Thr Ala Ser His Ala Ala Leu Ile
           35                  40                  45
   Glu Lys Tyr Ser Val Ala Val Ile Lys Asp Lys Asp Tyr Phe Lys Ser
       50                  55                  60
   Leu Ala Asp Phe Glu His Pro Asp Ser Ile Tyr Trp Ala His Glu Asp
   65                  70                  75                  80
   His Asn Lys Pro Glu Glu Glu Val Val Glu Gln Ile Val Lys Val Ala
                   85                  90                  95
   Glu Met Phe Gly Ala Asp Ala Ile Thr Thr Asn Asn Glu Leu Phe Ile
               100                 105                 110
   Ala Pro Met Ala Lys Ala Cys Glu Arg Leu Gly Leu Arg Gly Ala Gly
           115                 120                 125
   Val Gln Ala Ala Glu Asn Ala Arg Asp Lys Asn Lys Met Arg Asp Ala
       130                 135                 140
   Phe Asn Lys Ala Gly Val Lys Ser Ile Lys Asn Lys Arg Val Thr Thr
   145                 150                 155                 160
   Leu Glu Asp Phe Arg Ala Ala Leu Glu Glu Ile Gly Thr Pro Leu Ile
                   165                 170                 175
   Leu Lys Pro Thr Tyr Leu Ala Ser Ser Ile Gly Val Thr Leu Ile Thr
               180                 185                 190
   Asp Thr Glu Thr Ala Glu Asp Glu Phe Asn Arg Val Asn Asp Tyr Leu
           195                 200                 205
   Lys Ser Ile Asn Val Pro Lys Ala Val Thr Phe Glu Ala Pro Phe Ile
       210                 215                 220
   Ala Glu Glu Phe Leu Gln Gly Glu Tyr Gly Asp Trp Tyr Gln Thr Glu
   225                 230                 235                 240
   Gly Tyr Ser Asp Tyr Ile Ser Ile Glu Gly Ile Met Ala Asp Gly Glu
                   245                 250                 255
   Tyr Phe Pro Ile Ala Ile His Asp Lys Thr Pro Gln Ile Gly Phe Thr
               260                 265                 270
   Glu Thr Ser His Ile Thr Pro Ser Ile Leu Asp Glu Glu Ala Lys Lys
           275                 280                 285
   Lys Ile Val Glu Ala Ala Lys Lys Ala Asn Glu Gly Leu Gly Leu Gln
       290                 295                 300
   Asn Cys Ala Thr His Thr Glu Ile Lys Leu Met Lys Asn Arg Glu Pro
   305                 310                 315                 320
   Gly Leu Ile Glu Ser Ala Ala Arg Phe Ala Gly Trp Asn Met Ile Pro
   325 330 335
   Asn Ile Lys Lys Val Phe Gly Leu Asp Met Ala Gln Leu Leu Leu Asp
   340 345 350
   Val Leu Cys Phe Gly Lys Asp Ala Asp Leu Pro Asp Gly Leu Leu Asp
   355 360 365
   Gln Glu Pro Tyr Tyr Val Ala Asp Cys His Leu Tyr Pro Gln His Phe
   370 375 380
   Lys Gln Asn Gly Gln Ile Pro Glu Thr Ala Glu Asp Leu Val Ile Glu
   385 390 395 400
   Ala Ile Asp Ile Pro Asp Gly Leu Leu Lys Gly Asp Thr Glu Ile Val
   405 410 415
   Ser Phe Ser Ala Ala Ala Pro Gly Thr Ser Val Asp Leu Thr Leu Phe
   420 425 430
   Glu Ala Phe Asn Ser Ile Ala Ala Phe Glu Leu Lys Gly Ser Asn Ser
   435 440 445
   Gln Asp Val Ala Glu Ser Ile Arg Gln Ile Gln Gln His Ala Lys Leu
   450 455 460
   Thr Ala Lys Tyr Val Leu Pro Val
   465 470
<210> 20
   <211> 472
   <212> PRT
   <213> Bacillus subtilis ATCC6633
<400> 20
   Met Glu Arg Lys Thr Val Leu Val Ile Ala Asp Leu Gly Gly Cys Pro
   1 5 10 15
   Pro His Met Phe Tyr Lys Ser Ala Ala Glu Lys Tyr Asn Leu Val Ser
   20 25 30
   Phe Ile Pro Arg Pro Phe Ala Ile Thr Ala Ser His Ala Ala Leu Ile
   35 40 45
   Glu Lys Tyr Ser Val Ala Val Ile Lys Asp Lys Asp Tyr Phe Gln Ser
   50 55 60
   Leu Ala Asp Phe Glu His Pro Asp Ser Ile Tyr Trp Ala His Glu Asp
   65 70 75 80
   His Asp Lys Pro Glu Glu Glu Val Val Glu Gln Ile Val Lys Val Ala
   85 90 95
   Gln Met Phe Glu Ala Asp Ala Ile Thr Thr Asn Asn Glu Leu Phe Ile
   100 105 110
   Ala Pro Met Ala Lys Ala Cys Glu Arg Leu Gly Leu Arg Gly Ala Gly
   115 120 125
   Val Gln Ala Ala Glu Asn Ala Arg Asp Lys Asn Lys Met Arg Asp Ala
   130 135 140
   Phe Asn Lys Ala Gly Val Lys Ser Ile Lys Asn Lys Arg Val Thr Thr
   145 150 155 160
   Leu Glu Asp Phe Arg Ala Ala Leu Glu Glu Ile Gly Thr Pro Leu Ile
   165 170 175
   Leu Lys Pro Thr Tyr Leu Ala Ser Ser Ile Gly Val Thr Leu Ile Thr
   180 185 190
   Asp Thr Glu Thr Ala Glu Asp Glu Phe Asn Arg Val Asn Asp Tyr Leu
   195 200 205
   Lys Ser Ile Asn Val Pro Lys Ala Val Thr Phe Glu Ala Pro Phe Ile
   210 215 220
   Ala Glu Glu Phe Leu Gln Gly Glu Tyr Gly Asp Trp Tyr Gln Thr Glu
   225 230 235 240
   Gly Tyr Ser Asp Tyr Ile Ser Ile Glu Gly Ile Met Ala Asp Gly Glu
   245 250 255
   Tyr Phe Pro Ile Ala Ile His Asp Lys Thr Pro Gln Ile Gly Phe Thr
   260 265 270
   Glu Thr Ser His Ile Thr Pro Ser Ile Leu Asp Glu Glu Ala Lys Lys
   275 280 285
   Lys Ile Val Glu Ala Ala Lys Lys Ala Asn Glu Gly Leu Gly Leu Gln
   290 295 300
   Asn Cys Ala Thr His Thr Glu Ile Lys Leu Met Lys Asn Arg Glu Pro
   305 310 315 320
   Gly Leu Ile Glu Ser Ala Ala Arg Phe Ala Gly Trp Asn Met Ile Pro
   325 330 335
   Asn Ile Lys Lys Val Phe Gly Leu Asp Met Ala Gln Leu Leu Leu Asp
   340 345 350
   Val Leu Cys Phe Gly Lys Asp Ala Asp Leu Pro Asp Gly Leu Leu Asp
   355 360 365
   Gln Glu Pro Tyr Tyr Val Ala Asp Cys His Leu Tyr Pro Gln His Phe
   370 375 380
   Lys Gln Asn Gly Gln Ile Pro Glu Thr Ala Glu Asp Leu Val Ile Glu
   385 390 395 400
   Ala Ile Asp Ile Pro Asp Gly Leu Leu Lys Gly Asp Thr Glu Ile Val
   405 410 415
   Thr Phe Ser Ala Ala Ala Pro Gly Thr Ser Val Asp Leu Thr Leu Phe
   420 425 430
   Glu Ala Phe Asn Ser Ile Ala Ala Phe Glu Leu Lys Gly Ser Asn Ser
   435 440 445
   Gln Asp Val Ala Glu Ser Ile Arg Gln Ile Gln Gln His Ala Lys Leu
   450 455 460
   Thr Ala Lys Tyr Val Leu Pro Val
   465 470
<210> 21
   <211> 472
   <212> PRT
   <213> Bacillus subtilis IAM1213
<400> 21
   Met Glu Arg Lys Thr Val Leu Val Ile Ala Asp Leu Gly Gly Cys Pro
   1 5 10 15
   Pro His Met Phe Tyr Lys Ser Ala Ala Glu Lys Tyr Asn Leu Val Ser
   20 25 30
   Phe Ile Pro Arg Pro Phe Ala Ile Thr Ala Ser His Ala Ala Leu Ile
   35 40 45
   Glu Lys Tyr Ser Val Ala Val Ile Lys Asp Lys Asp Tyr Phe Lys Ser
   50 55 60
   Leu Ala Asp Phe Glu His Pro Asp Ser Ile Tyr Trp Ala His Glu Asp
   65 70 75 80
   His Asn Lys Pro Glu Glu Glu Val Val Glu Gln Ile Val Lys Val Ala
   85 90 95
   Glu Met Phe Gly Ala Asp Ala Ile Thr Thr Asn Asn Glu Leu Phe Ile
   100 105 110
   Ala Pro Met Ala Lys Ala Cys Glu Arg Leu Gly Leu Arg Gly Ala Gly
   115 120 125
   Val Gln Ala Ala Glu Asn Ala Arg Asp Lys Asn Lys Met Arg Asp Ala
   130 135 140
   Phe Asn Lys Ala Gly Val Lys Ser Ile Lys Asn Lys Arg Val Thr Thr
   145 150 155 160
   Leu Glu Asp Phe Arg Ala Ala Leu Glu Glu Ile Gly Thr Pro Leu Ile
   165 170 175
   Leu Lys Pro Thr Tyr Leu Ala Ser Ser Ile Gly Val Thr Leu Ile Thr
   180 185 190
   Asp Thr Glu Thr Ala Glu Asp Glu Phe Asn Arg Val Asn Asp Tyr Leu
   195 200 205
   Lys Ser Ile Asn Val Pro Lys Ala Val Thr Phe Glu Ala Pro Phe Ile
   210 215 220
   Ala Glu Glu Phe Leu Gln Gly Glu Tyr Gly Asp Trp Tyr Gln Thr Glu
   225 230 235 240
   Gly Tyr Ser Asp Tyr Ile Ser Ile Glu Gly Ile Met Ala Asp Gly Glu
   245 250 255
   Tyr Phe Pro Ile Ala Ile His Asp Lys Thr Pro Gln Ile Gly Phe Thr
   260 265 270
   Glu Thr Ser His Ile Thr Pro Ser Ile Leu Asp Glu Glu Ala Lys Lys
   275 280 285
   Lys Ile Val Glu Ala Ala Lys Lys Ala Asn Glu Gly Leu Gly Leu Gln
   290 295 300
   Asn Cys Ala Thr His Thr Glu Ile Lys Leu Met Lys Asn Arg Glu Pro
   305 310 315 320
   Gly Leu Ile Glu Ser Ala Ala Arg Phe Ala Gly Trp Asn Met Ile Pro
   325 330 335
   Asn Ile Lys Lys Val Phe Gly Leu Asp Met Ala Gln Leu Leu Leu Asp
   340 345 350
   Val Leu Cys Phe Gly Lys Asp Ala Asp Leu Pro Asp Gly Leu Leu Asp
   355 360 365
   Gln Glu Pro Tyr Tyr Val Ala Asp Cys His Leu Tyr Pro Gln His Phe
       370                 375                 380
   Lys Gln Asn Gly Gln Ile Pro Glu Thr Ala Glu Asp Leu Val Ile Glu
   385                 390                 395                 400
   Ala Ile Asp Leu Pro Asp Gly Leu Leu Lys Gly Asp Thr Glu Ile Val
                   405                 410                 415
   Ser Phe Ser Ala Ala Ala Pro Gly Thr Ser Val Asp Leu Thr Leu Phe
               420                 425                 430
   Glu Ala Phe Asn Ser Ile Ala Ala Phe Glu Leu Lys Gly Ser Asn Ser
           435                 440                 445
   Gln Asp Val Ala Glu Ser Ile Arg Gln Ile Gln Gln His Ala Lys Leu
       450                 455                 460
   Thr Ala Lys Tyr Val Leu Pro Val
   465                 470
<210> 22
   <211> 472
   <212> PRT
   <213> Bacillus subtilis IAM1107
<400> 22
   Met Glu Arg Lys Thr Val Leu Val Ile Ala Asp Leu Gly Gly Cys Pro
   1               5                   10                  15
   Pro His Met Phe Tyr Lys Ser Ala Ala Glu Lys Tyr Asn Leu Val Ser
               20                  25                  30
   Phe Ile Pro Arg Pro Phe Ala Ile Thr Ala Ser His Ala Ala Leu Ile
           35                  40                  45
   Glu Lys Tyr Ser Val Ala Val Val Lys Asp Lys Asp Tyr Phe Lys Ser
       50                  55                  60
   Leu Ala Asp Phe Glu His Pro Asp Ser Ile Tyr Trp Ala His Glu Asp
   65                  70                  75                  80
   His Asn Lys Pro Glu Glu Glu Val Val Glu Gln Ile Val Lys Val Ala
                   85                  90                  95
   Glu Met Phe Gly Ala Asp Ala Ile Thr Thr Asn Asn Glu Leu Phe Ile
               100                 105                 110
   Ala Pro Met Ala Lys Ala Cys Glu Arg Leu Gly Leu Arg Gly Ala Gly
           115                 120                 125
   Val Gln Ala Ala Glu Asn Ala Arg Asp Lys Asn Lys Met Arg Asp Ala
       130                 135                 140
   Phe Asn Lys Ala Gly Val Lys Ser Ile Lys Asn Lys Arg Val Thr Thr
   145                 150                 155                 160
   Leu Glu Asp Phe Arg Ala Ala Leu Glu Glu Ile Gly Thr Pro Leu Ile
                   165                 170                 175
   Leu Lys Pro Thr Tyr Leu Ala Ser Ser Ile Gly Val Thr Leu Ile Thr
               180                 185                 190
   Asp Thr Glu Thr Ala Glu Asp Glu Phe Asn Arg Val Asn Asp Tyr Leu
           195                 200                 205
   Lys Ser Ile Asn Val Pro Lys Ala Val Thr Phe Glu Ala Pro Phe Ile
   210 215 220
   Ala Glu Glu Phe Leu Gln Gly Glu Tyr Gly Asp Trp Tyr Gln Thr Glu
   225 230 235 240
   Gly Tyr Ser Asp Tyr Ile Ser Ile Glu Gly Ile Met Ala Asp Gly Glu
   245 250 255
   Tyr Phe Pro Ile Ala Ile His Asp Lys Thr Pro Gln Ile Gly Phe Thr
   260 265 270
   Glu Thr Ser His Ile Thr Pro Ser Ile Leu Asp Glu Glu Ala Lys Lys
   275 280 285
   Lys Ile Val Glu Ala Ala Lys Lys Ala Asn Glu Gly Leu Gly Leu Gln
   290 295 300
   Asn Cys Ala Thr His Thr Glu Val Lys Leu Met Lys Asn Arg Glu Pro
   305 310 315 320
   Gly Leu Ile Glu Ser Ala Ala Arg Phe Ala Gly Trp Asn Met Ile Pro
   325 330 335
   Asn Ile Lys Lys Val Phe Gly Leu Asp Met Ala Gln Leu Leu Leu Asp
   340 345 350
   Val Leu Cys Phe Gly Lys Asp Ala Asp Leu Pro Asp Gly Leu Leu Asp
   355 360 365
   Gln Glu Pro Tyr Tyr Val Ala Asp Cys His Leu Tyr Pro Gln His Phe
   370 375 380
   Lys Gln Asn Gly Gln Ile Pro Glu Thr Ala Glu Asp Leu Val Ile Glu
   385 390 395 400
   Ala Ile Asp Ile Pro Asp Gly Leu Leu Lys Gly Asp Thr Glu Ile Phe
   405 410 415
   Ser Phe Ser Ala Ala Ala Pro Gly Thr Ser Val Asp Leu Thr Leu Phe
   420 425 430
   Glu Ala Phe Asn Ser Ile Ala Ala Phe Glu Leu Lys Gly Ser Asn Ser
   435 440 445
   Gln Asp Val Ala Glu Ser Ile Arg Gln Ile Gln Gln His Ala Lys Leu
   450 455 460
   Thr Ala Lys Tyr Val Leu Pro Val
   465 470
<210> 23
   <211> 472
   <212> PRT
   <213> Bacillus subtilis IAM1214
<400> 23
   Met Glu Arg Lys Thr Val Leu Val Ile Ala Asp Leu Gly Gly Cys Pro
   1 5 10 15
   Pro His Met Phe Tyr Lys Ser Ala Ala Glu Lys Tyr Asn Leu Val Ser
   20 25 30
   Phe Ile Pro Arg Pro Phe Ala Ile Thr Ala Ser His Ala Ala Leu Ile
   35 40 45
   Glu Lys Tyr Ser Val Ala Val Ile Lys Asp Lys Asp Tyr Phe Gln Ser
   50 55 60
   Leu Ala Asp Phe Glu His Pro Asp Ser Ile Tyr Trp Ala His Glu Asp
   65 70 75 80
   His Asp Lys Pro Glu Glu Glu Val Val Glu Gln Ile Val Lys Val Ala
   85 90 95
   Gln Met Phe Glu Ala Asp Ala Ile Thr Thr Asn Asn Glu Leu Phe Ile
   100 105 110
   Ala Pro Met Ala Lys Ala Cys Glu Arg Leu Gly Leu Arg Gly Ala Gly
   115 120 125
   Val Gln Ala Ala Glu Asn Ala Arg Asp Lys Asn Lys Met Arg Asp Ala
   130 135 140
   Phe Asn Lys Ala Gly Val Lys Ser Ile Lys Asn Lys Arg Val Thr Thr
   145 150 155 160
   Leu Glu Asp Phe Arg Ala Ala Leu Glu Glu Ile Gly Thr Pro Leu Ile
   165 170 175
   Leu Lys Pro Thr Tyr Leu Ala Ser Ser Ile Gly Val Thr Leu Ile Thr
   180 185 190
   Asp Thr Glu Thr Ala Glu Asp Glu Phe Asn Arg Val Asn Asp Tyr Leu
   195 200 205
   Lys Ser Ile Asn Val Pro Lys Ala Val Thr Phe Glu Ala Pro Phe Ile
   210 215 220
   Ala Glu Glu Phe Leu Gln Gly Glu Tyr Gly Asp Trp Tyr Gln Thr Glu
   225 230 235 240
   Gly Tyr Ser Asp Tyr Ile Ser Ile Glu Gly Ile Met Ala Asp Gly Glu
   245 250 255
   Tyr Phe Pro Ile Ala Ile His Asp Lys Thr Pro Gln Ile Gly Phe Thr
   260 265 270
   Glu Thr Ser His Ile Thr Pro Ser Ile Leu Asp Glu Glu Ala Lys Lys
   275 280 285
   Lys Ile Val Glu Ala Ala Lys Lys Ala Asn Glu Gly Leu Gly Leu Gln
   290 295 300
   Asn Cys Ala Thr His Thr Glu Ile Lys Leu Met Lys Asn Arg Glu Pro
   305 310 315 320
   Gly Leu Ile Glu Ser Ala Ala Arg Phe Ala Gly Trp Asn Met Ile Pro
   325 330 335
   Asn Ile Lys Lys Val Phe Gly Leu Asp Met Ala Gln Leu Leu Leu Asp
   340 345 350
   Val Leu Cys Phe Gly Lys Asp Ala Asp Leu Pro Asp Gly Leu Leu Asp
   355 360 365
   Gln Glu Pro Tyr Tyr Val Ala Asp Cys His Leu Tyr Pro Gln His Phe
   370 375 380
   Lys Gln Asn Gly Gln Ile Pro Glu Thr Ala Glu Asp Leu Val Ile Glu
   385 390 395 400
   Ala Ile Asp Ile Pro Asp Gly Leu Leu Lys Gly Asp Thr Glu Ile Val
   405 410 415
   Thr Phe Ser Ala Ala Ala Pro Gly Thr Ser Val Asp Leu Thr Leu Phe
   420 425 430
   Glu Ala Phe Asn Ser Ile Ala Ala Phe Glu Leu Lys Gly Ser Asn Ser
   435 440 445
   Gln Asp Val Ala Glu Ser Ile Arg Gln Ile Gln Gln His Ala Lys Leu
   450 455 460
   Thr Ala Lys Tyr Val Leu Pro Val
   465 470
<210> 24
   <211> 472
   <212> PRT
   <213> Bacillus subtilis ATCC21555
<400> 24
   Met Glu Arg Lys Thr Val Leu Val Ile Ala Asp Leu Gly Gly Cys Pro
   1               5                   10                  15
   Pro His Met Phe Tyr Lys Ser Ala Ala Glu Lys Tyr Asn Leu Val Ser
               20                  25                  30
   Phe Ile Pro Arg Pro Phe Ala Ile Thr Ala Ser His Ala Ala Leu Ile
           35                  40                  45
   Glu Lys Tyr Ser Ile Ala Val Ile Lys Asp Lys Asp Tyr Phe Lys Ser
       50                  55                  60
   Leu Ala Asp Phe Glu His Pro Asp Ser Ile Tyr Trp Ala His Glu Asp
   65                  70                  75                  80
   His Asp Lys Pro Glu Glu Glu Val Val Glu Glu Ile Val Lys Val Ala
                   85                  90                  95
   Asp Met Phe Gly Val Asp Ala Ile Thr Thr Asn Asn Glu Leu Phe Ile
               100                 105                 110
   Ala Pro Met Ala Lys Ala Cys Lys Arg Leu Gly Leu Arg Gly Ala Gly
           115                 120                 125
   Val Gln Ala Ala Glu Asn Ala Arg Asp Lys Asn Lys Met Arg Ala Ala
       130                 135                 140
   Phe Asn Arg Ala Gly Val Lys Ser Ile Lys Asn Lys Arg Val Thr Thr
   145                 150                 155                 160
   Leu Glu Asp Phe Arg Ala Ala Leu Gln Glu Ile Gly Thr Pro Leu Ile
                   165                 170                 175
   Leu Lys Pro Thr Tyr Leu Ala Ser Ser Ile Gly Val Thr Leu Ile Lys
               180                 185                 190
   Glu Met Glu Thr Ala Glu Ala Glu Phe Asn Arg Val Asn Glu Tyr Leu
           195                 200                 205
   Lys Ser Ile Asn Val Pro Lys Ala Val Thr Phe Glu Ala Pro Phe Ile
       210                 215                 220
   Ala Glu Glu Phe Leu Gln Gly Glu Tyr Asp Asp Trp Tyr Glu Thr Ser
   225                 230                 235                 240
   Gly Tyr Ser Asp Tyr Ile Ser Ile Glu Gly Ile Met Ala Asp Gly Glu
                   245                 250                 255
   Tyr Phe Pro Val Ala Ile His Asp Lys Thr Pro Gln Ile Gly Phe Thr
               260                 265                 270
   Glu Thr Ala His Ile Thr Pro Ser Ile Leu Asp Asp Asp Ala Lys Arg
   275 280 285
   Lys Ile Val Glu Ala Ala Lys Lys Ala Asn Glu Gly Leu Gly Leu Glu
   290 295 300
   Asn Cys Ala Thr His Thr Glu Ile Lys Leu Met Lys Asn Arg Glu Ala
   305 310 315 320
   Gly Leu Ile Glu Ser Ala Ala Arg Phe Ala Gly Trp Asn Met Ile Pro
   325 330 335
   Asn Ile Lys Lys Val Phe Gly Val Asp Met Ala Gln Leu Leu Leu Asp
   340 345 350
   Val Leu Cys Tyr Gly Lys Glu Ala Asp Leu Pro Lys Gly Leu Leu Glu
   355 360 365
   Gln Glu Pro Cys Tyr Val Ala Asp Cys His Leu Tyr Pro Gln His Phe
   370 375 380
   Lys Glu Asn Gly Gln Leu Pro Glu Thr Val Val Asp Phe Val Ile Glu
   385 390 395 400
   Ser Ile Glu Ile Pro Asp Gly Val Leu Lys Gly Asp Thr Glu Leu Val
   405 410 415
   Ser Phe Ser Ala Ala Glu Ala Gly Thr Ser Val Asp Leu Arg Leu Phe
   420 425 430
   Glu Ala Phe Asn Ser Ile Ala Ala Phe Glu Leu Lys Gly Ser Asn Ser
   435 440 445
   Asn Asp Val Ala Glu Ser Ile Lys Gln Ile Gln Gln Gln Ala Lys Leu
   450 455 460
   Thr Ala Lys Tyr Ala Leu Ser Val
   465 470
<210> 25
   <211> 472
   <212> PRT
   <213> Bacillus amyloliquefaciens IFO3022
<400> 25
   Met Glu Arg Lys Thr Val Leu Val Ile Ala Asp Leu Gly Gly Cys Pro
   1 5 10 15
   Pro His Met Phe Tyr Lys Ser Ala Ala Glu Lys Tyr Asn Leu Val Ser
   20 25 30
   Phe Ile Pro Arg Pro Phe Ala Ile Thr Ala Ser His Ala Ala Leu Ile
   35 40 45
   Glu Lys Tyr Ser Val Ala Val Ile Lys Asp Lys Asp Tyr Phe Lys Ser
   50 55 60
   Leu Ala Asp Phe Glu His Pro Asp Ser Ile Tyr Trp Ala His Glu Asp
   65 70 75 80
   His Asp Lys Pro Glu Glu Glu Val Val Glu Glu Ile Val Lys Val Ala
   85 90 95
   Gly Met Phe Ala Val Asp Ala Ile Thr Thr Asn Asn Glu Leu Phe Ile
   100 105 110
   Ala Pro Met Ala Lys Ala Cys Glu Arg Leu Gly Leu Arg Gly Ala Gly
   115 120 125
   Val Gln Ala Ala Glu Asn Ala Arg Asp Lys Asn Lys Met Arg Ala Ala
   130 135 140
   Phe Asn Arg Ala Gly Val Lys Ser Ile Lys Asn Arg Arg Val Thr Thr
   145 150 155 160
   Leu Glu Asp Phe Arg Ala Ala Leu Gln Glu Ile Gly Thr Pro Leu Ile
   165 170 175
   Leu Lys Pro Thr Tyr Leu Ala Ser Ser Ile Gly Val Thr Leu Ile Lys
   180 185 190
   Glu Arg Glu Thr Ala Glu Ala Glu Phe Asn Arg Val Asn Glu Tyr Leu
   195 200 205
   Lys Ser Ile Asn Val Pro Lys Ala Val Thr Phe Glu Ala Pro Phe Ile
   210 215 220
   Ala Glu Glu Phe Leu Gln Gly Glu Tyr Asp Asp Trp Tyr Glu Thr Ser
   225 230 235 240
   Gly Tyr Ser Asp Tyr Ile Ser Ile Glu Gly Ile Met Ala Asp Gly Glu
   245 250 255
   Tyr Phe Pro Val Ala Ile His Asp Lys Thr Pro Gln Ile Gly Phe Thr
   260 265 270
   Glu Thr Ser His Ile Thr Pro Ser Ile Leu Asp Asp Asp Ala Lys Arg
   275 280 285
   Lys Ile Val Glu Ala Ala Lys Lys Ala Asn Glu Gly Leu Gly Leu Glu
   290 295 300
   Asn Cys Ala Thr His Thr Glu Ile Lys Leu Met Lys Asn Arg Glu Ala
   305 310 315 320
   Gly Leu Ile Glu Ser Ala Ala Arg Phe Ala Gly Trp Asn Met Ile Pro
   325 330 335
   Asn Ile Lys Lys Val Phe Gly Val Asp Met Ala Gln Leu Leu Leu Asp
   340 345 350
   Val Leu Cys Phe Gly Lys Glu Ala Asp Leu Pro Lys Gly Leu Leu Glu
   355 360 365
   Gln Glu Pro Cys Tyr Val Ala Asp Cys His Leu Tyr Pro Gln His Phe
   370 375 380
   Lys Glu Asn Gly Gln Leu Pro Glu Thr Ala Val Asp Phe Val Ile Glu
   385 390 395 400
   Ser Ile Asp Ile Pro Asp Gly Val Leu Lys Gly Asp Thr Glu Ile Val
   405 410 415
   Ser Phe Ser Ala Ala Glu Ala Gly Thr Ser Val Asp Leu Arg Leu Phe
   420 425 430
   Glu Ala Phe Asn Ser Ile Ala Ala Phe Glu Leu Lys Gly Ser Asn Ser
   435 440 445
   Gly Asp Val Ala Glu Ser Ile Lys Gln Ile Gln Gln Gln Ala Lys Leu
   450 455 460
   Thr Ala Lys Tyr Ala Leu Pro Val
   465 470
<210> 26
   <211> 1416
   <212> DNA
   <213> Bacillus subtilis 168
<400> 26
   atg gag aga aaa aca gta ttg gtc atc gct gat ctt gga ggc tgc ccg      48
   Met Glu Arg Lys Thr Val Leu Val Ile Ala Asp Leu Gly Gly Cys Pro
   1               5                   10                  15
   ccg cac atg ttt tat aaa agc gct gct gaa aaa tat aac ctg gtc agc      96
   Pro His Met Phe Tyr Lys Ser Ala Ala Glu Lys Tyr Asn Leu Val Ser
               20                  25                  30
   ttt att cca aga cct ttt gca att aca gcc tcc cat gca gca ttg att      144
   Phe Ile Pro Arg Pro Phe Ala Ile Thr Ala Ser His Ala Ala Leu Ile
           35                  40                  45
   gaa aaa tac tcg gtc gcg gtc ata aaa gat aaa gac tat ttt aag agt      192
   Glu Lys Tyr Ser Val Ala Val Ile Lys Asp Lys Asp Tyr Phe Lys Ser
       50                  55                  60
   tta gct gat ttt gaa cac cct gat tcc att tat tgg gcg cat gaa gat      240
   Leu Ala Asp Phe Glu His Pro Asp Ser Ile Tyr Trp Ala His Glu Asp
   65                  70                  75                  80
   cat aac aag cct gag gaa gag gtc gtc gag caa atc gtc aag gtt gcc      288
   His Asn Lys Pro Glu Glu Glu Val Val Glu Gln Ile Val Lys Val Ala
                   85                  90                  95
   gaa atg ttt ggg gcg gat gcc atc aca aca aac aat gaa tta ttc att      336
   Glu Met Phe Gly Ala Asp Ala Ile Thr Thr Asn Asn Glu Leu Phe Ile
               100                 105                 110
   gct ccg atg gcg aaa gcc tgt gaa cgt ctg ggc ttg aga ggt gcc ggc      384
   Ala Pro Met Ala Lys Ala Cys Glu Arg Leu Gly Leu Arg Gly Ala Gly
           115                 120                 125
   gtg cag gca gcc gaa aat gcc aga gat aaa aat aaa atg agg gac gct      432
   Val Gln Ala Ala Glu Asn Ala Arg Asp Lys Asn Lys Met Arg Asp Ala
       130                 135                 140
   ttt aat aag gcc gga gtc aaa tcg atc aaa aac aaa cga gtc aca act      480
   Phe Asn Lys Ala Gly Val Lys Ser Ile Lys Asn Lys Arg Val Thr Thr
   145                 150                 155                 160
   ctt gaa gat ttc cgt gct gct ctt gaa gag atc ggc aca cct ctt atc      528
   Leu Glu Asp Phe Arg Ala Ala Leu Glu Glu Ile Gly Thr Pro Leu Ile
                   165                 170                 175
   tta aag cct aca tac tta gcg agt tct atc ggt gta acg ctg att acg      576
   Leu Lys Pro Thr Tyr Leu Ala Ser Ser Ile Gly Val Thr Leu Ile Thr
               180                 185                 190
   gac act gag acg gca gaa gat gaa ttt aac aga gtc aat gac tat ctg      624
   Asp Thr Glu Thr Ala Glu Asp Glu Phe Asn Arg Val Asn Asp Tyr Leu
           195                 200                 205
   aaa tca att aac gtg cca aag gcg gtt acg ttt gaa gcg ccg ttt atc      672
   Lys Ser Ile Asn Val Pro Lys Ala Val Thr Phe Glu Ala Pro Phe Ile
       210                 215                 220
   gct gaa gaa ttt tta cag ggt gag tac gga gac tgg tat caa aca gaa      720
   Ala Glu Glu Phe Leu Gln Gly Glu Tyr Gly Asp Trp Tyr Gln Thr Glu
   225                 230                 235                 240
   ggg tac tcc gac tat atc agt ata gaa ggc atc atg gct gac ggt gag      768
   Gly Tyr Ser Asp Tyr Ile Ser Ile Glu Gly Ile Met Ala Asp Gly Glu
                   245                 250                 255
   tat ttc ccg atc gcc att cat gat aaa acg ccg caa atc ggg ttt aca      816
   Tyr Phe Pro Ile Ala Ile His Asp Lys Thr Pro Gln Ile Gly Phe Thr
               260                 265                 270
   gag aca tcc cac att acg ccg tcc att ctg gat gaa gag gca aaa aag      864
   Glu Thr Ser His Ile Thr Pro Ser Ile Leu Asp Glu Glu Ala Lys Lys
           275                 280                 285
   aaa att gtc gaa gct gcc aaa aag gca aat gaa ggg ctt gga ctg caa      912
   Lys Ile Val Glu Ala Ala Lys Lys Ala Asn Glu Gly Leu Gly Leu Gln
       290                 295                 300
   aat tgc gca aca cat aca gag atc aag cta atg aaa aac aga gaa ccg      960
   Asn Cys Ala Thr His Thr Glu Ile Lys Leu Met Lys Asn Arg Glu Pro
   305                 310                 315                 320
   ggt tta ata gag tcg gca gcc aga ttt gcc ggc tgg aat atg atc ccc     1008
   Gly Leu Ile Glu Ser Ala Ala Arg Phe Ala Gly Trp Asn Met Ile Pro
                   325                 330                 335
   aat att aaa aag gtc ttt ggc ctt gat atg gcg caa tta tta tta gat     1056
   Asn Ile Lys Lys Val Phe Gly Leu Asp Met Ala Gln Leu Leu Leu Asp
               340                 345                 350
   gtc ctc tgt ttc gga aaa gac gcc gat ctg ccg gac gga tta ttg gat     1104
   Val Leu Cys Phe Gly Lys Asp Ala Asp Leu Pro Asp Gly Leu Leu Asp
           355                 360                 365
   caa gag cct tat tat gtt gcc gac tgc cat ttg tac ccg cag cat ttc     1152
   Gln Glu Pro Tyr Tyr Val Ala Asp Cys His Leu Tyr Pro Gln His Phe
       370                 375                 380
   aaa caa aat ggc caa att cct gaa act gct gag gat ttg gtc att gaa     1200
   Lys Gln Asn Gly Gln Ile Pro Glu Thr Ala Glu Asp Leu Val Ile Glu
   385                 390                 395                 400
   gcg atc gat att ccg gac ggg ctt tta aaa ggg gat act gaa atc gtt     1248
   Ala Ile Asp Ile Pro Asp Gly Leu Leu Lys Gly Asp Thr Glu Ile Val
                   405                 410                 415
   tct ttt tcg gcc gca gca cca ggc act tca gtt gat ttg aca ttg ttt     1296
   Ser Phe Ser Ala Ala Ala Pro Gly Thr Ser Val Asp Leu Thr Leu Phe
               420                 425                 430
   gaa gct ttc aat tcc att gct gca ttt gaa ctg aaa ggc agt aat tca     1344
   Glu Ala Phe Asn Ser Ile Ala Ala Phe Glu Leu Lys Gly Ser Asn Ser
           435                 440                 445
   cag gat gtg gct gaa tca atc aga caa att cag cag cat gcg aag ctg     1392
   Gln Asp Val Ala Glu Ser Ile Arg Gln Ile Gln Gln His Ala Lys Leu
       450                 455                 460
   acg gca aag tat gtg ctg cca gta                                     1416
   Thr Ala Lys Tyr Val Leu Pro Val
   465                 470
<210> 27
   <211> 1416
   <212> DNA
   <213> Bacillus subtilis ATCC6633
<400> 27
   atg gag aga aaa aca gta ttg gtc atc gct gat ctt gga ggc tgc ccg 48
   Met Glu Arg Lys Thr Val Leu Val Ile Ala Asp Leu Gly Gly Cys Pro
   1 5 10 15
   ccg cac atg ttt tat aaa agc gct gct gaa aaa tat aac ctg gtt agc 96
   Pro His Met Phe Tyr Lys Ser Ala Ala Glu Lys Tyr Asn Leu Val Ser
   20 25 30
   ttt att ccg aga cct ttt gca ata aca gcc tcc cat gca gca ctg att 144
   Phe Ile Pro Arg Pro Phe Ala Ile Thr Ala Ser His Ala Ala Leu Ile
   35 40 45
   gaa aaa tac tcg gtc gcg gtc ata aaa gat aaa gac tat ttt cag agc 192
   Glu Lys Tyr Ser Val Ala Val Ile Lys Asp Lys Asp Tyr Phe Gln Ser
   50 55 60
   tta gct gat ttt gag cat ccc gat tca att tat tgg gcg cat gag gat 240
   Leu Ala Asp Phe Glu His Pro Asp Ser Ile Tyr Trp Ala His Glu Asp
   65 70 75 80
   cat gac aag cct gaa gaa gag gtt gtc gag caa atc gtc aag gtt gcc 288
   His Asp Lys Pro Glu Glu Glu Val Val Glu Gln Ile Val Lys Val Ala
   85 90 95
   caa atg ttt gag gcg gac gcc atc aca aca aac aat gaa tta ttc att 336
   Gln Met Phe Glu Ala Asp Ala Ile Thr Thr Asn Asn Glu Leu Phe Ile
   100 105 110
   gcc ccg atg gcg aaa gcc tgt gaa cgc ctt ggc ctg agg ggc gcc gga 384
   Ala Pro Met Ala Lys Ala Cys Glu Arg Leu Gly Leu Arg Gly Ala Gly
   115 120 125
   gtg cag gca gcg gaa aat gcc aga gat aaa aat aaa atg agg gac gct 432
   Val Gln Ala Ala Glu Asn Ala Arg Asp Lys Asn Lys Met Arg Asp Ala
   130 135 140
   ttt aat aag gcg gga gtc aaa tcg atc aaa aac aaa cga gtc aca act 480
   Phe Asn Lys Ala Gly Val Lys Ser Ile Lys Asn Lys Arg Val Thr Thr
   145 150 155 160
   ctt gag gat ttt cgt gct gca ctt gaa gag atc ggc aca cct cta atc 528
   Leu Glu Asp Phe Arg Ala Ala Leu Glu Glu Ile Gly Thr Pro Leu Ile
   165 170 175
   tta aag cct aca tac tta gcg agt tca atc ggc gta acg ctg att acc 576
   Leu Lys Pro Thr Tyr Leu Ala Ser Ser Ile Gly Val Thr Leu Ile Thr
   180 185 190
   gac acg gag acg gca gaa gat gaa ttt aac aga gtc aat gac tac ctg 624
   Asp Thr Glu Thr Ala Glu Asp Glu Phe Asn Arg Val Asn Asp Tyr Leu
   195 200 205
   aaa tcg att aac gtg ccg aag gcg gtc aca ttt gaa gca ccg ttt att 672
   Lys Ser Ile Asn Val Pro Lys Ala Val Thr Phe Glu Ala Pro Phe Ile
   210 215 220
   gct gag gaa ttt tta cag ggt gag tac gga gac tgg tat caa aca gaa 720
   Ala Glu Glu Phe Leu Gln Gly Glu Tyr Gly Asp Trp Tyr Gln Thr Glu
   225 230 235 240
   ggg tac tcc gac tat atc agc ata gaa ggc att atg gca gat ggt gag 768
   Gly Tyr Ser Asp Tyr Ile Ser Ile Glu Gly Ile Met Ala Asp Gly Glu
   245 250 255
   tat ttt ccg atc gcc att cat gac aaa acg ccg caa att gga ttt aca 816
   Tyr Phe Pro Ile Ala Ile His Asp Lys Thr Pro Gln Ile Gly Phe Thr
   260 265 270
   gag aca tca cat att acg cca tcc att ctg gat gaa gag gcg aaa aag 864
   Glu Thr Ser His Ile Thr Pro Ser Ile Leu Asp Glu Glu Ala Lys Lys
           275                 280                 285
   aaa att gtc gaa gcg gct aaa aag gca aat gaa ggg ctt gga ctg caa      912
   Lys Ile Val Glu Ala Ala Lys Lys Ala Asn Glu Gly Leu Gly Leu Gln
       290                 295                 300
   aat tgc gca aca cat aca gaa atc aag cta atg aaa aac aga gaa ccg      960
   Asn Cys Ala Thr His Thr Glu Ile Lys Leu Met Lys Asn Arg Glu Pro
   305                 310                 315                 320
   ggt tta ata gag tcg gct gcc aga ttc gca ggc tgg aat atg att cct     1008
   Gly Leu Ile Glu Ser Ala Ala Arg Phe Ala Gly Trp Asn Met Ile Pro
                   325                 330                 335
   aac att aaa aag gtt ttc ggc ctt gat atg gcg caa tta tta tta gat     1056
   Asn Ile Lys Lys Val Phe Gly Leu Asp Met Ala Gln Leu Leu Leu Asp
               340                 345                 350
   gtt ctc tgt ttc gga aaa gat gct gat ctg ccg gac ggg tta ttg gat     1104
   Val Leu Cys Phe Gly Lys Asp Ala Asp Leu Pro Asp Gly Leu Leu Asp
           355                 360                 365
   caa gag cct tac tat gtt gct gac tgc cat ctg tac cct cag cat ttc     1152
   Gln Glu Pro Tyr Tyr Val Ala Asp Cys His Leu Tyr Pro Gln His Phe
       370                 375                 380
   aaa caa aat ggc cag atc cct gaa act gcc gag gat ttg gta atc gaa     1200
   Lys Gln Asn Gly Gln Ile Pro Glu Thr Ala Glu Asp Leu Val Ile Glu
   385                 390                 395                 400
   gcg atc gat att ccg gat ggg ctt ttg aag ggt gat aca gaa atc gtt     1248
   Ala Ile Asp Ile Pro Asp Gly Leu Leu Lys Gly Asp Thr Glu Ile Val
                   405                 410                 415
   act ttt tcg gct gcg gca cca gga aca tca gtt gat ttg aca ctg ttt     1296
   Thr Phe Ser Ala Ala Ala Pro Gly Thr Ser Val Asp Leu Thr Leu Phe
               420                 425                 430
   gaa gcc ttc aac tcc att gct gca ttt gaa ctg aaa ggc agc aat tca     1344
   Glu Ala Phe Asn Ser Ile Ala Ala Phe Glu Leu Lys Gly Ser Asn Ser
           435                 440                 445
   cag gat gtg gct gaa tca atc aga caa att cag cag cat gcg aag ctg     1392
   Gln Asp Val Ala Glu Ser Ile Arg Gln Ile Gln Gln His Ala Lys Leu
       450                 455                 460
   acg gca aag tat gtg ctg cca gta                                     1416
   Thr Ala Lys Tyr Val Leu Pro Val
   465                 470
<210> 28
   <211> 1416
   <212> DNA
   <213> Bacillus subtilis IAM1213
<400> 28
   atg gag aga aaa aca gta ttg gtc atc gct gat ctt gga ggc tgc ccg    48
   Met Glu Arg Lys Thr Val Leu Val Ile Ala Asp Leu Gly Gly Cys Pro
    1               5                  10                  15
   ccg cac atg ttt tat aaa agc gct gct gaa aaa tat aac ctg gtc agc    96
   Pro His Met Phe Tyr Lys Ser Ala Ala Glu Lys Tyr Asn Leu Val Ser
               20                  25                  30
   ttt att cca aga cct ttt gca att aca gcc tcc cat gca gca ttg att   144
   Phe Ile Pro Arg Pro Phe Ala Ile Thr Ala Ser His Ala Ala Leu Ile
   35 40 45
   gaa aaa tac tcg gtc gcg gtc ata aaa gat aaa gac tat ttt aag agt 192
   Glu Lys Tyr Ser Val Ala Val Ile Lys Asp Lys Asp Tyr Phe Lys Ser
   50 55 60
   tta gct gat ttt gag cat cct gac tcc att tat tgg gcg cat gag gat 240
   Leu Ala Asp Phe Glu His Pro Asp Ser Ile Tyr Trp Ala His Glu Asp
   65 70 75 80
   cat aac aag cct gag gaa gag gtc gtc gag caa atc gtc aag gtt gcc 288
   His Asn Lys Pro Glu Glu Glu Val Val Glu Gln Ile Val Lys Val Ala
   85 90 95
   gaa atg ttt ggg gcg gat gcc atc aca aca aac aat gaa tta ttc att 336
   Glu Met Phe Gly Ala Asp Ala Ile Thr Thr Asn Asn Glu Leu Phe Ile
   100 105 110
   gct ccg atg gcg aaa gcc tgt gaa cgt ctg ggc ctg aga ggt gcc ggc 384
   Ala Pro Met Ala Lys Ala Cys Glu Arg Leu Gly Leu Arg Gly Ala Gly
   115 120 125
   gtg cag gca gcc gaa aat gcc aga gat aaa aat aaa atg agg gac gct 432
   Val Gln Ala Ala Glu Asn Ala Arg Asp Lys Asn Lys Met Arg Asp Ala
   130 135 140
   ttt aat aag gcc gga gtc aaa tcg atc aaa aac aaa cga gtc aca act 480
   Phe Asn Lys Ala Gly Val Lys Ser Ile Lys Asn Lys Arg Val Thr Thr
   145 150 155 160
   ctc gaa gat ttc cgt gct gct ctt gaa gag atc ggc aca cct ctt atc 528
   Leu Glu Asp Phe Arg Ala Ala Leu Glu Glu Ile Gly Thr Pro Leu Ile
   165 170 175
   tta aag cct aca tac tta gcg agt tca atc ggt gta acg ctg att acg 576
   Leu Lys Pro Thr Tyr Leu Ala Ser Ser Ile Gly Val Thr Leu Ile Thr
   180 185 190
   gac act gag acg gca gaa gat gaa ttt aac aga gtc aat gac tat ctg 624
   Asp Thr Glu Thr Ala Glu Asp Glu Phe Asn Arg Val Asn Asp Tyr Leu
   195 200 205
   aaa tca att aac gtg cca aag gcg gtt acg ttt gaa gcg ccg ttt atc 672
   Lys Ser Ile Asn Val Pro Lys Ala Val Thr Phe Glu Ala Pro Phe Ile
   210 215 220
   gct gaa gaa ttt tta cag ggt gag tac gga gac tgg tat caa aca gaa 720
   Ala Glu Glu Phe Leu Gln Gly Glu Tyr Gly Asp Trp Tyr Gln Thr Glu
   225 230 235 240
   ggg tac tcc gac tat atc agt ata gaa ggc atc atg gct gac ggt gag 768
   Gly Tyr Ser Asp Tyr Ile Ser Ile Glu Gly Ile Met Ala Asp Gly Glu
   245 250 255
   tat ttc ccg atc gcc att cat gat aaa acg ccg caa atc ggg ttt aca 816
   Tyr Phe Pro Ile Ala Ile His Asp Lys Thr Pro Gln Ile Gly Phe Thr
   260 265 270
   gag aca tcc cac att acg ccg tcc att ctg gat gaa gag gca aaa aag 864
   Glu Thr Ser His Ile Thr Pro Ser Ile Leu Asp Glu Glu Ala Lys Lys
   275 280 285
   aaa att gtc gaa gct gcc aaa aag gca aat gaa ggt ctt ggc ctg caa 912
   Lys Ile Val Glu Ala Ala Lys Lys Ala Asn Glu Gly Leu Gly Leu Gln
   290 295 300
   aat tgc gca aca cat aca gag atc aag cta atg aaa aat aga gaa ccg 960
   Asn Cys Ala Thr His Thr Glu Ile Lys Leu Met Lys Asn Arg Glu Pro
   305                 310                 315                 320
   ggt tta att gaa tcg gca gcc aga ttc gcc ggc tgg aat atg atc ccc     1008
   Gly Leu Ile Glu Ser Ala Ala Arg Phe Ala Gly Trp Asn Met Ile Pro
                   325                 330                 335
   aat att aaa aag gtc ttt ggc ctt gat atg gcg caa tta tta tta gat     1056
   Asn Ile Lys Lys Val Phe Gly Leu Asp Met Ala Gln Leu Leu Leu Asp
               340                 345                 350
   gtc ctt tgt ttc gga aaa gac gcc gat ctg ccg gac gga tta ttg gat     1104
   Val Leu Cys Phe Gly Lys Asp Ala Asp Leu Pro Asp Gly Leu Leu Asp
           355                 360                 365
   caa gag cct tat tat gtt gcc gac tgc cat ttg tac ccg caa cat ttc     1152
   Gln Glu Pro Tyr Tyr Val Ala Asp Cys His Leu Tyr Pro Gln His Phe
       370                 375                 380
   aaa caa aat ggc cag att cca gaa act gct gag gat ttg gtc att gaa     1200
   Lys Gln Asn Gly Gln Ile Pro Glu Thr Ala Glu Asp Leu Val Ile Glu
   385                 390                 395                 400
   gcg atc gat ctg cct gac ggg ctt tta aaa ggg gat act gag atc gtt     1248
   Ala Ile Asp Leu Pro Asp Gly Leu Leu Lys Gly Asp Thr Glu Ile Val
                   405                 410                 415
   tct ttt tcg gcc gca gca cca gga act tca gtt gat ttg aca ttg ttt     1296
   Ser Phe Ser Ala Ala Ala Pro Gly Thr Ser Val Asp Leu Thr Leu Phe
               420                 425                 430
   gaa gct ttc aat tcc att gct gca ttt gaa ctg aaa ggc agt aat tca     1344
   Glu Ala Phe Asn Ser Ile Ala Ala Phe Glu Leu Lys Gly Ser Asn Ser
           435                 440                 445
   cag gat gtg gct gaa tca atc aga caa att cag cag cat gcg aag ctg     1392
   Gln Asp Val Ala Glu Ser Ile Arg Gln Ile Gln Gln His Ala Lys Leu
       450                 455                 460
   acg gca aag tat gtg ctg cca gta                                     1416
   Thr Ala Lys Tyr Val Leu Pro Val
   465                 470
<210> 29
   <211> 1416
   <212> DNA
   <213> Bacillus subtilis IAM1107
<400> 29
   atg gag aga aaa aca gta ttg gtc atc gct gat ctt gga ggc tgc ccg      48
   Met Glu Arg Lys Thr Val Leu Val Ile Ala Asp Leu Gly Gly Cys Pro
    1               5                  10                  15
   ccg cac atg ttt tat aaa agc gct gct gaa aaa tat aac ctg gtc agc      96
   Pro His Met Phe Tyr Lys Ser Ala Ala Glu Lys Tyr Asn Leu Val Ser
               20                  25                  30
   ttt att cca aga cct ttt gca att aca gcc tcc cat gca gca ttg att     144
   Phe Ile Pro Arg Pro Phe Ala Ile Thr Ala Ser His Ala Ala Leu Ile
           35                  40                  45
   gaa aaa tac tcg gtc gcg gtc gta aaa gat aaa gac tat ttt aag agt     192
   Glu Lys Tyr Ser Val Ala Val Val Lys Asp Lys Asp Tyr Phe Lys Ser
       50                  55                  60
   tta gct gat ttt gag cat cct gac tcc att tat tgg gcg cat gag gat     240
   Leu Ala Asp Phe Glu His Pro Asp Ser Ile Tyr Trp Ala His Glu Asp
   65                  70                  75                  80
   cat aac aag cct gag gaa gag gtc gtc gag caa atc gtc aag gtt gcc      288
   His Asn Lys Pro Glu Glu Glu Val Val Glu Gln Ile Val Lys Val Ala
                   85                  90                  95
   gaa atg ttc ggg gcg gat gcc atc aca aca aac aat gaa tta ttc att      336
   Glu Met Phe Gly Ala Asp Ala Ile Thr Thr Asn Asn Glu Leu Phe Ile
               100                 105                 110
   gct ccg atg gcg aaa gcc tgt gaa cgt ctg ggc ttg aga ggt gcc ggc      384
   Ala Pro Met Ala Lys Ala Cys Glu Arg Leu Gly Leu Arg Gly Ala Gly
           115                 120                 125
   gtg cag gca gcc gaa aat gcc aga gat aaa aat aaa atg agg gac gct      432
   Val Gln Ala Ala Glu Asn Ala Arg Asp Lys Asn Lys Met Arg Asp Ala
       130                 135                 140
   ttt aat aag gcc gga gtc aaa tcg atc aaa aac aaa cga gtc aca act      480
   Phe Asn Lys Ala Gly Val Lys Ser Ile Lys Asn Lys Arg Val Thr Thr
   145                 150                 155                 160
   ctt gaa gat ttc cgt gct gct ctt gaa gag atc ggc aca cct ctt atc      528
   Leu Glu Asp Phe Arg Ala Ala Leu Glu Glu Ile Gly Thr Pro Leu Ile
                   165                 170                 175
   tta aag cct aca tac tta gcg agt tct atc ggt gta acg ctg att acg      576
   Leu Lys Pro Thr Tyr Leu Ala Ser Ser Ile Gly Val Thr Leu Ile Thr
               180                 185                 190
   gac act gag acg gca gaa gat gaa ttt aac aga gtc aat gac tat ctg      624
   Asp Thr Glu Thr Ala Glu Asp Glu Phe Asn Arg Val Asn Asp Tyr Leu
           195                 200                 205
   aaa tca att aac gtg cca aag gcg gtt acg ttt gaa gcg ccg ttt atc      672
   Lys Ser Ile Asn Val Pro Lys Ala Val Thr Phe Glu Ala Pro Phe Ile
       210                 215                 220
   gct gaa gaa ttt tta cag ggt gag tac gga gac tgg tat caa aca gaa      720
   Ala Glu Glu Phe Leu Gln Gly Glu Tyr Gly Asp Trp Tyr Gln Thr Glu
   225                 230                 235                 240
   ggg tac tcc gac tat atc agt ata gaa ggc atc atg gct gac ggt gag      768
   Gly Tyr Ser Asp Tyr Ile Ser Ile Glu Gly Ile Met Ala Asp Gly Glu
                   245                 250                 255
   tat ttc ccg atc gcc att cat gat aaa acg ccg caa atc ggg ttt aca      816
   Tyr Phe Pro Ile Ala Ile His Asp Lys Thr Pro Gln Ile Gly Phe Thr
               260                 265                 270
   gag aca tcc cac att acg ccg tcc att ctg gat gaa gag gca aaa aag      864
   Glu Thr Ser His Ile Thr Pro Ser Ile Leu Asp Glu Glu Ala Lys Lys
           275                 280                 285
   aaa att gtc gaa gct gcc aaa aag gca aat gaa ggg ctt ggc ctg caa      912
   Lys Ile Val Glu Ala Ala Lys Lys Ala Asn Glu Gly Leu Gly Leu Gln
       290                 295                 300
   aat tgc gca aca cat aca gag gtc aag cta atg aaa aac aga gaa ccg      960
   Asn Cys Ala Thr His Thr Glu Val Lys Leu Met Lys Asn Arg Glu Pro
   305                 310                 315                 320
   ggt tta att gaa tcg gca gcc aga ttt gcc ggc tgg aat atg atc cct     1008
   Gly Leu Ile Glu Ser Ala Ala Arg Phe Ala Gly Trp Asn Met Ile Pro
                   325                 330                 335
   aac att aaa aag gtt ttc ggc ctt gat atg gcg caa tta tta tta gat     1056
   Asn Ile Lys Lys Val Phe Gly Leu Asp Met Ala Gln Leu Leu Leu Asp
               340                 345                 350
   gtc ctc tgt ttc gga aaa gat gcc gat ctg ccg gac gga tta ttg gat     1104
   Val Leu Cys Phe Gly Lys Asp Ala Asp Leu Pro Asp Gly Leu Leu Asp
           355                 360                 365
   caa gag cct tac tat gtc gcc gac tgc cat ttg tac ccg cag cat ttc     1152
   Gln Glu Pro Tyr Tyr Val Ala Asp Cys His Leu Tyr Pro Gln His Phe
       370                 375                 380
   aaa caa aat ggc cag att cca gaa acc gct gag gat ttg gtc att gaa     1200
   Lys Gln Asn Gly Gln Ile Pro Glu Thr Ala Glu Asp Leu Val Ile Glu
   385                 390                 395                 400
   gcg atc gat att ccg gac ggg ctt tta aaa ggg gat act gaa atc ttt     1248
   Ala Ile Asp Ile Pro Asp Gly Leu Leu Lys Gly Asp Thr Glu Ile Phe
                   405                 410                 415
   tct ttt tcg gcc gca gca cca ggc act tca gtt gat ttg aca ttg ttt     1296
   Ser Phe Ser Ala Ala Ala Pro Gly Thr Ser Val Asp Leu Thr Leu Phe
               420                 425                 430
   gaa gct ttc aat tcc att gct gca ttt gaa ctg aaa ggc agt aat tca     1344
   Glu Ala Phe Asn Ser Ile Ala Ala Phe Glu Leu Lys Gly Ser Asn Ser
           435                 440                 445
   cag gat gtg gct gaa tca atc aga caa att cag cag cat gcg aag ctg     1392
   Gln Asp Val Ala Glu Ser Ile Arg Gln Ile Gln Gln His Ala Lys Leu
       450                 455                 460
   acg gca aag tat gtg ctg cca gta                                     1416
   Thr Ala Lys Tyr Val Leu Pro Val
   465                 470
<210> 30
   <211> 1416
   <212> DNA
   <213> Bacillus subtilis IAM1214
<400> 30
   atg gag aga aaa aca gta ttg gtc atc gct gat ctt gga ggc tgc ccg      48
   Met Glu Arg Lys Thr Val Leu Val Ile Ala Asp Leu Gly Gly Cys Pro
    1               5                  10                  15
   ccg cac atg ttt tat aaa agc gct gct gaa aaa tat aac ctg gtt agc      96
   Pro His Met Phe Tyr Lys Ser Ala Ala Glu Lys Tyr Asn Leu Val Ser
               20                  25                  30
   ttt att ccg aga cct ttt gca ata aca gcc tcc cat gca gca ctg att     144
   Phe Ile Pro Arg Pro Phe Ala Ile Thr Ala Ser His Ala Ala Leu Ile
           35                  40                  45
   gaa aaa tac tcg gtc gcg gtc ata aaa gat aaa gac tat ttt cag agc     192
   Glu Lys Tyr Ser Val Ala Val Ile Lys Asp Lys Asp Tyr Phe Gln Ser
       50                  55                  60
   tta gct gat ttt gag cat ccc gat tca att tat tgg gcg cat gag gat     240
   Leu Ala Asp Phe Glu His Pro Asp Ser Ile Tyr Trp Ala His Glu Asp
    65                  70                  75                  80
   cat gac aag cct gaa gaa gag gtt gtc gag caa atc gtc aag gtt gcc     288
   His Asp Lys Pro Glu Glu Glu Val Val Glu Gln Ile Val Lys Val Ala
                   85                  90                  95
   caa atg ttt gag gcg gac gcc atc aca aca aac aat gaa tta ttc att     336
   Gln Met Phe Glu Ala Asp Ala Ile Thr Thr Asn Asn Glu Leu Phe Ile
               100                 105                 110
   gcc ccg atg gcg aaa gcc tgt gaa cgc ctt ggc ctg agg ggc gcc gga 384
   Ala Pro Met Ala Lys Ala Cys Glu Arg Leu Gly Leu Arg Gly Ala Gly
   115 120 125
   gtg cag gca gcg gaa aat gcc aga gat aaa aat aaa atg agg gac gct 432
   Val Gln Ala Ala Glu Asn Ala Arg Asp Lys Asn Lys Met Arg Asp Ala
   130 135 140
   ttt aat aag gcg gga gtc aaa tcg atc aaa aac aaa cga gtc aca act 480
   Phe Asn Lys Ala Gly Val Lys Ser Ile Lys Asn Lys Arg Val Thr Thr
   145 150 155 160
   ctt gag gat ttt cgt gct gca ctt gaa gag atc ggc aca cct cta atc 528
   Leu Glu Asp Phe Arg Ala Ala Leu Glu Glu Ile Gly Thr Pro Leu Ile
   165 170 175
   tta aag cct aca tac tta gcg agt tca atc ggc gta acg ctg att acc 576
   Leu Lys Pro Thr Tyr Leu Ala Ser Ser Ile Gly Val Thr Leu Ile Thr
   180 185 190
   gac acg gag acg gca gaa gat gaa ttt aac aga gtc aat gac tac ctg 624
   Asp Thr Glu Thr Ala Glu Asp Glu Phe Asn Arg Val Asn Asp Tyr Leu
   195 200 205
   aaa tcg att aac gtg ccg aag gcg gtc aca ttt gaa gca ccg ttt att 672
   Lys Ser Ile Asn Val Pro Lys Ala Val Thr Phe Glu Ala Pro Phe Ile
   210 215 220
   gct gag gaa ttt tta cag ggt gag tac gga gac tgg tat caa aca gaa 720
   Ala Glu Glu Phe Leu Gln Gly Glu Tyr Gly Asp Trp Tyr Gln Thr Glu
   225 230 235 240
   ggg tac tcc gac tat atc agc ata gaa ggc att atg gca gat ggt gag 768
   Gly Tyr Ser Asp Tyr Ile Ser Ile Glu Gly Ile Met Ala Asp Gly Glu
   245 250 255
   tat ttt ccg atc gcc att cat gac aaa acg ccg caa att gga ttt aca 816
   Tyr Phe Pro Ile Ala Ile His Asp Lys Thr Pro Gln Ile Gly Phe Thr
   260 265 270
   gag aca tca cat att acg cca tcc att ctg gat gaa gag gcg aaa aag 864
   Glu Thr Ser His Ile Thr Pro Ser Ile Leu Asp Glu Glu Ala Lys Lys
   275 280 285
   aaa att gtc gaa gcg gct aaa aag gca aat gaa ggg ctt gga ctg caa 912
   Lys Ile Val Glu Ala Ala Lys Lys Ala Asn Glu Gly Leu Gly Leu Gln
   290 295 300
   aat tgc gca aca cat aca gaa atc aag cta atg aaa aac aga gaa ccg 960
   Asn Cys Ala Thr His Thr Glu Ile Lys Leu Met Lys Asn Arg Glu Pro
   305 310 315 320
   ggt tta ata gag tcg gct gcc aga ttc gca ggc tgg aat atg att cct 1008
   Gly Leu Ile Glu Ser Ala Ala Arg Phe Ala Gly Trp Asn Met Ile Pro
   325 330 335
   aac att aaa aag gtt ttc ggc ctt gat atg gcg caa tta tta tta gat 1056
   Asn Ile Lys Lys Val Phe Gly Leu Asp Met Ala Gln Leu Leu Leu Asp
   340 345 350
   gtt ctc tgt ttc gga aaa gat gct gat ctg ccg gac ggg tta ttg gat 1104
   Val Leu Cys Phe Gly Lys Asp Ala Asp Leu Pro Asp Gly Leu Leu Asp
   355 360 365
   caa gag cct tac tat gtt gct gac tgc cat ctg tac cct cag cat ttc 1152
   Gln Glu Pro Tyr Tyr Val Ala Asp Cys His Leu Tyr Pro Gln His Phe
   370 375 380
   aaa caa aat ggc cag atc cct gaa act gcc gag gat ttg gta atc gaa      1200
   Lys Gln Asn Gly Gln Ile Pro Glu Thr Ala Glu Asp Leu Val Ile Glu
   385                 390                 395                 400
   gcg atc gat att ccg gat ggg ctt ttg aag ggt gat aca gaa atc gtt      1248
   Ala Ile Asp Ile Pro Asp Gly Leu Leu Lys Gly Asp Thr Glu Ile Val
                   405                 410                 415
   act ttt tcg gct gcg gca cca gga aca tca gtt gat ttg aca ctg ttt      1296
   Thr Phe Ser Ala Ala Ala Pro Gly Thr Ser Val Asp Leu Thr Leu Phe
               420                 425                 430
   gaa gcc ttc aac tcc att gct gca ttt gaa ctg aaa ggc agc aat tca      1344
   Glu Ala Phe Asn Ser Ile Ala Ala Phe Glu Leu Lys Gly Ser Asn Ser
           435                 440                 445
   cag gat gtg gct gaa tca atc aga caa att cag cag cat gcg aag ctg      1392
   Gln Asp Val Ala Glu Ser Ile Arg Gln Ile Gln Gln His Ala Lys Leu
       450                 455                 460
   acg gca aag tat gtg ctg cca gta                                      1416
   Thr Ala Lys Tyr Val Leu Pro Val
   465                 470
<210> 31
   <211> 1416
   <212> DNA
   <213> Bacillus subtilis ATCC21555
<400> 31
   atg gag aga aaa aca gta ttg gtt atc gct gat ctt ggg ggc tgc ccg      48
   Met Glu Arg Lys Thr Val Leu Val Ile Ala Asp Leu Gly Gly Cys Pro
    1               5                  10                  15
   ccg cat atg ttt tac aaa agc gca gcc gaa aaa tac aac ctc gtc agc      96
   Pro His Met Phe Tyr Lys Ser Ala Ala Glu Lys Tyr Asn Leu Val Ser
               20                  25                  30
   ttt att ccg aga ccc ttt gca att aca gcc tct cat gcg gcc tta att     144
   Phe Ile Pro Arg Pro Phe Ala Ile Thr Ala Ser His Ala Ala Leu Ile
           35                  40                  45
   gaa aaa tac tcg att gcg gtc att aaa gat aaa gac tat ttt aag agt     192
   Glu Lys Tyr Ser Ile Ala Val Ile Lys Asp Lys Asp Tyr Phe Lys Ser
       50                  55                  60
   ctg gct gat ttt gaa cat ccc gat tcg att tat tgg gct cat gaa gat     240
   Leu Ala Asp Phe Glu His Pro Asp Ser Ile Tyr Trp Ala His Glu Asp
   65                  70                  75                  80
   cat gac aaa cct gag gaa gaa gtc gtc gaa gaa atc gtg aaa gtg gcc     288
   His Asp Lys Pro Glu Glu Glu Val Val Glu Glu Ile Val Lys Val Ala
                   85                  90                  95
   gac atg ttt ggg gtt gac gcc att acg acc aac aat gaa ctg ttt atc     336
   Asp Met Phe Gly Val Asp Ala Ile Thr Thr Asn Asn Glu Leu Phe Ile
               100                 105                 110
   gct ccg atg gca aaa gcg tgt aaa cgt ctc ggc ctg cgg gga gcg ggc     384
   Ala Pro Met Ala Lys Ala Cys Lys Arg Leu Gly Leu Arg Gly Ala Gly
           115                 120                 125
   gta cag gcc gct gaa aac gcc aga gat aaa aat aaa atg aga gcc gcc     432
   Val Gln Ala Ala Glu Asn Ala Arg Asp Lys Asn Lys Met Arg Ala Ala
       130                 135                 140
   ttc aac cgg gcc ggc gtc aaa tcc atc aaa aac aaa cgg gtg acg acc     480
   Phe Asn Arg Ala Gly Val Lys Ser Ile Lys Asn Lys Arg Val Thr Thr
   145                 150                 155                 160
   ctg gaa gat ttc cgc gcc gcg ctt cag gaa atc gga acg ccg ctt att      528
   Leu Glu Asp Phe Arg Ala Ala Leu Gln Glu Ile Gly Thr Pro Leu Ile
                   165                 170                 175
   ctg aag cct aca tat ctg gca agc tcg atc ggc gtg acg ctt att aaa      576
   Leu Lys Pro Thr Tyr Leu Ala Ser Ser Ile Gly Val Thr Leu Ile Lys
               180                 185                 190
   gag atg gaa acg gcc gaa gct gaa ttc aac aga gtc aat gag tac ttg      624
   Glu Met Glu Thr Ala Glu Ala Glu Phe Asn Arg Val Asn Glu Tyr Leu
           195                 200                 205
   aaa tcg att aat gta ccg aaa gcg gtg acg ttt gaa gcg ccg ttt atc      672
   Lys Ser Ile Asn Val Pro Lys Ala Val Thr Phe Glu Ala Pro Phe Ile
       210                 215                 220
   gcg gaa gaa ttc ttg cag ggc gag tat gat gac tgg tac gaa aca agc      720
   Ala Glu Glu Phe Leu Gln Gly Glu Tyr Asp Asp Trp Tyr Glu Thr Ser
   225                 230                 235                 240
   ggt tat tcc gac tat atc agc atc gaa ggc atc atg gcc gac gga gaa      768
   Gly Tyr Ser Asp Tyr Ile Ser Ile Glu Gly Ile Met Ala Asp Gly Glu
                   245                 250                 255
   tac ttc ccc gtt gcg atc cat gat aaa aca ccg caa atc gga ttc acg      816
   Tyr Phe Pro Val Ala Ile His Asp Lys Thr Pro Gln Ile Gly Phe Thr
               260                 265                 270
   gag aca gcg cat att acg ccg tcc atc ctg gat gat gac gcc aag cgg      864
   Glu Thr Ala His Ile Thr Pro Ser Ile Leu Asp Asp Asp Ala Lys Arg
           275                 280                 285
   aaa atc gtc gaa gct gcc aag aag gcg aat gaa gga ctc ggc ctc gaa      912
   Lys Ile Val Glu Ala Ala Lys Lys Ala Asn Glu Gly Leu Gly Leu Glu
       290                 295                 300
   aac tgt gca acg cat aca gaa ata aaa tta atg aaa aac cgg gaa gcc      960
   Asn Cys Ala Thr His Thr Glu Ile Lys Leu Met Lys Asn Arg Glu Ala
   305                 310                 315                 320
   gga ctg att gag tca gcg gcc aga ttc gcg gga tgg aat atg att ccg     1008
   Gly Leu Ile Glu Ser Ala Ala Arg Phe Ala Gly Trp Asn Met Ile Pro
                   325                 330                 335
   aat att aaa aag gtc ttc ggc gtt gat atg gcg cag cta tta ttg gat     1056
   Asn Ile Lys Lys Val Phe Gly Val Asp Met Ala Gln Leu Leu Leu Asp
               340                 345                 350
   gtt ctc tgt tac gga aaa gaa gct gat ctg ccg aaa gga tta ttg gag     1104
   Val Leu Cys Tyr Gly Lys Glu Ala Asp Leu Pro Lys Gly Leu Leu Glu
           355                 360                 365
   cag gag cca tgc tat gtc gca gac tgc cac ttg tat cct cag cat ttc     1152
   Gln Glu Pro Cys Tyr Val Ala Asp Cys His Leu Tyr Pro Gln His Phe
       370                 375                 380
   aaa gag aac ggc cag ctg cct gag acg gtt gtc gat ttc gtc att gaa     1200
   Lys Glu Asn Gly Gln Leu Pro Glu Thr Val Val Asp Phe Val Ile Glu
   385                 390                 395                 400
   agc att gaa att cct gac ggc gtc tta aag gga gac act gaa ctc gtt     1248
   Ser Ile Glu Ile Pro Asp Gly Val Leu Lys Gly Asp Thr Glu Leu Val
                   405                 410                 415
   tct ttc tca gcg gct gag gcg ggt acg tca gtg gat ctg cgg ctg ttc     1296
   Ser Phe Ser Ala Ala Glu Ala Gly Thr Ser Val Asp Leu Arg Leu Phe
               420                 425                 430
   gaa gcg ttc aac agc att gcg gcg ttt gag ctg aaa gga agc aat tcg     1344
   Glu Ala Phe Asn Ser Ile Ala Ala Phe Glu Leu Lys Gly Ser Asn Ser
           435                 440                 445
   aac gac gtg gcc gaa tca atc aaa caa att cag cag cag gcg aag ctg     1392
   Asn Asp Val Ala Glu Ser Ile Lys Gln Ile Gln Gln Gln Ala Lys Leu
       450                 455                 460
   act gca aag tat gcg tta tcg gta                                     1416
   Thr Ala Lys Tyr Ala Leu Ser Val
<210> 32
   <211> 1416
   <212> DNA
   <213> Bacillus amyloliquefaciens IFO3022
<400> 32
   atg gag aga aaa aca gta ttg gtt atc gct gac ctt ggg gga tgc ccg     48
   Met Glu Arg Lys Thr Val Leu Val Ile Ala Asp Leu Gly Gly Cys Pro
   1               5                   10                  15
   ccg cat atg ttt tac aaa agc gca gcc gaa aaa tac aac ctc gtc agc     96
   Pro His Met Phe Tyr Lys Ser Ala Ala Glu Lys Tyr Asn Leu Val Ser
               20                  25                  30
   ttt att ccg aga cct ttt gca att aca gcc tct cat gcg gca tta att     144
   Phe Ile Pro Arg Pro Phe Ala Ile Thr Ala Ser His Ala Ala Leu Ile
           35                  40                  45
   gaa aaa tac tcg gtc gcg gtc ata aaa gat aaa gac tat ttt aag agt     192
   Glu Lys Tyr Ser Val Ala Val Ile Lys Asp Lys Asp Tyr Phe Lys Ser
       50                  55                  60
   ctg gct gat ttt gag cat ccc gat tcg att tac tgg gct cat gaa gat     240
   Leu Ala Asp Phe Glu His Pro Asp Ser Ile Tyr Trp Ala His Glu Asp
   65                  70                  75                  80
   cat gac aaa cct gag gaa gaa gta gtc gaa gaa atc gtc aag gtg gcc     288
   His Asp Lys Pro Glu Glu Glu Val Val Glu Glu Ile Val Lys Val Ala
                   85                  90                  95
   ggc atg ttc gcg gtt gac gcc att acg acc aac aat gaa ctg ttt atc     336
   Gly Met Phe Ala Val Asp Ala Ile Thr Thr Asn Asn Glu Leu Phe Ile
               100                 105                 110
   gct ccg atg gca aaa gcg tgt gaa cgt ctc ggc ctg cgg gga gcg ggc     384
   Ala Pro Met Ala Lys Ala Cys Glu Arg Leu Gly Leu Arg Gly Ala Gly
           115                 120                 125
   gta cag gcc gct gaa aat gcc aga gat aaa aac aaa atg aga gcc gct     432
   Val Gln Ala Ala Glu Asn Ala Arg Asp Lys Asn Lys Met Arg Ala Ala
       130                 135                 140
   ttc aac cgg gcc ggc gtc aag tct atc aaa aac aga cgg gtg acg acg     480
   Phe Asn Arg Ala Gly Val Lys Ser Ile Lys Asn Arg Arg Val Thr Thr
   145                 150                 155                 160
   ctg gaa gat ttc cgc gcc gcg ctt cag gaa atc gga acg ccg ctc att     528
   Leu Glu Asp Phe Arg Ala Ala Leu Gln Glu Ile Gly Thr Pro Leu Ile
                   165                 170                 175
   ctg aag cct aca tat ctg gcg agc tcc atc ggc gtg acg ctc atc aaa     576
   Leu Lys Pro Thr Tyr Leu Ala Ser Ser Ile Gly Val Thr Leu Ile Lys
               180                 185                 190
   gag agg gaa acg gcc gaa gcc gaa ttt aac aga gtc aat gaa tac ctg     624
   Glu Arg Glu Thr Ala Glu Ala Glu Phe Asn Arg Val Asn Glu Tyr Leu
           195                 200                 205
   aag tcg atc aac gta ccg aaa gcg gtc acg ttt gaa gcg ccg ttt atc     672
   Lys Ser Ile Asn Val Pro Lys Ala Val Thr Phe Glu Ala Pro Phe Ile
       210                 215                 220
   gcg gaa gaa ttt ttg cag ggc gag tat gac gac tgg tac gaa aca agc     720
   Ala Glu Glu Phe Leu Gln Gly Glu Tyr Asp Asp Trp Tyr Glu Thr Ser
   225                 230                 235                     240
   ggt tat tcc gac tat atc agc ata gaa ggc atc atg gcc gac gga gaa     768
   Gly Tyr Ser Asp Tyr Ile Ser Ile Glu Gly Ile Met Ala Asp Gly Glu
                   245                 250                 255
   tac ttc cct gtc gca att cat gat aaa aca ccg caa atc gga ttc acg     816
   Tyr Phe Pro Val Ala Ile His Asp Lys Thr Pro Gln Ile Gly Phe Thr
               260                 265                 270
   gag aca tcg cat att acg ccg tcc atc ctg gat gat gac gcg aag cgg     864
   Glu Thr Ser His Ile Thr Pro Ser Ile Leu Asp Asp Asp Ala Lys Arg
           275                 280                 285
   aaa atc gtc gaa gca gcc aaa aag gcg aat gaa gga ctc ggc ctc gaa     912
   Lys Ile Val Glu Ala Ala Lys Lys Ala Asn Glu Gly Leu Gly Leu Glu
       290                 295                 300
   aac tgc gca acc cat aca gag att aaa tta atg aaa aac cgg gaa gcc     960
   Asn Cys Ala Thr His Thr Glu Ile Lys Leu Met Lys Asn Arg Glu Ala
   305                 310                 315                     320
   gga ctg att gaa tca gcg gca cga ttt gcg ggc tgg aac atg att ccg     1008
   Gly Leu Ile Glu Ser Ala Ala Arg Phe Ala Gly Trp Asn Met Ile Pro
                   325                 330                 335
   aat att aaa aag gtc ttc ggc gtc gat atg gcg cag ctg tta ttg gat     1056
   Asn Ile Lys Lys Val Phe Gly Val Asp Met Ala Gln Leu Leu Leu Asp
               340                 345                 350
   gtt ctc tgt ttc gga aaa gaa gcc gat ctg ccg aaa gga tta ttg gag     1104
   Val Leu Cys Phe Gly Lys Glu Ala Asp Leu Pro Lys Gly Leu Leu Glu
           355                 360                 365
   cag gag ccg tgc tat gtc gcc gac tgc cac ttg tat cct cag cat ttc     1152
   Gln Glu Pro Cys Tyr Val Ala Asp Cys His Leu Tyr Pro Gln His Phe
       370                 375                 380
   aaa gag aac ggc cag ctg cct gag acg gct gtc gat ttc gtc att gaa     1200
   Lys Glu Asn Gly Gln Leu Pro Glu Thr Ala Val Asp Phe Val Ile Glu
   385                 390                 395                     400
   agc att gac att ccc gac ggc gtc tta aag gga gac acc gaa atc gtt     1248
   Ser Ile Asp Ile Pro Asp Gly Val Leu Lys Gly Asp Thr Glu Ile Val
                   405                 410                 415
   tct ttc tcg gcg gcc gag gcg ggt aca tcc gtg gat ctg cgg ctg ttc     1296
   Ser Phe Ser Ala Ala Glu Ala Gly Thr Ser Val Asp Leu Arg Leu Phe
               420                 425                 430
   gaa gcg ttc aac agc att gcg gcg ttc gag ctg aaa gga agc aat tcg     1344
   Glu Ala Phe Asn Ser Ile Ala Ala Phe Glu Leu Lys Gly Ser Asn Ser
           435                 440                 445
   ggt gac gtg gcc gaa tca atc aaa caa att cag cag cag gcg aag ctg     1392
   Gly Asp Val Ala Glu Ser Ile Lys Gln Ile Gln Gln Gln Ala Lys Leu
       450                 455                 460
   act gca aag tat gcg tta ccg gta                                 1416
   Thr Ala Lys Tyr Ala Leu Pro Val
<210> 33
   <211> 93
   <212> PRT
   <213> Bacillus subtilis 168
<400> 33
   Gly Ala Gly Val Gln Ala Ala Glu Asn Ala Arg Asp Lys Asn Lys Met
   1               5                   10                  15
   Arg Asp Ala Phe Asn Lys Ala Gly Val Lys Ser Ile Lys Asn Lys Arg
               20                  25                  30
   Val Thr Thr Leu Glu Asp Phe Arg Ala Ala Leu Glu Glu Ile Gly Thr
           35                  40                  45
   Pro Leu Ile Leu Lys Pro Thr Tyr Leu Ala Ser Ser Ile Gly Val Thr
       50                  55                  60
   Leu Ile Thr Asp Thr Glu Thr Ala Glu Asp Glu Phe Asn Arg Val Asn
   65                  70                  75                  80
   Asp Tyr Leu Lys Ser Ile Asn Val Pro Lys Ala Val Thr
                   85                  90
<210> 34
   <211> 279
   <212> DNA
   <213> Bacillus subtilis 168
<400> 34
   ggt gcc ggc gtg cag gca gcc gaa aat gcc aga gat aaa aat aaa atg      48
   Gly Ala Gly Val Gln Ala Ala Glu Asn Ala Arg Asp Lys Asn Lys Met
   1               5                   10                  15
   agg gac gct ttt aat aag gcc gga gtc aaa tcg atc aaa aac aaa cga      96
   Arg Asp Ala Phe Asn Lys Ala Gly Val Lys Ser Ile Lys Asn Lys Arg
               20                  25                  30
   gtc aca act ctt gaa gat ttc cgt gct gct ctt gaa gag atc ggc aca     144
   Val Thr Thr Leu Glu Asp Phe Arg Ala Ala Leu Glu Glu Ile Gly Thr
           35                  40                  45
   cct ctt atc tta aag cct aca tac tta gcg agt tct atc ggt gta acg     192
   Pro Leu Ile Leu Lys Pro Thr Tyr Leu Ala Ser Ser Ile Gly Val Thr
       50                  55                  60
   ctg att acg gac act gag acg gca gaa gat gaa ttt aac aga gtc aat     240
   Leu Ile Thr Asp Thr Glu Thr Ala Glu Asp Glu Phe Asn Arg Val Asn
   65                  70                  75                  80
   gac tat ctg aaa tca att aac gtg cca aag gcg gtt acg                 279
   Asp Tyr Leu Lys Ser Ile Asn Val Pro Lys Ala Val Thr
                   85                  90
<210> 35
   <211> 27
   <212> DNA
   <213> Artificial Sequence
<220>
   <223> Description of Artificial Sequence: Synthetic DNA
<400> 35
   ttaaccatgg agagaaaaac agtattg 27
<210> 36
   <211> 30
   <212> DNA
   <213> Artificial Sequence
<220>
   <223> Description of Artificial Sequence: synthetic DNA
<400> 36
   atatggatcc tactggcagc acatactttg 30
<210> 37
   <211> 21
   <212> DNA
   <213> Artificial sequence
<220>
   <223> Description of Artificial Sequence: Synthetic DNA
<400> 37
   caccgcagac ggaggataca c 21
<210> 38
   <211> 22
   <212> DNA
   <213> Artificial Sequence
<220>
   <223> Description of Artificial Sequence: Synthetic DNA
<400> 38
   cggacgtcac ccaataatcg tg 22
<210> 39
   <211> 34
   <212> DNA
   <213> Artificial Sequence
<220>
   <223> Description of Artificial Sequence: Synthetic DNA
<400> 39
   gaagttccta tactttctag agaataggaa cttc 34
<210> 40
   <211> 56
   <212> DNA
   <213> Artificial Sequence
<220>
   <223> Description of Artificial Sequence: Synthetic DNA
<400> 40
   ctaaccctgt gacctgcaat actgttttgc gggtgagtgt aggctggagc tgcttc 56
<210> 41
   <211> 56
   <212> DNA
   <213> Artificial Sequence
<220>
   <223> Description of Artificial Sequence: Synthetic DNA
<400> 41
   gaaactgccg gaaggcgatt aaacgccatc cggcagcata tgaatatcct ccttag 56
<210> 42
   <211> 56
   <212> DNA
   <213> Artificial Sequence
<220>
   <223> Description of Artificial Sequence: Synthetic DNA
<400> 42
   ttacgcaaca ggaatagact gaacaccaga ctctatgtgt aggctggagc tgcttc 56
<210> 43
   <211> 56
   <212> DNA
   <213> Artificial sequence
<220>
   <223> Description of Artificial Sequence: Synthetic DNA
<400> 43
   agaaaacagg ggtaaattcc ccgaatggcg gcgctacata tgaatatcct ccttag 56
<210> 44
   <211> 56
   <212> DNA
   <213> Artificial sequence
<220>
   <223> Description of Artificial Sequence: Synthetic DNA
<400> 44
   atggagttta gtgtaaaaag cggtagcccg gagaaagtgt aggctggagc tgcttc 56
<210> 45
   <211> 56
   <212> DNA
   <213> Artificial Sequence
<220>
   <223> Description of Artificial Sequence: Synthetic DNA
<400> 45
   ttactcttcg ccgttaaacc cagcgcggtt taacagcata tgaatatcct ccttag 56
<210> 46
   <211> 56
   <212> DNA
   <213> Artificial Sequence
<220>
   <223> Description of Artificial Sequence: Synthetic DNA
<400> 46
   atgacagaag cgatgaagat taccctctct acccaagtgt aggctggagc tgcttc 56
<210> 47
   <211> 56
   <212> DNA
   <213> Artificial Sequence
<220>
   <223> Description of Artificial Sequence: Synthetic DNA
<400> 47
   ttacgccgtt aacagattag ctatcgtgcg cacacccata tgaatatcct ccttag 56
<210> 48
   <211> 56
   <212> DNA
   <213> Artificial Sequence
<220>
   <223> Description of Artificial Sequence: Synthetic DNA
<400> 48
   gcatccccac ctcataacgt tgacccgacc gggcaagtgt aggctggagc tgcttc 56
<210> 49
   <211> 56
   <212> DNA
   <213> Artificial Sequence
<220>
   <223> Description of Artificial Sequence: Synthetic DNA
<400> 49
   ctgtacggca ttttgctatg cttgtcgcca ctgttgcata tgaatatcct ccttag 56
<210> 50
   <211> 21
   <212> DNA
   <213> Artificial Sequence
<220>
   <223> Description of Artificial Sequence: Synthetic DNA
<400> 50
   gtgtctgaac tgtctcaatt a 21
<210> 51
   <211> 21
   <212> DNA
   <213> Artificial Sequence
<220>
   <223> Description of Artificial Sequence: synthetic DNA
<400> 51
   cggaatttct ttcagcagtt c 21
<210> 52
   <211> 21
   <212> DNA
   <213> Artificial Sequence
<220>
   <223> Description of Artificial Sequence: Synthetic DNA
<400> 52
   atgactcaac agccacaagc c 21
<210> 53
   <211> 21
   <212> DNA
   <213> Artificial Sequence
<220>
   <223> Description of Artificial Sequence: Synthetic DNA
<400> 53
   tgctttagtt atcttctcgt a 21
<210> 54
   <211> 21
   <212> DNA
   <213> Artificial Sequence
<220>
   <223> Description of Artificial Sequence: Synthetic DNA
<400> 54
   agtgcctgca tcgtcgtggg c 21
<210> 55
   <211> 21
   <212> DNA
   <213> Artificial Sequence
<220>
   <223> Description of Artificial Sequence: Synthetic DNA
<400> 55
   ggcgcctttt gctttaccag a 21
<210> 56
   <211> 21
   <212> DNA
   <213> Artificial Sequence
<220>
   <223> Description of Artificial Sequence: Synthetic DNA
<400> 56
   gacgcgcgct ggggagaaaa a 21
<210> 57
   <211> 21
   <212> DNA
   <213> Artificial Sequence
<220>
   <223> Description of Artificial Sequence: Synthetic DNA
<400> 57
   cgtagcgccc gcagaccact g 21
<210> 58
   <211> 21
   <212> DNA
   <213> Artificial Sequence
<220>
   <223> Description of Artificial Sequence: Synthetic DNA
<400> 58
   atgcgtattt ccttgaaaaa g 21
<210> 59
   <211> 21
   <212> DNA
   <213> Artificial Sequence
<220>
   <223> Description of Artificial Sequence: Synthetic DNA
<400> 59
   ttattcgata gagacgtttt c 21
<210> 60
   <211> 29
   <212> DNA
   <213> Artificial Sequence
<220>
   <223> Description of Artificial Sequence: Synthetic DNA
<400> 60
   tacactcgag attaaagagg agaaattaa 29
<210> 61
   <211> 30
   <212> DNA
   <213> Artificial Sequence
<220>
   <223> Description of Artificial Sequence: Synthetic DNA
<400> 61
   ttaggatcct catactggca gcacatactt 30
<210> 62
   <211> 24
   <212> DNA
   <213> Artificial Sequence
<220>
   <223> Description of Artificial Sequence: Synthetic DNA
<400> 62
   caagaattct catgtttgac agct 24
<210> 63
   <211> 28
   <212> DNA
   <213> Artificial Sequence
<220>
   <223> Description of Artificial Sequence: Synthetic DNA
<400> 63
   taactcgaga ttcccttttt acgtgaac 28
<210> 64
   <211> 1428
   <212> DNA
   <213> Bacillus pumilus NRRL B-12025
<400> 64
   gtg ctt tca ttg agt aaa aaa act gta ctt gtc att gct gac tta gga 48
   Val Leu Ser Leu Ser Lys Lys Thr Val Leu Val Ile Ala Asp Leu Gly
   1 5 10 15
   ggg tgc ccg ccc cat atg ttt tat gaa agc gtg gcg gca tca tac cat 96
   Gly Cys Pro Pro His Met Phe Tyr Glu Ser Val Ala Ala Ser Tyr His
   20 25 30
   atc gtt tct tat atc cca aga ccc ttt gcg att aca aag gga cat gcc 144
   Ile Val Ser Tyr Ile Pro Arg Pro Phe Ala Ile Thr Lys Gly His Ala
   35 40 45
   gag cta atc gaa aaa tac tcc att gcc gtc atc aaa gac cgt gat tat 192
   Glu Leu Ile Glu Lys Tyr Ser Ile Ala Val Ile Lys Asp Arg Asp Tyr
   50 55 60
   ttt gag aca cac cct tct ttt gaa cac cct gat tct att tac tgg gca 240
   Phe Glu Thr His Pro Ser Phe Glu His Pro Asp Ser Ile Tyr Trp Ala
   65 70 75 80
   cat gat gat tat cca aaa tca gaa gaa gaa gtt gtg gaa gac ttc att 288
   His Asp Asp Tyr Pro Lys Ser Glu Glu Glu Val Val Glu Asp Phe Ile
   85 90 95
   cga gta gct tcc ttt ttc aaa gca gat gca atc acg acc aat aat gaa 336
   Arg Val Ala Ser Phe Phe Lys Ala Asp Ala Ile Thr Thr Asn Asn Glu
   100 105 110
   tta ttc att gca ccg atg gca aag gcc gct gaa cgt ctt ggg cta cga 384
   Leu Phe Ile Ala Pro Met Ala Lys Ala Ala Glu Arg Leu Gly Leu Arg
   115 120 125
   ggt gcc ggt gtc aag gca gcc gaa atg gcg cgt gat aaa agc caa atg 432
   Gly Ala Gly Val Lys Ala Ala Glu Met Ala Arg Asp Lys Ser Gln Met
   130 135 140
   agg gct gca ttc aat gcc tct ggc gtc aaa gcg gtg aaa act cag cct 480
   Arg Ala Ala Phe Asn Ala Ser Gly Val Lys Ala Val Lys Thr Gln Pro
   145 150 155 160
   gtc acg act tta tct gat ttc caa caa gcc att gag tct atc gga aca 528
   Val Thr Thr Leu Ser Asp Phe Gln Gln Ala Ile Glu Ser Ile Gly Thr
   165 170 175
   ccg ctc att tta aag cct aca tat tta gcc agt tct att ggc gtc acc 576
   Pro Leu Ile Leu Lys Pro Thr Tyr Leu Ala Ser Ser Ile Gly Val Thr
   180 185 190
   ttg ttt cat gac aaa gcc gga agt gat gac ttg ttt tta caa gta caa 624
   Leu Phe His Asp Lys Ala Gly Ser Asp Asp Leu Phe Leu Gln Val Gln
   195 200 205
   tcg tat ttg gaa acc ata cca gtc cca gac gct gtc acg tat gaa gca 672
   Ser Tyr Leu Glu Thr Ile Pro Val Pro Asp Ala Val Thr Tyr Glu Ala
   210 215 220
   ccg ttt gtc gct gaa aca tat tta gag ggt gct tac gaa gat tgg tat 720
   Pro Phe Val Ala Glu Thr Tyr Leu Glu Gly Ala Tyr Glu Asp Trp Tyr
   225 230 235 240
   gaa gac gaa gga tat gct gat tat gtc agt gta gaa ggg ctg gtc gta 768
   Glu Asp Glu Gly Tyr Ala Asp Tyr Val Ser Val Glu Gly Leu Val Val
                       245                        250                        255
   gag ggc gaa tat ctc cct ttt gtc ata cat gat aaa acc cct caa atc        816
   Glu Gly Glu Tyr Leu Pro Phe Val Ile His Asp Lys Thr Pro Gln Ile
               260                     265                     270
   ggc ttt aca gaa acg gct cat atc act ccg acg atc tta gac aat gaa        864
   Gly Phe Thr Glu Thr Ala His Ile Thr Pro Thr Ile Leu Asp Asn Glu
           275                     280                     285
   gcc aag caa atc atc att gaa gca gca agg aag gca aat gaa ggg cta        912
   Ala Lys Gln Ile Ile Ile Glu Ala Ala Arg Lys Ala Asn Glu Gly Leu
       290                     295                     300
   ggt ctt gaa cat tgt gca acc cat aca gaa atc aaa ctc atg aaa aat        960
   Gly Leu Glu His Cys Ala Thr His Thr Glu Ile Lys Leu Met Lys Asn
   305                     310                     315                     320
   cga gaa act gga ctg atc gag gca gcg gct cga ttc gct ggc tgg aat       1008
   Arg Glu Thr Gly Leu Ile Glu Ala Ala Ala Arg Phe Ala Gly Trp Asn
                   325                     330                     335
   atg atc ccg aat att aaa aaa gtc ttt ggc gtc gat atg gcg aag cta       1056
   Met Ile Pro Asn Ile Lys Lys Val Phe Gly Val Asp Met Ala Lys Leu
               340                     345                     350
   ttg att gat gta tta gtt gat ggt aaa aag gct gta ctg cca aaa cag       1104
   Leu Ile Asp Val Leu Val Asp Gly Lys Lys Ala Val Leu Pro Lys Gln
           355                     360                     365
   ctg ctt tct gga cat aca ttt tat gta gcg gac tgc cac ctg tac cct       1152
   Leu Leu Ser Gly His Thr Phe Tyr Val Ala Asp Cys His Leu Tyr Pro
       370                     375                     380
   cag cat ttt aaa gag agt ggg ctt atc ccg cct gaa gcc aca cat att       1200
   Gln His Phe Lys Glu Ser Gly Leu Ile Pro Pro Glu Ala Thr His Ile
   385                     390                     395                     400
   acc att gat cat gtg tct att ccg cag gaa gca ttc gtt gga gat act       1248
   Thr Ile Asp His Val Ser Ile Pro Gln Glu Ala Phe Val Gly Asp Thr
                   405                     410                     415
   gcg att gtc agt caa tca ttc cct gcc aaa ggg act att gtg gat ctt       1296
   Ala Ile Val Ser Gln Ser Phe Pro Ala Lys Gly Thr Ile Val Asp Leu
               420                     425                     430
   gaa tta ttt gaa gct ttt aat gga atc gta tct ctt gaa tta aaa gga       1344
   Glu Leu Phe Glu Ala Phe Asn Gly Ile Val Ser Leu Glu Leu Lys Gly
           435                     440                     445
   tca tcc tca caa gat gtt gcc gcg tcc atc cgc aac att cag aaa cag       1392
   Ser Ser Ser Gln Asp Val Ala Ala Ser Ile Arg Asn Ile Gln Lys Gln
       450                     455                     460
   gca acg att cag tta atg gat gaa tta gtg aag gga                       1428
   Ala Thr Ile Gln Leu Met Asp Glu Leu Val Lys Gly
   465                     470                     475
<210> 65
   <211> 1416
   <212> DNA
   <213> Bacillus subtilis ATCC 15245 and Bacillus subtilis IAM 1033
<400> 65
   atg gag aga aaa aca gta ttg gtc atc gct gat ctt gga ggc tgc ccg    48
   Met Glu Arg Lys Thr Val Leu Val Ile Ala Asp Leu Gly Gly Cys Pro
    1               5                  10                  15
   ccg cac atg ttt tat aaa agc gct gct gaa aaa tat aac ctg gtc agc 96
   Pro His Met Phe Tyr Lys Ser Ala Ala Glu Lys Tyr Asn Leu Val Ser
   20 25 30
   ttt att cca aga cct ttt gca att aca gcc tcc cat gca gca ttg att 144
   Phe Ile Pro Arg Pro Phe Ala Ile Thr Ala Ser His Ala Ala Leu Ile
   35 40 45
   gaa aaa tac tcg gtc gcg gtc ata aaa gat aaa gac tat ttt aag agt 192
   Glu Lys Tyr Ser Val Ala Val Ile Lys Asp Lys Asp Tyr Phe Lys Ser
   50 55 60
   tta gct gat ttt gag cat cct gat tcc att tat tgg gcg cat gag gat 240
   Leu Ala Asp Phe Glu His Pro Asp Ser Ile Tyr Trp Ala His Glu Asp
   65 70 75 80
   cat aac aag cct gag gaa gag gtc gtc gag caa atc gtc aag gtt gcc 288
   His Asn Lys Pro Glu Glu Glu Val Val Glu Gln Ile Val Lys Val Ala
   85 90 95
   gaa atg ttt ggg gcg gat gcc atc aca aca aac aat gaa tta ttc att 336
   Glu Met Phe Gly Ala Asp Ala Ile Thr Thr Asn Asn Glu Leu Phe Ile
   100 105 110
   gct ccg atg gcg aaa gcc tgt gaa cgt ctg ggc ctg aga ggt gcc ggc 384
   Ala Pro Met Ala Lys Ala Cys Glu Arg Leu Gly Leu Arg Gly Ala Gly
   115 120 125
   gtg cag gca gcc gaa aat gcc aga gat aaa aat aaa atg agg gac gct 432
   Val Gln Ala Ala Glu Asn Ala Arg Asp Lys Asn Lys Met Arg Asp Ala
   130 135 140
   ttt aat aag gcc gga gtc aaa tcg atc aaa aac aaa cga gtc aca act 480
   Phe Asn Lys Ala Gly Val Lys Ser Ile Lys Asn Lys Arg Val Thr Thr
   145 150 155 160
   ctt gaa gat ttc cgt gct gct ctt gaa gag atc ggc aca cct ctt atc 528
   Leu Glu Asp Phe Arg Ala Ala Leu Glu Glu Ile Gly Thr Pro Leu Ile
   165 170 175
   tta aag cct aca tac tta gcg agt tca atc ggt gta acg ctg att acg 576
   Leu Lys Pro Thr Tyr Leu Ala Ser Ser Ile Gly Val Thr Leu Ile Thr
   180 185 190
   gac act gag acg gca gaa gat gaa ttt aac aga gtc aat gac tat ctg 624
   Asp Thr Glu Thr Ala Glu Asp Glu Phe Asn Arg Val Asn Asp Tyr Leu
   195 200 205
   aaa tca att aac gtg cca aag gcg gtt acg ttt gaa gcg ccg ttt atc 672
   Lys Ser Ile Asn Val Pro Lys Ala Val Thr Phe Glu Ala Pro Phe Ile
   210 215 220
   gct gaa gaa ttt tta cag ggt gag tac gga gac tgg tat caa aca gaa 720
   Ala Glu Glu Phe Leu Gln Gly Glu Tyr Gly Asp Trp Tyr Gln Thr Glu
   225 230 235 240
   ggg tac tcc gac tat atc agt ata gaa ggc atc atg gct gac ggt gag 768
   Gly Tyr Ser Asp Tyr Ile Ser Ile Glu Gly Ile Met Ala Asp Gly Glu
   245 250 255
   tat ttc ccg atc gcc att cat gat aaa acg ccg caa atc ggg ttt aca 816
   Tyr Phe Pro Ile Ala Ile His Asp Lys Thr Pro Gln Ile Gly Phe Thr
   260 265 270
   gag aca tcc cac att acg ccg tcc att ctg gat gaa gag gca aaa aag 864
   Glu Thr Ser His Ile Thr Pro Ser Ile Leu Asp Glu Glu Ala Lys Lys
   275 280 285
   aaa att gtc gaa gct gcc aaa aag gca aat gaa ggg ctt ggc ctg caa    912
   Lys Ile Val Glu Ala Ala Lys Lys Ala Asn Glu Gly Leu Gly Leu Gln
       290                 295                 300
   aat tgc gca aca cat aca gag atc aag cta atg aaa aac aga gaa ccg    960
   Asn Cys Ala Thr His Thr Glu Ile Lys Leu Met Lys Asn Arg Glu Pro
   305                 310                 315                 320
   ggt tta ata gag tcg gca gcc aga ttc gca ggc tgg aat atg att cct   1008
   Gly Leu Ile Glu Ser Ala Ala Arg Phe Ala Gly Trp Asn Met Ile Pro
                   325                 330                 335
   aat att aaa aag gtc ttt ggc ctt gat atg gcg caa tta tta tta gat   1056
   Asn Ile Lys Lys Val Phe Gly Leu Asp Met Ala Gln Leu Leu Leu Asp
               340                 345                 350
   gtc ctc tgt ttc gga aaa gac gcc gat ctg ccg gac gga tta ttg gat   1104
   Val Leu Cys Phe Gly Lys Asp Ala Asp Leu Pro Asp Gly Leu Leu Asp
           355                 360                 365
   caa gag cct tat tat gtt gcc gac tgc cat ttg tac ccg cag cat ttc   1152
   Gln Glu Pro Tyr Tyr Val Ala Asp Cys His Leu Tyr Pro Gln His Phe
       370                 375                 380
   aaa caa aat ggc cag att cca gaa acc gct gag gat ttg gtc att gaa   1200
   Lys Gln Asn Gly Gln Ile Pro Glu Thr Ala Glu Asp Leu Val Ile Glu
   385                 390                 395                 400
   gcg atc gat att ccg gac ggg ctt tta aaa ggg gat act gaa atc gtt   1248
   Ala Ile Asp Ile Pro Asp Gly Leu Leu Lys Gly Asp Thr Glu Ile Val
                   405                 410                 415
   tca ttt tca gcc gca gca cca ggc act tca gtt gat ttg aca ttg ttt   1296
   Ser Phe Ser Ala Ala Ala Pro Gly Thr Ser Val Asp Leu Thr Leu Phe
               420                 425                 430
   gaa gct ttc aat tcc att gct gca ttt gaa ctg aaa ggc agt aat tca   1344
   Glu Ala Phe Asn Ser Ile Ala Ala Phe Glu Leu Lys Gly Ser Asn Ser
           435                 440                 445
   cag gat gtg gct gaa tca atc aga caa att cag cag cat gca aag ctg   1392
   Gln Asp Val Ala Glu Ser Ile Arg Gln Ile Gln Gln His Ala Lys Leu
       450                 455                 460
   acg gca aag tat gtg ctg cca gta                                   1416
   Thr Ala Lys Tyr Val Leu Pro Val
   465                 470
<210> 66
   <211> 23
   <212> DNA
   <213> Artificial Sequence
<220>
   <223> Description of Artificial Sequence: Synthetic DNA
<400> 66
   ccgatggcra aagcstgtra acg 23
<210> 67
   <211> 21
   <212> DNA
   <213> Artificial Sequence
<220>
   <223> Description of Artificial Sequence: Synthetic DNA
<400> 67
   cggcagatcr gcdtcttttc c 21
<210> 68
   <211> 476
   <212> PRT
   <213> Bacillus pumilus NRRL B-12025
<400> 68
   Val Leu Ser Leu Ser Lys Lys Thr Val Leu Val Ile Ala Asp Leu Gly
    1               5                  10                  15
   Gly Cys Pro Pro His Met Phe Tyr Glu Ser Val Ala Ala Ser Tyr His
               20                  25                  30
   Ile Val Ser Tyr Ile Pro Arg Pro Phe Ala Ile Thr Lys Gly His Ala
           35                  40                  45
   Glu Leu Ile Glu Lys Tyr Ser Ile Ala Val Ile Lys Asp Arg Asp Tyr
       50                  55                  60
   Phe Glu Thr His Pro Ser Phe Glu His Pro Asp Ser Ile Tyr Trp Ala
   65                  70                  75                      80
   His Asp Asp Tyr Pro Lys Ser Glu Glu Glu Val Val Glu Asp Phe Ile
                   85                  90                  95
   Arg Val Ala Ser Phe Phe Lys Ala Asp Ala Ile Thr Thr Asn Asn Glu
               100                 105                 110
   Leu Phe Ile Ala Pro Met Ala Lys Ala Ala Glu Arg Leu Gly Leu Arg
           115                 120                 125
   Gly Ala Gly Val Lys Ala Ala Glu Met Ala Arg Asp Lys Ser Gln Met
       130                 135                 140
   Arg Ala Ala Phe Asn Ala Ser Gly Val Lys Ala Val Lys Thr Gln Pro
   145                 150                 155                     160
   Val Thr Thr Leu Ser Asp Phe Gln Gln Ala Ile Glu Ser Ile Gly Thr
                   165                 170                 175
   Pro Leu Ile Leu Lys Pro Thr Tyr Leu Ala Ser Ser Ile Gly Val Thr
               180                 185                 190
   Leu Phe His Asp Lys Ala Gly Ser Asp Asp Leu Phe Leu Gln Val Gln
           195                 200                 205
   Ser Tyr Leu Glu Thr Ile Pro Val Pro Asp Ala Val Thr Tyr Glu Ala
       210                 215                 220
   Pro Phe Val Ala Glu Thr Tyr Leu Glu Gly Ala Tyr Glu Asp Trp Tyr
   225                 230                 235                     240
   Glu Asp Glu Gly Tyr Ala Asp Tyr Val Ser Val Glu Gly Leu Val Val
                   245                 250                 255
   Glu Gly Glu Tyr Leu Pro Phe Val Ile His Asp Lys Thr Pro Gln Ile
               260                 265                 270
   Gly Phe Thr Glu Thr Ala His Ile Thr Pro Thr Ile Leu Asp Asn Glu
           275                 280                 285
   Ala Lys Gln Ile Ile Ile Glu Ala Ala Arg Lys Ala Asn Glu Gly Leu
       290                 295                 300
   Gly Leu Glu His Cys Ala Thr His Thr Glu Ile Lys Leu Met Lys Asn
   305 310 315 320
   Arg Glu Thr Gly Leu Ile Glu Ala Ala Ala Arg Phe Ala Gly Trp Asn
   325 330 335
   Met Ile Pro Asn Ile Lys Lys Val Phe Gly Val Asp Met Ala Lys Leu
   340 345 350
   Leu Ile Asp Val Leu Val Asp Gly Lys Lys Ala Val Leu Pro Lys Gln
   355 360 365
   Leu Leu Ser Gly His Thr Phe Tyr Val Ala Asp Cys His Leu Tyr Pro
   370 375 380
   Gln His Phe Lys Glu Ser Gly Leu Ile Pro Pro Glu Ala Thr His Ile
   385 390 395 400
   Thr Ile Asp His Val Ser Ile Pro Gln Glu Ala Phe Val Gly Asp Thr
   405 410 415
   Ala Ile Val Ser Gln Ser Phe Pro Ala Lys Gly Thr Ile Val Asp Leu
   420 425 430
   Glu Leu Phe Glu Ala Phe Asn Gly Ile Val Ser Leu Glu Leu Lys Gly
   435 440 445
   Ser Ser Ser Gln Asp Val Ala Ala Ser Ile Arg Asn Ile Gln Lys Gln
   450 455 460
   Ala Thr Ile Gln Leu Met Asp Glu Leu Val Lys Gly
   465 470 475
<210> 69
   <211> 44
   <212> DNA
   <213> Artificial Sequence
<220>
   <223> Description of Artificial Sequence: Synthetic DNA
<400> 69
   catgccatgg agaaaaaaac tgtacttgtc attgctgact tagg □@□@□@ 44
<210> 70
   <211> 38
   <212> DNA
   <213> Artificial Sequence
<220>
   <223> Description of Artificial Sequence: Synthetic DNA
<400> 70
   cgcggatccc ttcactaatt catccattaa ctgaatcg □@□@□@38
<210> 71
   <211> 26
   <212> DNA
   <213> Artificial Sequence
<220>
   <223> Description of Artificial Sequence: Synthetic DNA
<400> 71
   gctaggtctt gaacattgtg caaccc 26
<210> 72
   <211> 23
   <212> DNA
   <213> Artificial Sequence
<220>
   <223> Description of Artificial Sequence: Synthetic DNA
<400> 72
   ggtgttccga tagactcaat ggc 23